(19) Europäisches Patentamt

European Patent Office

Office européen des brevets

(11) **EP 3 852 868 B1**

(12) **EUROPEAN PATENT SPECIFICATION**

(45) Date of publication and mention of the grant of the patent:
**12.02.2025 Bulletin 2025/07**

(21) Application number: **19861489.3**

(22) Date of filing: **18.09.2019**

(51) International Patent Classification (IPC):
**A61N 1/32** *(2006.01)*     **A61N 1/06** *(2006.01)*
**A61B 18/14** *(2006.01)*     A61N 1/04 *(2006.01)*
A61N 1/05 *(2006.01)*     A61B 18/00 *(2006.01)*

(52) Cooperative Patent Classification (CPC):
**A61N 1/06; A61B 18/1477; A61N 1/327;**
A61B 2018/00613; A61B 2018/00827; A61N 1/0412;
A61N 1/0502

(86) International application number:
**PCT/US2019/051731**

(87) International publication number:
**WO 2020/061192 (26.03.2020 Gazette 2020/13)**

(54) **TREATMENT PLANNING SYSTEM FOR IMMUNOTHERAPY ENHANCEMENT VIA NON-THERMAL ABLATION**

SYSTEM ZUR BEHANDLUNGSPLANUNG EINER IMMUNTHERAPIE MITTELS NICHT-THERMISCHER ABLATION

SYSTÈME DE PLANIFICATION DE TRAITEMENT POUR AMÉLIORATION DE L'IMMUNOTHÉRAPIE PAR ABLATION NON THERMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.09.2018 US 201862732703 P**

(43) Date of publication of application:
**28.07.2021 Bulletin 2021/30**

(73) Proprietor: **Virginia Tech Intellectual Properties, Inc.**
**Blacksburg, VA 24061 (US)**

(72) Inventors:
• **DAVALOS, Rafael V.**
**Blacksburg, VA 24060 (US)**

• **WHITE, Natalie**
**Christiansburg, VA 24073 (US)**

(74) Representative: **Jaekel, Robert Sebastian**
**Witthoff Jaekel Steinecke**
**Patentanwälte PartG mbB**
**Widdersdorfer Straße 236-240**
**50825 Köln (DE)**

(56) References cited:
| | |
|---|---|
| US-A1- 2006 278 241 | US-A1- 2014 017 218 |
| US-A1- 2015 152 504 | US-A1- 2015 320 999 |
| US-A1- 2016 066 977 | US-A1- 2016 143 698 |
| US-A1- 2016 367 310 | US-A1- 2017 319 851 |
| US-A1- 2017 348 525 | US-A1- 2018 071 014 |

**Description**

**BACKGROUND**

Some types of cancers come with the most dismal prognoses, particularly after metastasis has already occurred. Although several immunotherapies for cancers exists, there still is a great need for techniques to allow treatment customization to improve treatment outcomes on the individual patient level. US 2017/348525 A1 shows delivery of electrical pulses and monitoring of the current for adjusting or stopping the pulse delivery. US 2016/066977 A1 shows delivery of IRE pulses and monitoring of the current to indicate completeness or progress of the treatment. US 2017/319851 A1 shows delivery of IRE pulses and monitoring of the current to determine a load impedance, which may be used to determine a setup problem, a load problem, an electrode problem, or another system problem. US 2016/143698 A1 shows delivery of electrical pulses and monitoring of the current to determine the extent of electroporation in the tissue and predict the treatment volume. US 2018/071014 A11 shows delivery of IRE pulses and monitoring of the current for controlling delivery of a perfusion fluid.

**SUMMARY**

The present invention relates to a medical diagnostic system as defined by the appended claims. Also described hereinafter, is an exemplary, non-claimed method of treating a tissue in a patient that can include ablating the tissue using a non-thermal ablation technique; measuring a change in a treatment parameter in real-time during the step of ablating; and administering an additional treatment to the subject in response to the measured change in the treatment parameter. The step of administering the additional treatment can occur 4-30 days post ablating. The additional treatment can be selected from the group of: tissue resection, thermal ablation, non-thermal ablation, chemotherapy, radiation therapy, immunotherapy, biologic therapy, genetic therapy, and combinations thereof. The additional treatment can be measuring the amount of a pro-inflammatory immune molecule or cell, a suppressive immune molecule or cell, or both in a bodily fluid or a biopsied tissue of the patient. The non-thermal ablation technique can be irreversible electroporation. The non-thermal ablation technique, can be high-frequency irreversible electroporation. The treatment parameter can be bulk tissue conductivity. The change in bulk tissue conductivity can be measured by measuring current during the step of ablating. The step of administering the additional treatment can occur 4-30 days post ablating when the current measured is between 25 A and 100 A. The treatment parameter can be measured by measuring current during the step of ablating. The step of administering the additional treatment can occur 4-30 days post ablating when the current measured is between 25 A and 100 A.

Also described herein is an exemplary, non-claimed method that can include a method of treating a tissue in a patient comprising: inserting at least a first electrode into the tissue applying a plurality of electrical pulses through the first electrode and to a second electrode (which may also be inserted into the tissue or may be on the surface of the patient's body), wherein the electrical pulses are configured to cause non-thermal irreversible electroporation of the tissue; wherein the electrical pulses are configured to be delivered in either AC or DC, a monopolar, monophasic, single polarity, or in a bipolar, biphasic, alternative polarity; measuring a current of the applied electrical pulses; and administering an additional treatment to the subject in response to the subject, wherein the step of administering occurs 0 to 5 days after non-thermal irreversible electroporation when the current is less than 25A, and wherein the step of administering occurs 4-30 days after non-thermal irreversible electroporation when the current is between 25 A and 100 A. The additional treatment can be selected from the group consisting of: tissue resection, thermal ablation, non-thermal ablation, chemotherapy, radiation therapy, immunotherapy, biologic therapy, genetic therapy, and combinations thereof. The additional treatment can be measuring the amount of a pro-inflammatory immune molecule or cell, a suppressive immune molecule or cell, or both in a bodily fluid or a biopsied tissue of the patient. The electrical pulses can be configured to cause non-thermal irreversible electroporation of the tissue.

Also described herein is an exemplary, non-claimed method of treating a tissue in a patient that can include the step of applying a plurality of electrical pulses to the tissue, wherein the electrical pulses are configured to cause non-thermal irreversible electroporation of the tissue; measuring a current of the applied electrical pulses; administering an additional treatment to the subject in response to the subject, wherein the step of administering occurs 0 to 5 days after non-thermal irreversible electroporation when the current is less than 25A, and wherein the step of administering occurs 4-30 days after non-thermal irreversible electroporation when the current is between 25 A and 100 A. The additional treatment is selected from the group consisting of: tissue resection, thermal ablation, non-thermal ablation, chemotherapy, radiation therapy, immunotherapy, biologic therapy, genetic therapy, and combinations thereof. The additional treatment can be measuring the amount of a pro-inflammatory immune molecule or cell, a suppressive immune molecule or cell, or both in a bodily fluid or a biopsied tissue of the patient. The electrical pulses can be configured to cause non-thermal irreversible electroporation of the tissue. The plurality of electrical pulses can be applied through a plurality of electrodes inserted into the tissue.

The scope of the present invention is defined by the appended claims.

## BRIEF DESCRIPTION OF THE DRAWINGS

Further embodiments of the present disclosure will be readily appreciated upon review of the detailed description of its various embodiments, described below, when taken in conjunction with the accompanying drawings.

**FIG. 1** is a graph that can demonstrate imputed median primary tumor volumes ($mm^3$) for each group of mice, showing significantly smaller median tumor size in the treated immunocompetent (IC) BALB/c mice.

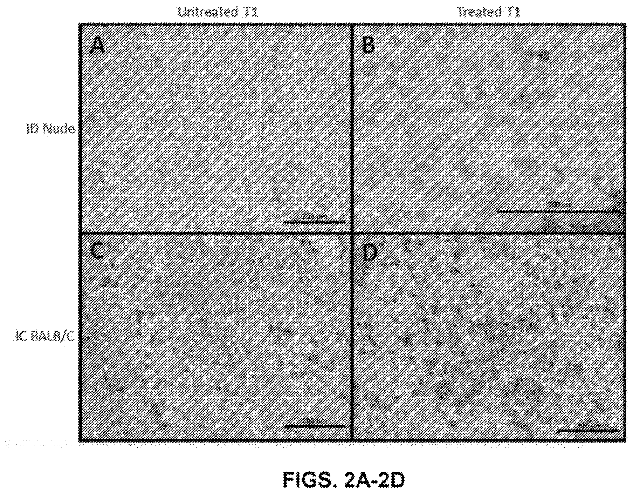

**FIGS. 2A-2D** are microscopic images showing CD3+ staining, which is indicative for T-cell presences, performed for **(FIGS. 2A and 2C)** untreated and **(FIGS. 2B and 2D)** treated initial T1 tumors between **(FIGS. 2A and 2B)** ID nude and **(FIGS. 2C and 2D)** IC BALB/c mice. There was no notable difference observed in CD3+ infiltration for ID nude mice between **(FIG. 2A)** untreated and **(FIG. 2B)** treated tumors. For the IC BALB/c mice, a robust increase in CD3+ (T-cell) infiltration is observed in treated tumors (**FIG. 2D**) relative to untreated T1 controls (**FIG. 2C**). Increased T-cell presence in treated T1 IC mice was also more robust than for both groups for nude mice (**FIGS. 2A and 2B**). All scale bars 200 mm. Panels **(FIGS. 2A, 2C, and 2D)** 200x, panel (**FIG. 2B**) 400x magnification

**FIG. 3** is a graph that can demonstrate percent CD4+CD25+FoxP3+ of total T cells 24 h post-IRE vs. difference in current (n=7).

**FIG. 4** is a graph that can demonstrate percent CD4+CD25+ cells 24 h post-irreversible electroporation (IRE) vs. difference in current (n=7).

**FIG. 5** is a graph that can demonstrate percent CD4+CD25+ of total T cells 24 h post-IRE vs. average current.

**FIG. 6** is a graph that can demonstrate the percent change in CD4+CD25+ and CD4+CD25+FoxP3+ 24 hours after IRE. The changes are shown in order of increasing overall change in output current (lower most panel) during the treatment in each patient. Populations were calculated as a subset of total CD4+ cells (n=7).

**FIG. 7** is a representative 3D reconstruction of a human pancreas, tumor, and vasculature used for treatment planning purposes. Two electrodes are inserted into the tumor in this example. Pancreas and vasculature reconstruction was prepared using 3matic and Gmsh software using a pre-operative CT scan; electrodes and tumor mimic placed using COMSOL software.

**FIG. 8** is a graph that can demonstrate a representative IRE pulse delivered using the Nanoknife® device. The resistance value resulting from the applied voltage and current is displayed to the user during treatment.

**FIG. 9** is a graph that can demonstrate the percent change in Regulatory T-cell subpopulations i.e. CD4+CD25+, CD4+CD25+FoxP3+, and CD4+CD25+FoxP3-, after 24 hours following IRE treatment. Populations were calculated as a subset of total CD4+ cells. *n=7*

**FIGS. 10A-10B** are graphs that can demonstrate the change in **FIG. 10A**) CD4+CD25+FoxP3+ and **FIG. 10B**) CD4+CD25+ after 24 hours following IRE decreases linearly with the range of current delivered during the IRE treatment for n=7 patients. A linear regression was performed using JMP Pro software.

**FIGS. 11A-11 C** are CT scan images of the pelvis and hind limbs visualizing the target region, practically surrounding the femur and adjacent to the sciatic nerve and femoral arteries. IRE resulted in marked influx of mixed inflammatory cells into the treatment region (**FIG. 11B**), which was composed of primarily CD3+ lymphocytes, 24 hours post-treatment (**FIG.11A→** pretreatment, **FIG. 11A→** post treatment).

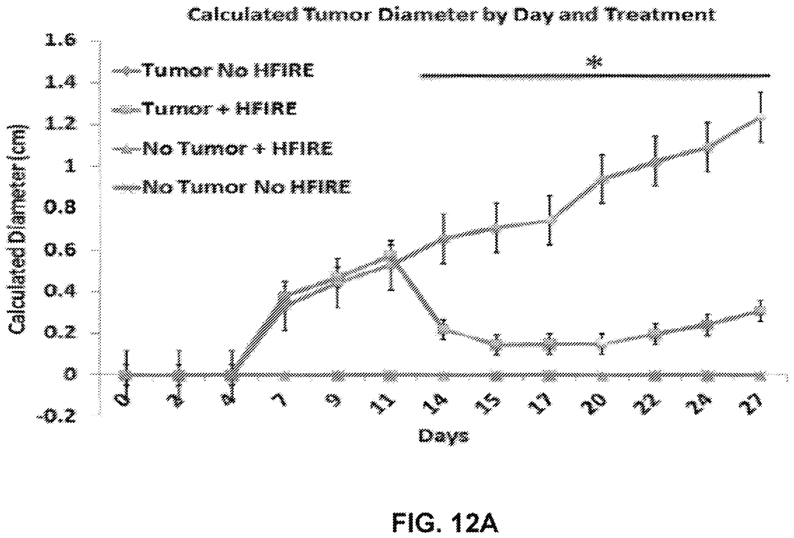

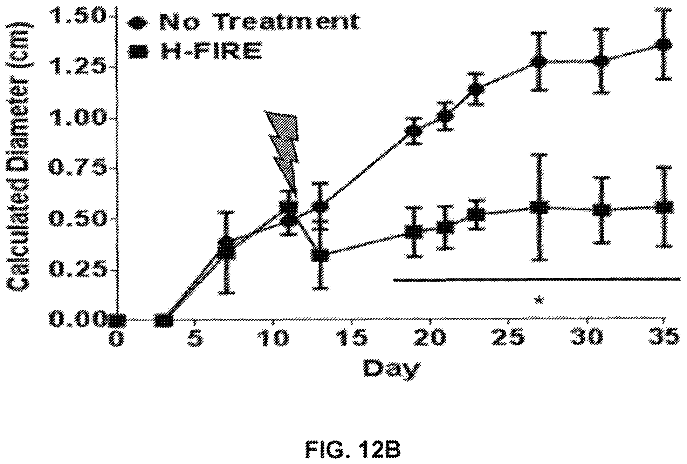

**FIGS. 12A-12B** are graphs that can demonstrate that H-FIRE treatment significantly reduces tumor progression and ablates the primary 4T1 mammary tumor. **(FIG. 12A)** Mice were injected with 1.26 X 106 4T1 cells directly into mammary tissue. On day 11, H-FIRE was administered. Clinical and tumor progression were monitored daily through day 27 where the tumors reached about 1.6 cm in diameter in the untreated mice. n=10 mice per treatment group. *p<0.05. **(FIG. 12B)** Mice were injected with 1.26 X 106 Pan02 cells subcutaneously (s.c.) and tumors were allowed to progress for 10 days. On day 11 (0.5 cm), H-FIRE was administered. Clinical and tumor progression were monitored daily through day 35 where the tumors reached about 1.35 cm in diameter in the untreated mice. n=3 mice per treatment group. *p<0.05.

**FIGS. 13A-13B** are graphs that can demonstrate a decrease in circulating metastases following H-FIRE treatment of the primary tumor in immunocompetent mice. Circulating metastases were quantified in both **(FIG. 13A)** BALB/c and **(FIG. 13B)** NSG mice following treatment of the primary 4T1 (breast cancer) tumor with an ablation dose of H-FIRE. Following necropsy, blood samples were plated in media with 6mM 6-thioguanine. After 10-14 days, formed colonies were counted. n= 6-10 mice. *p=0.05.

**FIG. 14** is a microscopic image that can demonstrate increased lymphocyte infiltration following H-FIRE in the local Pan02 tumor. Histopathology assessments revealed increased marginal and intra-tumor lymphocytes 14 days post-treatment with an ablation dose of H-FIRE.

**FIG. 15** is a schematic of a gene expression pathway analysis of tumor microenvironment following H-FIRE. Gene expression profiling revealed significant changes in inflammatory signaling in the 4T1 mammary tumor model. Data indicate a significant shift in the tumor microenvironment to a Th1/Th17 profile and high levels of IL-6 following full ablation. A significant increase in IL-2 signaling was found in tumors that received a sub-ablation dose of H-FIRE, consistent with increased lymphocyte expansion and recruitment.

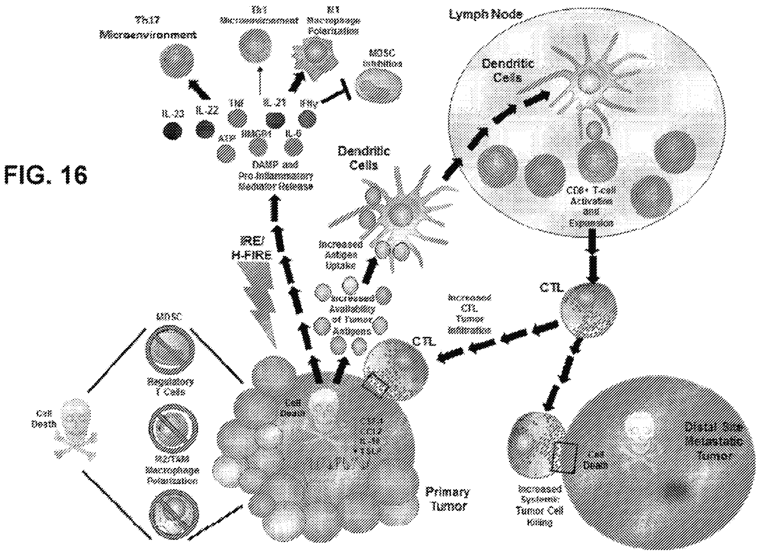

**FIG. 16** is a schematic of the local and systemic efficacy of IRE and H-FIRE ablation is associated with immune system activation/promotion. In this model, the cell death and tumor ablation driven by IRE/H-FIRE treatment promotes the innate immune system and promotes a shift in the tumor microenvironment from immunosuppressive to pro-inflammatory (Th1 and Th17), which serves to recruit increased numbers of antigen presenting cells to the local tumor. The cell death and damage associated with treatment increases antigen availability and presentation, further engaging the adaptive immune system. Increased tumor specific antigen presentation promotes T cell activation and increased CTL mediated cancer cell killing in the primary tumor and metastatic cells in distal locations. Increased activation/promotion of the adaptive immune system will also improve immunological memory, which should attenuate recurrence. IRE and H-FIRE would be excellent candidates for combination therapeutic approaches using new classes of checkpoint inhibitors and/or other immunomodulatory therapeutics.

**FIG. 17** is a schematic of H-FIRE targeting within a tumor.

**FIGS. 18A-18E** can demonstrate H-FIRE treatment and modeling in a 3D tumor mimic, where **FIG. 18A** is a photograph showing an experimental setup with electrodes inserted into tissue mimic and **FIGS. 18B-18E** are photographs showing live /dead staining reveals regions of the tissue impacted following 80 bursts containing **(FIG. 18B)** 2, **(FIG. 18C)** 24, and **(FIG. 18D)** 50 bipolar 2$\mu$s pulses with a 2 $\mu$s delay between alternating pulses. **(FIG. 18E)** Diffuse treatment of 50 bipolar 2$\mu$s pulses with 20ms between alternating pulses. Scale bar=2mm.

**FIGS. 19A-19D** are graphs that can demonstrate that IRE enhances the pro-inflammatory tumor microenvironment in Pan02 cells. **FIG. 19A)** Viability of cells after overnight incubation measured using LDH following 99 pulses at increasing field strengths. **FIGS. 19B-19D)** Influence of electric pulse parameters on select gene expression in Pan02 cells measured with real time PCR (ddCt). **(FIG. 19B)** Il-6, **(FIG. 19C)** Tslp, **(FIG. 19D)** Ccl-2. (n=3 repetitions; *, **, +, #p<0.05 ANOVA with Tukeys post-test).

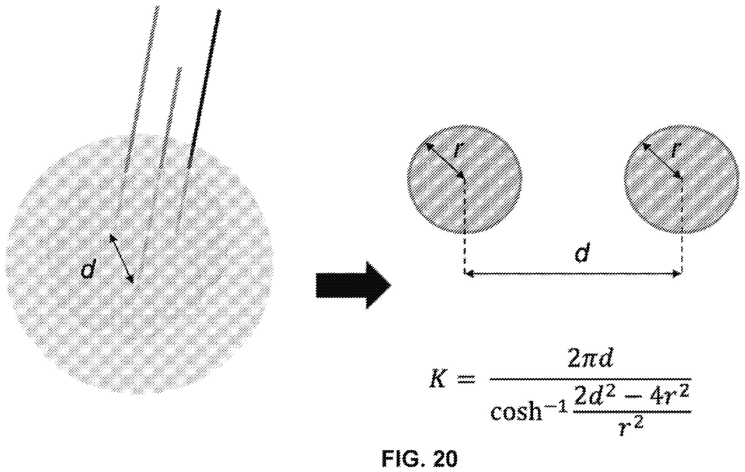

**FIG. 20** is a schematic showing the appropriate shape factor calculation for voltage applied across two needle electrodes. An example three needle configuration is shown. During IRE, pulses are delivered across probe pairs, therefore only two will act as source and sink at any given point. Bulk tissue conductivity is calculated using this shape factor and the resistance according to Ohm's Law (voltage equals current times resistance). The relationship is given by $R = 1/(\sigma *K)$ where $R$ is resistance ($\Omega$), $\sigma$ is the conductivity (S/m), and $K$ is the shape factor given in **FIG. 20** where l is the spacing between the electrodes and $r$ is the electrode radius.

**FIG. 21** is a table that shows three example shape factor calculations assuming a static, bulk tissue conductivity of 0.2 S/m. The changes in this conductivity value result in changes in the output current. Additionally, changes in the electrode and/or voltage parameters affects the output current value as in the examples shown; $V$ is applied voltage, is $\sigma$ the bulk electrical conductivity, $d$ is the spacing between the electrodes, $r$ is the radius of the electrodes, $K$ is the shape factor (dimensionless), and $I$ is output current.

**FIG. 22** is a scatter plot of gene expression array data illustrating the results from **FIG. 34.** CXCL10 and HIF1A expression is significantly up-regulated and TLR2, SPP1 and MYC are significantly down-regulated following H-FIRE treatment in all 3 patients compared to baseline. Change in expression of all other genes was less than 2-fold (unchanged) and represented by the solid black line

**FIG. 23** is an image showing results of an Ingenuity Pathway Analysis (IPA) of global changes in gene expression patterns following H-FIRE in 3 patients. Results revealed diverse, but functionally related predications in canonical pathways significantly increased by H-FIRE for Patients 1 and 2. Conversely, the gene expression profile for Patient 3 showed either no change or down-regulation of functionally similar canonical pathways.

**FIGS. 24A-24F** are graphs showing results from the top 6 canonical pathways impacted by H-FIRE, comparing pre-treatment to post-treatment, ranked by z-score. Patient 1 and 2 are highly consistent, with patient 3 demonstrating opposing results.

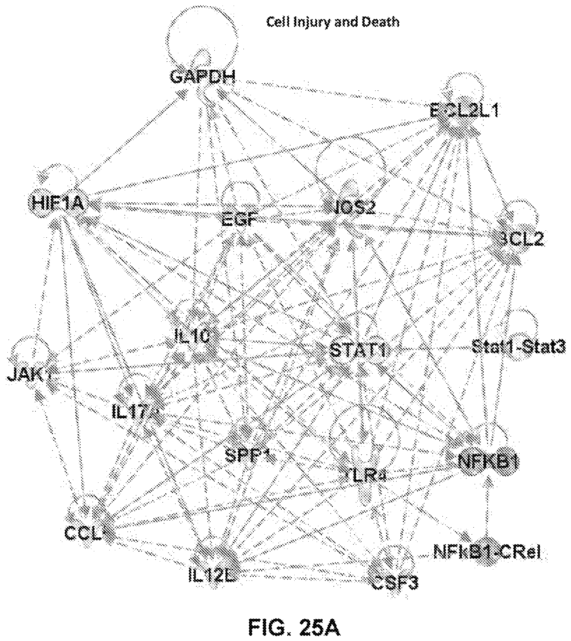

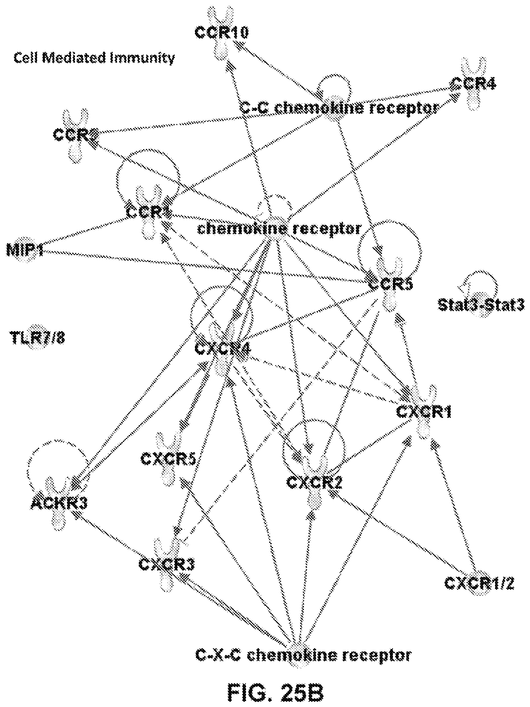

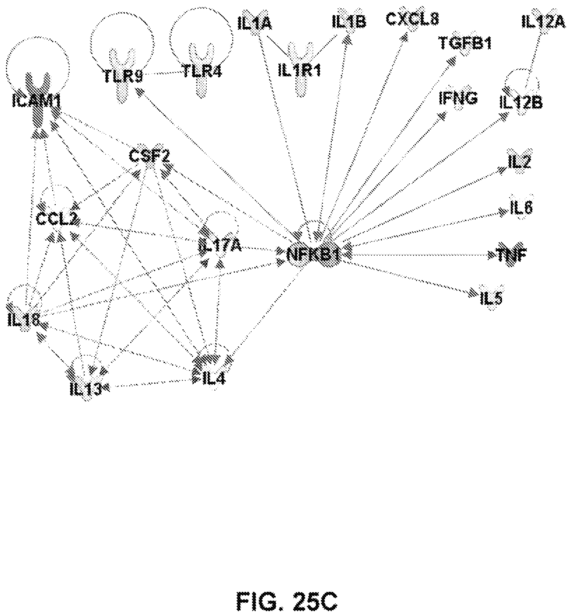

**FIGS. 25A-25C** are diagrams showing pathways associated with the activation of cellular immunity **(FIG. 25B)** and cell death **(FIG. 25A)** were significantly up-regulated following H-FIRE treatment. **FIG. 25A.** IPA network analysis identified 2 functional networks that fit the canonical pathways identified above, cell injury/death and cell mediated immunity. Both of these networks were significantly upregulated in all patients following H-FIRE treatment **FIG. 25C.** NF-κB signaling was one of the most dominate pathways impacted by H-FIRE treatment. Gene expression patterns revealed a significant global up-regulation in NF-κB signaling.

**FIG. 26A** is a schematic of HFIRE experimental set-up.

**FIG. 26B** is a rendering of a standard IRE voltage waveform pulse.

**FIG. 26C** is a photograph of an 18-gauge, bipolar electrode.

**FIG. 26D** is a rendering of a HFIRE 2-5-2 voltage waveform burst.

**FIG. 27** is a schematic of the 3D-reconstruction and numerical modeling process for patient 1.

**FIG. 28A** is a photograph showing placement of the bipolar treatment probe into the tumor under ultrasound guidance.

**FIG. 28B** is an ultrasound image of the bipolar treatment probe inserted into the tumor during treatment delivery; arrow - HFIRE bipolar electrode.

**FIG. 29** is a panel of CT images Pre- (left) and post-treatment (center) and images of gross tumor samples (right). CT images from all three patients showing predictable ablation volume (outlined) resulting from H-FIRE treatment. This correlated with the ablation volume noted in gross tumor samples (right).

**FIG. 30** is a panel of images of tumor histopathology from patient 1 (top) and patient 2 (bottom) showing the well-defined ablation/tumor interface (arrows) with H&E at 40X (left) and 100X (center). IHC for CD3 revealed positive staining cells infiltrating the tumor-ablation interface. Untreated HCC is denoted by (*) and the ablation zone by the bolt.

**FIG. 31** is a panel of images of tumor histopathology from patient 3 showing absence of a well-defined ablation/tumor interface (arrows) with H&E at 40X (left) and 100x (center). IHC for CD3 (right) shows the lack of CD3+ cells within the ablation/tumor interface. Untreated HCC is denoted by (*) and the ablation zone by the bolt.

**FIG. 32** is a panel of images showing IHC results for CD3 (left), CD4 (center) and CD8 (right) on tumor samples from patient 1 (top) and patient 2 (bottom) showing infiltration of the ablation/tumor interface (arrows) with CD3+/CD4-/CD8- lymphocytes. Untreated HCC is denoted by (*) and the ablation zone by the bolt.

**FIGS. 33A-33B** are graphs that can demonstrate that ALT **(FIG. 33A)** and ALP **(FIG. 33B)** increased following H-FIRE treatment (bolt), but resolved over time following tumor removal (day 4).

**FIG. 34** is a table showing tissue and probe properties employed for numerical modeling.

**FIG. 35** is a table showing mean (n = 3) fold change in gene expression following H-FIRE treatment compared to baseline. Only genes with significant changes in expression are shown.

**FIG. 36** is a block diagram of an electroporation device according to embodiments of the present invention.

**FIG. 37** is a block diagram of a treatment control computer of **FIG. 36.**

**FIG. 38** is a block diagram of a pulse generator shown in **FIG. 36.**

**FIG. 39** is a block diagram of a sensor of **FIG. 38.**

**FIG. 40** is a flow chart depicting a method according to an embodiment.

## DETAILED DESCRIPTION

Before the present disclosure is described in greater detail, it is to be understood that this disclosure is not limited to particular embodiments described, and as such may, of course, vary. It is also to be understood that the terminology used herein is for the purpose of describing particular embodiments only, and is not intended to be limiting.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

As will be apparent to those of skill in the art upon reading this disclosure, each of the individual embodiments described and illustrated herein has discrete components and features which may be readily separated from or combined with the features of any of the other several embodiments. Any recited method can be carried out in the order of events recited or in any other order that is logically possible.

Where a range is expressed, embodiments include from the one particular value and/or to the other particular value. Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure. For example, where the stated range includes one or both of the limits, ranges excluding either or both of those included limits are also included in the disclosure, e.g. the phrase "x to y" includes the range from 'x' to 'y' as well as the range greater than 'x' and less than 'y'. The range can also be expressed as an upper limit, e.g. 'about x, y, z, or less' and should be interpreted to include the specific ranges of 'about x', 'about y', and 'about z' as well as the ranges of 'less than x', less than y', and 'less than z'. Likewise, the phrase 'about x, y, z, or greater' should be interpreted to include the specific ranges of 'about x', 'about y', and 'about z' as well as the ranges of 'greater than x', greater than y', and 'greater than z'. In addition, the phrase "about 'x' to 'y'", where 'x' and 'y' are numerical values, includes "about 'x' to about 'y'".

It should be noted that ratios, concentrations, amounts, and other numerical data can be expressed herein in a range format. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. Ranges can be expressed herein as from "about" one particular value, and/or to "about" another particular value. Similarly, when values are expressed as approximations, by use of the antecedent "about," it will be understood that the particular value forms a further embodiment. For example, if the value "about 10" is disclosed, then "10" is also disclosed.

It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a numerical range of "about 0.1% to 5%" should be interpreted to include not only the explicitly recited values of about 0.1% to about 5%, but also include individual values (e.g., about 1%, about 2%, about 3%, and about 4%) and the sub-ranges (e.g., about 0.5% to about 1.1%; about 5% to about 2.4%; about 0.5% to about 3.2%, and about 0.5% to about 4.4%, and other possible sub-ranges) within the indicated range.

As used in the specification and the appended claims, the singular forms "a," "an," and "the" include plural referents unless the context clearly dictates otherwise.

As used herein, "about," "approximately," "substantially," and the like, when used in connection with a numerical variable, can generally refers to the value of the variable and to all values of the variable that are within the experimental error (e.g., within the 95% confidence interval for the mean) or within +/- 10% of the indicated value, whichever is greater. As used herein, the terms "about," "approximate," "at or about," and "substantially" can mean that the amount or value in question can be the exact value or a value that provides equivalent results or effects as recited in the claims or taught herein. That is, it is understood that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art such that equivalent results or effects are obtained. In some circumstances, the value that provides equivalent results or effects cannot be reasonably determined. In general, an amount, size, formulation, parameter or other quantity or characteristic is "about," "approximate," or "at or about" whether or not expressly stated to be such. It is understood that where "about," "approximate," or "at or about" is used before a quantitative value, the parameter also includes the specific quantitative value itself, unless specifically stated otherwise.

Embodiments of the present disclosure will employ, unless otherwise indicated, techniques of immunology, molecular biology, electrophysiology, physiology, cell biology, cancer biology, and the like, which are within the skill of the art. Such techniques are explained fully in the literature.

Before the embodiments of the present disclosure are described in detail, it is to be understood that, unless otherwise indicated, the present disclosure is not limited to particular materials, reagents, reaction materials, manufacturing processes, or the like, as such can vary. It is also to be understood that the terminology used herein is for purposes of describing particular embodiments only, and is not intended to be limiting. It is also possible in the present disclosure that steps can be executed in different sequence where this is logically possible unless the context clearly dictates otherwise.

*Definitions*

As used herein, "active agent" or "active ingredient" refers to a substance, compound, or molecule, which is biologically active or otherwise, induces a biological or physiological effect on a subject to which it is administered to. In other words, "active agent" or "active ingredient" refers to a component or components of a composition to which the whole or part of the effect of the composition is attributed.

As used herein, "administering" refers to an administration that is oral, topical, intravenous, subcutaneous, transcutaneous, transdermal, intramuscular, intra-joint, parenteral, intra-arteriole, intradermal, intraventricular, intraosseous, intraocular, intracranial, intraperitoneal, intralesional, intranasal, intracardiac, intraarticular, intracavernous, intrathecal, intravireal, intracerebral, and intracerebroventricular, intratympanic, intracochlear, rectal, vaginal, by inhalation, by catheters, stents or via an implanted reservoir or other device that administers, either actively or passively (e.g. by diffusion) a composition the perivascular space and adventitia. For example a medical device such as a stent can contain a composition or formulation disposed on its surface, which can then dissolve or be otherwise distributed to the surrounding tissue and cells. The term "parenteral" can include subcutaneous, intravenous, intramuscular, intra-articular, intra-synovial, intrasternal, intrathecal, intrahepatic, intralesional, and intracranial injections or infusion techniques. In some embodiments, administering can be achieved by any suitable mechanism, technique, and/or device. In some embodiments, administering can be intravenous through a catheter and/or needle.

As used herein, "agent" refers to any substance, compound, molecule, and the like, which can be biologically active or otherwise can induce a biological and/or physiological effect on a subject to which it is administered to. An agent can be a primary active agent, or in other words, the component(s) of a composition to which the whole or part of the effect of the composition is attributed. An agent can be a secondary agent, or in other words, the component(s) of a composition to which an additional part and/or other effect of the composition is attributed.

As used herein "a change in bulk tissue conductivity" can refer to either a change in the measured difference in current, the change in the measured average current, or a change in any other measured parameter of the conductivity within the tissue.

As used herein "cancer" can refer to one or more types of cancer including, but not limited to, acute lymphoblastic leukemia, acute myeloid leukemia, adrenocortical carcinoma, Kaposi Sarcoma, AIDS-related lymphoma, primary central nervous system (CNS) lymphoma, anal cancer, appendix cancer, astrocytomas, atypical teratoid/Rhabdoid tumors, basal cell carcinoma of the skin, bile duct cancer, bladder cancer, bone cancer (including but not limited to Ewing Sarcoma, osteosarcomas, and malignant fibrous histiocytoma), brain tumors, breast cancer, bronchial tumors, Burkitt lymphoma, carcinoid tumor, cardiac tumors, germ cell tumors, embryonal tumors, cervical cancer, cholangiocarcinoma, chordoma, chronic lymphocytic leukemia, chronic myelogenous leukemia, chronic myeloproliferative neoplasms, colorectal cancer, craniopharyngioma, cutaneous T-Cell lymphoma, ductal carcinoma in situ, endometrial cancer, ependymoma, esophageal cancer, esthesioneuroblastoma, extracranial germ cell tumor, extragonadal germ cell tumor, eye cancer (including, but not limited to, intraocular melanoma and retinoblastoma), fallopian tube cancer, gallbladder cancer, gastric cancer, gastrointestinal carcinoid tumor, gastrointestinal stromal tumors, central nervous system germ cell tumors, extracranial germ cell tumors, extragonadal germ cell tumors, ovarian germ cell tumors, testicular cancer, gestational trophoblastic disease, hairy cell leukemia, head and neck cancers, hepatocellular (liver) cancer, Langerhans cell histiocytosis, Hodgkin lymphoma, hypopharyngeal cancer, islet cell tumors, pancreatic neuroendocrine tumors, kidney (renal cell) cancer, laryngeal cancer, leukemia, lip cancer, oral cancer, lung cancer (non-small cell and small cell), lymphoma, melanoma, Merkel cell carcinoma, mesothelioma, metastatic squamous cell neck cancer, midline tract carcinoma with and without NUT gene changes, multiple endocrine neoplasia syndromes, multiple myeloma, plasma cell neoplasms, mycosis fungoides, myelodyspastic syndromes, myelodysplastic/myeloproliferative neoplasms, chronic myelogenous leukemia, nasal cancer, sinus cancer, non-Hodgkin lymphoma, pancreatic cancer, paraganglioma, paranasal sinus cancer, parathyroid cancer, penile cancer, pharyngeal cancer, pheochromocytoma, pituitary cancer, peritoneal cancer, prostate cancer, rectal cancer, Rhabdomyosarcoma, salivary gland cancer, uterine sarcoma, Sézary syndrome, skin cancer, small intestine cancer, large intestine cancer (colon cancer), soft tissue sarcoma, T-cell lymphoma, throat cancer, oropharyngeal cancer, nasopharyngeal cancer, hypopharyngeal cancer, thymoma, thymic carcinoma, thyroid cancer, transitional cell cancer of the renal pelvis and ureter, urethral cancer, uterine cancer, vaginal cancer, cervical cancer, vascular tumors and cancer, vulvar cancer, and Wilms Tumor.

As used herein, "chemotherapeutic agent" or "chemotherapeutic" refers to a therapeutic agent utilized to prevent or treat cancer.

As used herein, "control" can refer to an alternative subject or sample used in an experiment for comparison purpose and included to minimize or distinguish the effect of variables other than an independent variable.

As used herein, "differentially expressed," refers to the differential production of RNA, including but not limited to mRNA, tRNA, miRNA, siRNA, snRNA, and piRNA transcribed from a gene or regulatory region of a genome or the protein product encoded by a gene as compared to the level of production of RNA or protein by the same gene or regulator region in a normal or a control cell. In another context, "differentially expressed," also refers to nucleotide sequences or proteins in a cell or tissue which have different temporal and/or spatial expression profiles as compared to a normal or control cell.

As used herein, "dose," "unit dose," or "dosage" can refer to physically discrete units suitable for use in a subject, each unit containing a predetermined quantity of a pharmaceutical formulation or immunotherapy thereof calculated to produce the desired response or responses in association with its administration.

As used herein, "immunomodulator," refers to an agent, such as a therapeutic agent, which is capable of modulating or regulating one or more immune function or response.

As used herein, "mammal," for the purposes of treatments, can refer to any animal classified as a mammal, including human, domestic and farm animals, nonhuman primates, and zoo, sports, or pet animals, such as, but not limited to, dogs, horses, cats, and cows.

As used herein, "organism", "host", and "subject" refers to any living entity comprised of at least one cell. A living organism can be as simple as, for example, a single isolated eukaryotic cell or cultured cell or cell line, or as complex as a mammal, including a human being, and animals (e.g., vertebrates, amphibians, fish, mammals, e.g., cats, dogs, horses, pigs, cows, sheep, rodents, rabbits, squirrels, bears, primates (e.g., chimpanzees, gorillas, and humans).

As used herein, the terms "optional" or "optionally" means that the subsequently described event or circumstance can or cannot occur, and that the description includes instances where said event or circumstance occurs and instances where it does not.

As used herein, "patient" can refer to an organism, host, or subject in need of treatment.

As used herein, "pharmaceutical formulation" refers to the combination of an active agent, compound, or ingredient with a pharmaceutically acceptable carrier or excipient, making the composition suitable for diagnostic, therapeutic, or preventive use in vitro, in vivo, or ex vivo.

As used herein, "preventative" and "prevent" refers to hindering or stopping a disease or condition before it occurs, even if undiagnosed, or while the disease or condition is still in the subclinical phase.

As used interchangeably herein, the terms "sufficient" and "effective," can refer to an amount (e.g. mass, volume, dosage, concentration, and/or time period) needed to achieve one or more desired result(s). For example, a therapeutically effective amount refers to an amount needed to achieve one or more therapeutic effects.

A "suitable control" is a control that will be instantly appreciated by one of ordinary skill in the art as one that is included such that it can be determined if the variable being evaluated an effect, such as a desired effect or hypothesized effect. One of ordinary skill in the art will also instantly appreciate based on *inter alia,* the context, the variable(s), the desired or hypothesized effect, what is a suitable or an appropriate control needed.

As used herein, "therapeutic" can refer to treating, healing, and/or ameliorating a disease, disorder, condition, or side effect, or to decreasing in the rate of advancement of a disease, disorder, condition, or side effect. A "therapeutically effective amount" can therefore refer to an amount of a compound that can yield a therapeutic effect.

As used herein, the terms "treating" and "treatment" can refer generally to obtaining a desired pharmacological and/or physiological effect. The effect can be therapeutic in terms of a partial or complete cure of a disease, condition, symptom or adverse effect attributed to the disease, disorder, or condition. The term "treatment" as used herein covers any treatment of a tumor or a cancer in a subject, particularly a human, and can include any one or more of the following: (a) inhibiting the disease, i.e., arresting its development; and (b) relieving the disease, i.e., mitigating or ameliorating the disease and/or its symptoms or conditions. The term "treatment" as used herein can refer to both therapeutic treatment alone, prophylactic treatment alone, or both therapeutic and prophylactic treatment. Those in need of treatment (subjects in need thereof) can include those already with the disorder and/or those in which the disorder is to be prevented. As used herein, the term "treating", can include inhibiting the disease, disorder or condition, e.g., impeding its progress; and relieving the disease, disorder, or condition, e.g., causing regression of the disease, disorder and/or condition. Treating the disease, disorder, or condition can include ameliorating at least one symptom of the particular disease, disorder, or condition, even if the underlying pathophysiology is not affected, such as treating the pain of a subject by administration of an analgesic agent even though such agent does not treat the cause of the pain.

As used herein, "non-thermal ablation" refers to any technique that can result in the destruction and/or death of cells, cellular structures, cellular components, and/or tissue, including human tissue and non-human tissue, within and surrounding a target site, while preserving the extracellular matrix, nerves, major blood vessels, and other sensitive structures of the treated tissues, without raising the temperature of the cells or treated tissues local to and surrounding the target site of ablation to an overall temperature over about 65 °C at the conclusion of pulse delivery and those cells that do not die but are still affected by the treatment. In some embodiments, "non-thermal ablation" includes techniques that involve stimulating, killing or altering cellular components of cells and/or tissue with pulsed electrical energy that does not raise the temperature of the cells and/or tissue over about 65 °C at the conclusion of pulse. Non-thermal ablation techniques include, but are not limited to irreversible electroporation (IRE), high frequency irreversible electroporation (HFIRE or H-FIRE) supraporation, reversible electroporation, histotripsy (focused ultrasound), shock wave therapies, high-intensity focused ultrasound (HIFU), and ultrasonic ablation. Techniques and parameters of operation of non-thermal ablation techniques, including HFIRE and IRE can be found in U.S. Pat. No.: 8,926,606, U.S. Pat. Pub. 2012/0109122, and U.S. Pat. Pub.: 2014/0039489. Irreversible electroporation (IRE) treatment pulses are typically monopolar, monophasic, and are delivered in a single polarity. High frequency irreversible electroporation (HFIRE or H-FIRE) treatment pulses are typically bipolar, biphasic, and are delivered in alternating polarity. Also, HFIRE pulses typically are delivered at a higher frequency rate compared to IRE pulses frequency rate.

As used herein, "thermal ablation" refers to any technique that can result in the destruction and/or death of cells, cellular structures, cellular components, and/or tissue, including human tissue and non-human tissue, within and surrounding a target site, by transmitting energy, sufficient to increase the overall temperature of the tissue and/or cells within or surrounding a target site to above 65°C. Thermal ablation techniques can include but are not limited to electrical energy, microwave ablation, a radiofrequency (RF) ablation, dual thermal ablation, and laser ablation.

As used herein, "ablation" without further qualification refers to both thermal and non-thermal ablation.

As used herein, "immunosuppressive treatment" refers to any treatment modality, including, but not limited to, thermal ablation, resection, and/or chemotherapy, that do not allow for the innate and/or adaptive immune responses to active, peak, and/or come to completion.

As used herein, "non-immunosuppressive treatment" refers to any treatment modality, including, but not limited to, non-thermal ablation, such as IRE and/or HFIRE, that allows for the innate and/or adaptive immune responses to active, peak, and/or come to completion.

### *Discussion*

Some types of cancers come with the most dismal prognoses, particularly after metastasis has already occurred. In some cases, tumor resection and/or thermal ablation is not a viable option for therapy. Further, while delivery of chemotherapeutics is often part of a standard cancer therapy regimen or protocol, they may also be immunosuppressive, and thus can reduce the patient's own immune system's ability to combat the cancer and mute or otherwise suppress the patient's biological immune defense mechanisms. Additionally, tumors in some areas, such as immunotolerant organs and systems (e.g. liver, gastrointestinal organs, respiratory system, genitourinary tract), are not affected as much by the patient's immune system because its immediate microenvironment is effectively immunosuppressed. The immunosuppressive microenvironment characteristic in immunotolerant organs are characterized by a balance between pro-inflammatory and anti-inflammatory mediators produced by specialized immune cells that reside in these organs and serve to protect the system from overzealous immune responses that can lead to diseases, such as those associated with autoimmunity. However, this immunosuppression can promote tumorigenesis and become a hindrance to certain therapeutics, such as checkpoint inhibitor therapies, that function through activating the immune system. This immunosuppression in both healthy immunotolerant organs and in tumors is, in part, mediated by groups of immunosuppressive cells, including regulatory T cells (Treg), tumor associated macrophages (TAM), tumor associated neutrophils (TAN), and myeloid derived suppressor cells (MDSC). Based on the location and type of tumor, in many cases, tumor resection is immediately considered as a viable treatment option followed by chemotherapy and other modalities, such as thermal ablation. As stated above, the use of resection, chemotherapy, and thermal ablation may reduce a subject's or patient's ability to capitalize on their own immune response.

Cancer immunotherapy, which involves eliciting a host immune response that can result in tumor regression has been an increasingly compelling field of interest. Much focus in this field has been on the vaccination against various cancer types by injecting cancer antigens and thus stimulating the host's immune system to target the cancer. A more recent area of investigation for inducing anti-tumor immunity is based on the direct destruction of cancerous tumors by ablative methods. By treating cancer using ablative approaches, the tumor may undergo a multi-agent anti-cancer treatment or vaccine through the production of tumor antigens resulting from the destructed tissue. Although some efficacy in some patients has been achieved, the invention described herein provides for the ability to optimize patient treatment planning and improve treatment outcome at the individual patient level. Most common cancer treatments, including, but not limited to, thermal ablation, resection, and delivery of chemotherapies limit or hinder the natural biological immune response and what is needed in the art is a primary treatment modality of non-thermal ablation, such as IRE or HFIRE, that targets the destruction of tumor cells and/or tissue at the target site and the surrounding area, while also supporting, maintaining, and enhancing the natural biological immune response.

After treatment of a target site, including, but not limited to, thermal ablation, non-thermal ablation, resection, chemotherapy, radiation, and other modalities, a human patient will have both an innate immune response and an adaptive immune response. While the innate immune response peaks a couple of days (up to 72 hours or more) post treatment e.g. IRE, the adaptive immune response takes longer to promote (about 10-21 days). The adaptive immune response can destroy circulating and/or metastatic cancer cells. This peak or total response time for both the innate and adaptive immune responses are important factors when considering an overall treatment plan for a patient. As disclosed herein, and described in greater detail below, one advantage of using non-thermal ablation, such as IRE or HFIRE, as a primary modality for treatment of tumors is to allow the patient's innate and adaptive immune responses sufficient time to peak or respond. In other words, non-thermal ablation, such as IRE and HFIRE, may be described herein as a form of non-immunosuppressive treatment modalities. A non-immunosuppressive treatment modality is a form of treatment that allows for the innate and adaptive immune response to active, peak, and/or come to completion, thereby providing the patient with the important immune response defense to the tumor. Conversely, common treatment modalities used in the art today, including, but not limited to, thermal ablation, resection, and/or chemotherapy are considered immunosuppressive treatments, meaning they do not allow for the innate and adaptive immune responses to active, peak, and/or come to completion, thereby depriving the patient of important immune response defense.

Depending on the tumor type and tissue location of the tumor, it is common practice for an overall treatment plan to include delivery of multiple forms of treatment modalities to a patient, including, but not limited to, thermal ablation, resection, and/or chemotherapy. If any of these treatment modalities, such as thermal ablation, resection, and/or chemotherapy, is completed before the innate immune response can peak and trigger the adaptive immune response, then the subject may not benefit from this overall immune response effect. As described above, these treatment modalities may be considered immunosuppressive. If any of these treatment modalities, such as thermal ablation, resection, and/or chemotherapy, is completed before the innate immune response can peak and trigger the adaptive immune response, then the subject may not benefit from this overall immune response effect. As described above, these treatment modalities may be considered immunosuppressive. Moreover, if a non-immunosuppressive treatment modality, such as non-thermal ablation using IRE or HFIRE, is followed by an immunosuppressive treatment modality, such as thermal ablation, resection, and/or chemotherapy, without waiting a sufficient amount of time this secondary immunosuppressive treatment modality may impede the innate and/or adaptive immune response. Therefore, there is a need in the art to confirm that a sufficient delay in time, which is described below in greater detail, may be measured in terms of days, has passed after the primary non-immunosuppressive treatment has been delivered before any secondary immunosuppressive treatment is delivered. Improved immune system activation/promotion is expected to improve local tumor ablation, increased tumor surveillance, and increased targeting of metastatic lesions at sites distal to the primary tumor. If the delivery of an immunosuppressive treatment modality is given to a patient before the innate and/or adaptive immune responses active, peak, and/or come to completion, the patient may lose these benefits.

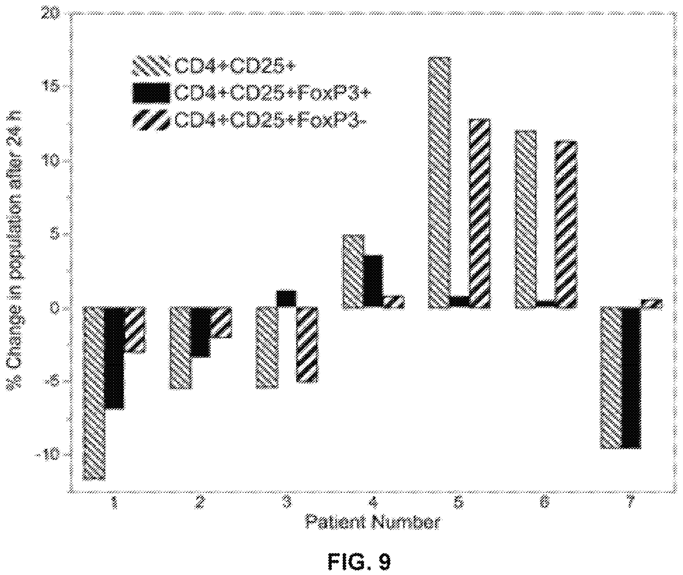

The present invention is defined by the appended claims. Methods described hereinafter are not part of the present invention but are an illustration of how the system of the present invention can be used. Described herein are exemplary treatment devices, diagnostic and treatment systems and exemplary methods of measuring, directly or indirectly, a treatment parameter, including, but not limited to, the bulk tissue conductivity using a suitable technique in real-time during a non-thermal ablation treatment and forming a treatment plan based on the measured the treatment parameter, such as the tumor bulk tissue conductivity of the tissue being treated. As shown in FIG. 9, the devices, systems, and methods described herein can result in patient stratification based on their ability to immunologically respond to non-thermal ablation treatment, which provides the opportunity for a clinician to prescribe customized precise care and improve treatment outcome. This treatment plan may include stopping the delivery of non-thermal ablation treatment pulses, such as stopping the delivery of electrical pulses used for IRE and/or HFIRE, before the non-thermal ablation is complete, and/or treating the subject with subsequent suitable treatment modalities, including, but not limited to, immunosuppressive treatment(s) and/or non-immunosuppressive treatment(s). The subsequent treatment(s) can be different than the current standard of care for that patient. In other words, the clinician can make different treatment decisions based on the real-time measurement of a treatment parameter, such as bulk tissue conductivity of the tissue being treated, a change in average current measured, or a change in the difference in current measured, during a non-thermal ablation treatment. As is discussed elsewhere herein the different treatment regimens that can be prescribed and applied can aim to take advantage of an innate and/or adaptive immune response that can be activated by the non-immunosuppressive non-thermal ablation treatment. In some instances, non-thermal ablation techniques may be used after immunosuppressive treatment efforts are delivered. The devices, systems, and methods described herein can be a departure from that standard paradigm and instead be used prior to down-stream treatments and co-therapies to provide a more precise and individualize treatment approach. Even when non-thermal ablation is already a first form of treatment, the devices, systems, and method described herein can be used to adjust and customize the patient's overall treatment protocol, thereby increasing the overall efficacy of secondary or downstream treatments.

Other compositions, compounds, methods, features, and advantages of the present disclosure will be or become apparent to one having ordinary skill in the art upon examination of the following drawings, detailed description, and examples. It is intended that all such additional compositions, compounds, methods, features, and advantages be included within this description.

Described herein is an exemplary, non-claimed method of treating a target site comprising target tissue (e.g. tumor and/or cancer) and/or target cells in a subject, that can include the step of measuring, directly or indirectly, a treatment parameter, including, but not limited to, bulk tissue conductivity, in real-time during a non-thermal ablation treatment and determining a downstream and/or secondary treatment regimen for the patient based on the measured treatment parameter, such as the bulk tissue conductivity, or change thereof during non-thermal ablation therapy. In some embodiments the non-thermal ablation treatment can be the delivery of electrical pulses capable of resulting in IRE and/or H-FIRE of the tissues and/or cells within and surrounding the target site. Other suitable non-thermal ablation treatments are described elsewhere herein. Moreover, the non-thermal ablation may also be a non-immunosuppressive treatment modality.

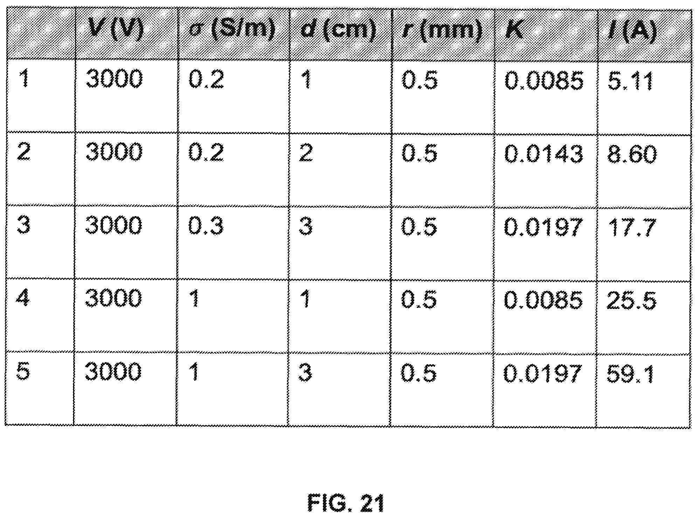

Using IRE as a model to discuss the specific treatment parameter of bulk tissue conductivity, attention is directed to FIG. 20 shows the appropriate shape factor calculation for voltage applied across two needle electrodes. An example three needle configuration is shown. During an IRE treatment, electrical pulses are delivered across at least a pair of electrodes, therefore only two electrodes may act as source and sink at any given point. Bulk tissue conductivity is calculated using this shape factor and the resistance according to Ohm's Law (voltage equals current times resistance). The relationship is given by $R = 1/(\sigma * K)$ where $R$ is resistance ($\Omega$), $\sigma$ is the conductivity (S/m), and $K$ is the shape factor given in **FIG. 20**, where $l$ is the spacing between the electrodes and $r$ is the electrode radius. **FIG. 21** shows a table that shows three example shape factor calculations assuming a static, bulk tissue conductivity of 0.2 S/m. The changes in this conductivity value result in changes in the output current. Additionally, changes in the electrode distance, size, and shape, and/or voltage and pulse parameters affects the output current value as in the output current value as in the examples shown; $V$ is applied voltage, is $\sigma$ the bulk electrical conductivity, $d$ is the spacing between the electrodes, $r$ is the radius of the electrodes, $K$ is the shape factor (dimensionless), and $I$ is output current. Although the change in amperage or absolute value in amperage that would indicate to a clinician to make a treatment decision, indicate that a patient would be a responder to immunotherapy, and/or apply a secondary treatment as described elsewhere herein, may change based on the specific device and treatment characteristics, based on those specific characteristics one of ordinary skill in the art will be able to calculate an equivalent change in amperage and/or threshold value to base a treatment decision, diagnosis, treatment application on.

In some embodiments, the clinician can determine a treatment strategy for a patient in response to a positive change in the treatment parameter. The positive change can be an increase in the absolute value in current, a percentage increase in the current, and/or a percentage increase or change in the bulk conductivity of the tissue. In some embodiments, the positive change can be an increase in the current to over 25 A. In some embodiments, the positive change can be an increase in the absolute value in the current to about 25-100 A. The amperage that is considered a positive change can vary based on, for example, the tissue and the characteristics of the device used (e.g. electrode length, distance apart, etc.). Based on the exact parameters used for treatment, one of skill in the art can calculate the appropriate amperage values that would guide a clinician in further treatment of the patient as described herein. This is discussed in greater detail elsewhere herein.

In some embodiments, the positive change can be in increase in the percentage change in current. In some embodiments, the percentage change can range from about 100% to about 400%. In some embodiments, the percentage change can be about 175%. In some embodiments, the positive change can be an increase or change in the tissue bulk conductivity such that tissue bulk conductivity ranges from about 0.7 S/m to about 1.5 S/m. In some embodiments, a positive change can be reached when the tissue bulk conductivity reaches about 1 S/m. In some embodiments, treatment is delayed for a period of time (e.g. 4-30 days) when a positive change in the treatment parameter occurs. In some times treatment that is different than the standard of care is taken when a positive change in the treatment parameter occurs. In some embodiments, immediate treatment action is taken when a positive change in the treatment parameter occurs. In some embodiments, patients that will respond to immunotherapy can be identified when a positive change in the treatment parameter occurs.

The downstream or secondary treatment regimen can be any suitable treatment modality and can include, but is not limited to, tumor resection, thermal ablation, a subsequent non-thermal ablation, chemotherapy, radiation therapy, immunotherapy, biologic therapy, genetic therapy (gene editing), and combinations thereof. In some embodiments, the subsequent downstream treatment(s) is/are delayed a period of time that can be at least 4-30 days after the initial non-thermal ablation treatment. This can allow both the innate and the adaptive immune systems to be promoted. In some embodiments, the period of time that downstream treatment can be delayed can be 4, 5, 6, 7, 8, 9, 10, 11, 12, 13, 14, 15, 16, 17, 18, 19, 20, 21, 22, 23, 24, 25, 26, 27, 28, 29, or 30 days.

The downstream treatment regimen can optionally be or include measuring an immune response in the subject at one or more time points post non-thermal ablation treatment. In some embodiments, the activation/promotion of the immune response can be evaluated by monitoring the reduction in immunosuppressive immune molecules and/or increase in pro-inflammatory molecules on the local and systemic level. In some embodiments, the population of Treg cells are monitored. Other suitable immune molecules and/or cell types that can be measured post non-thermal ablation can include, but are not limited to, interferon gamma (IFNg); TNF; IL-6; HMGB1; ATP; IL-21; IL-22; IL-23; IL-1B (which are all secreted proteins or damage associated molecular patterns that are associated with inflammatory conditions and whose increase is associated with an increase in the immune response) TGFB, IL-2, IL-10, Tregs, TAMs, TANs, and MDSCs (which are cell types and molecules whose decrease is associated with decreasing suppression of the immune response). These immune molecules and/or cells can be measured in biopsy or other tissue and/or a bodily fluid (e.g. blood, saliva, urine, breast milk). The local and/or systemic levels of one or more of these immune molecules and/or cells can be measured. Assays and techniques (e.g. flow cytometry, spectroscopy, immunoassays, and the like) for detecting and measuring these immune cells and/or molecules will be appreciated by those of ordinary skill in the art. Monitoring can occur for 1-30 days or more post non-thermal ablation treatment. In some embodiments, the only additional treatment step taken is monitoring of the immune response.

Downstream or secondary treatment(s) can be delayed when the current increases above 25 A. Downstream or secondary treatment(s) can be delayed when the current increases to between 25 A and 100 A. Downstream or secondary treatment(s) can be delayed when the current increases about 100% to about 400% from the start of treatment. Downstream or secondary treatment(s) can be delayed when the tissue bulk conductivity reaches about 0.7 S/m to about 1.5 S/m.

In some embodiments, a treatment parameter such as the bulk tissue conductivity can be measured during a non-thermal ablating treatment via measuring tissue impedance. Methods and techniques for measuring tumor tissue impedance can include measuring impedance using an impedance sensor during the initial non-thermal ablation treatment. In some embodiments, the bulk tissue conductivity can be measured during a non-thermal ablation treatment by evaluating current output during a non-thermal ablation treatment or a change in current during non-thermal ablation treatment. Methods and techniques for measuring bulk tissue conductivity of tumor tissue may include a current output monitor configured to collect data relating to electrical current resulting from one or more of the electrical pulses delivered. It will be appreciated that the tissue can be modeled as an electric circuit. Non-thermal ablation and/or tissue impedance measurement technique that relies on delivery of a current to the tissue can result in pores in the membrane that can disrupt cell membrane capacitance and result in a lower cell resistance and an observed periprocedural current rise, which can be measured in real time. Current can be measured by a variety of techniques, including but not limited to sensors, such as Hall effect sensors/probes, impedance spectroscopy, magnetic resonance electrical impedance tomography (MREIT), and impedance of tissue (previously discussed). In some embodiments, when the current rises to above 25 A, rise to between 25A and 100A, or not rise above 25 A during treatment, this can indicate to a clinician that an immune response in the patient can be promoted, which can result in alteration to downstream or secondary treatment procedures. In some embodiments, the non-thermal ablation treatment can be the delivery of electrical pulses that results in either IRE and/or H-FIRE of tissue and/or cells within the target site and surrounding the target site.

In some embodiments, where the current is measured in real-time and rises to above 25 A, then the non-thermal tissue ablation procedure can be ceased and a subsequent treatment plan may be developed that includes, but not limited to, the delivery of downstream or secondary treatment(s) that can be delayed 4-30 days. In some embodiments, when the current does not rise above 25 A, then a downstream or secondary treatment(s) can begin immediately or within 5 days post non-thermal ablation. In embodiments where the patient's immune response is going to be monitored by detecting and/or measuring the levels of immunosuppressive and/or pro-inflammatory immune molecules and/or cells, screening can begin before, during, and/or after non-thermal ablation treatment and is not depending on any change in or threshold level of bulk tissue conductivity.

The treating a tissue (e.g. a tumor or a cancer) in a subject can include: administering non-thermal ablation to a treatment site, measuring bulk tissue conductivity of the tissue during the non-thermal ablation, detecting a change in the bulk tissue conductivity during the non-thermal ablation and performing an additional downstream treatment in response to the change in the bulk tissue conductivity. The bulk tissue conductivity can be measured by measuring tissue impedance (e.g. via an impedance sensor). The bulk tissue conductivity can be measured by measuring a change in current that can be applied to the treatment site. Downstream treatment(s) can be delayed when the current increases above 25 A. Downstream treatment(s) can be delayed when the current increases to between 25 A and 100 A.

The treating a tissue and/or cells (e.g. a tumor or a cancer) within a target site in a subject can include: administering non-thermal ablation to a treatment site, measuring a treatment parameter, including, but not limited to the bulk tissue conductivity of the tissue during the non-thermal ablation, detecting a change in the treatment parameter, such as the bulk tissue conductivity during the non-thermal ablation and performing an additional downstream or secondary treatment in response to the change in the bulk tissue conductivity (the measured treatment parameter). The bulk tissue conductivity can be measured by measuring tissue impedance (e.g. via an impedance sensor). The bulk tissue conductivity can be measured by measuring a change in current that can be applied to the treatment site. Downstream or secondary treatment(s) can be delayed when the measured current increases above 25 A. Downstream or secondary treatment(s) can be delayed when the measured current increases to between 25 A and 100 A.

The treating tissue and/or cells (e.g. a tumor or a cancer) within a target site in a subject can include: administering a specific set of a plurality of electrical pulses to the target site which can induce non-thermal irreversible electroporation (IRE) of the treatment site; measuring a treatment parameter, including, but not limited to, current during the step of administering the plurality of electrical pulses to the tumor; detecting a change in the measured parameter such as current; and performing an additional downstream or secondary treatment as a result of the change in current (the measured treatment parameter), wherein the downstream or secondary treatment step can include, but is not limited to, tumor resection, thermal ablation, a secondary non-thermal ablation, chemotherapy, radiation therapy, immunotherapy, biologic therapy, genetic therapy (gene editing), and combinations thereofIf there is not a positive change in the current, then this can indicate that the immune system (local and/or systemic) of the subject will not be stimulated by the IRE and/or H-FIRE treatment and thus there is no reason to delay the downstream or secondary treatment.

The current can increase during non-thermal IRE and/or H-FIRE. Downstream or secondary treatment(s) can be delayed when the current increases above 25 A. Downstream or secondary treatment(s) can be delayed when the current increases to between 25 A and 100 A. Downstream or secondary treatment(s) can be delayed when the current increases about 100% to about 400% from the start of treatment. Downstream or secondary treatment(s) can be delayed when the tissue bulk conductivity reaches about 0.7 S/m to about 1.5 S/m.

The immune response can be measured by detecting one or more immune molecules or cell types at a time point after the delivery of the non-thermal ablation such as IRE and/or H-FIRE. This is also discussed in greater detail elsewhere herein. The immune response stimulated by the non-thermal ablation, such as IRE and/or H-FIRE, can be pro-inflammatory and/or non-immunosuppressive.

Insofar as the subject can be immunologically responsive to the IRE and/or H-FIRE where there is a positive change in current, for example a change of current between 25 A - 100 A, the downstream or secondary treatment of a tissue resection can be delayed for a period of time in an effort to best engage the adaptive immune system, which peaks 10-21 days post-stimulation. This intentional and/or planned delay in time between the primary non-immunosuppressive treatment of non-thermal ablation and the downstream or secondary immunosuppressive treatment of a tissue resection is an example of a treatment plan specifically devised by the clinician to allow, in-part, for the subject's own immune response to respond and work to destroy the tumor or metastatic cells in circulation. In some cases, the subject's own immune response can respond to tumor antigens released as a result of the non-thermal ablation such as IRE and/or H-FIRE or already present. When a positive change in current, for example a change of current between 25 A - 100 A, is detected, the downstream or secondary immunosuppressive treatment of a tissue resection of all or part of the tumor is delayed for a period of time to allow the subject's immune system to respond to the H-FIRE and/or IRE.

In yet another example, insofar as the subject can be immunologically responsive to the primary non-immunosuppressive treatment of non-thermal ablation and a positive change in current, for example a change of current between 25 A - 100 A, is detected, the primary non-immunosuppressive treatment of non-thermal ablation may be intentionally stopped by the clinician mid-treatment and before all of the planned electrical pulses have been delivered. For example, if the primary non-immunosuppressive treatment of non-thermal ablation has an initial treatment plan of delivering at least 90 total electrical pulses over the entire non-thermal ablation procedure, and a treatment parameter, such as a positive change in current is monitored and detected to have a change of between 25 A - 100 A, before all of the at least 90 total electrical pulses have been delivered, then the clinician may pause and/or stop the delivery of electrical pulses as this change in current may indicate that the subject's immune system is having a positive response to the non-thermal ablation procedure and the treatment does not need to be completed as originally planned.

In some embodiments, the patient is a subject that is eligible for tumor resection surgery as a current standard of care. Standards and guidelines for determining if a tumor and thus a subject is eligible for a resection procedure will be instantly appreciated by one of ordinary skill in the art. In some cases, the method can be performed on a subject that is ineligible for tumor resection to monitor the effect of other treatment modalities and/or determine when other treatment modalities should be administered as discussed in greater detail elsewhere herein.

The method described herein can be performed on the remaining tissue after a partial resection of tissue.

The method described herein can be used to screen patients to determine if their (or will not) immune response will be stimulated post- non-ablation treatment or not. In other words, the method described herein can be used to screen patients to determine if they will respond to this immunotherapy or not and thus be used by the clinician to determine and administer an appropriate treatment for the individual patient.

The immunotherapy, if administered and/or allowed to take place post primary therapy (such as a non-thermal ablation treatment) being administered, can be or can include ablation-based immunotherapy, monoclonal antibody therapies such as rituximab (RITUXAN) and alemtuzumab (CAMPATH), non-specific immunotherapies and adjuvants, such as BCG, Tolllike receptor (TLR) ligands, cytokines (such as interleukin-2 (IL-2) and interferon-alfa), saponins and bacterial exotoxins, immunomodulating drugs, for instance, thalidomide and lenalidomide (REVLIMID), and cancer vaccines such as PROVENGE and ONCOPHAGE. The immunotherapy can also be or can include an adoptive cell transfer therapy (ACT). In general, adoptive cell therapies harvest immune cells such as T-cells, dendritic cells, and/or macrophages from a patient and grow them outside the patient. The immune cells are then engineered such that, when introduced back to the patient, the immune response to a cancer is improved. The immune cells can be engineered to express a pro-inflammatory molecule such as a cytokine, or a receptor involved in immune cell signaling. The ACT immunotherapy can be chimeric antigen receptor (CAR) T-cell therapy.

The immunotherapy can be delivered to the patient by routes of administration such as orally, parenterally, intraperitoneally, intravenously, intraarterially, transdermally, sublingually, intramuscularly, rectally, transbuccally, intranasally, liposomally, via inhalation, vaginally, intraocularly, via local delivery, subcutaneously, intraadiposally, intraarticularly, intraperitoneally and intrathecally, as directed by the regulatory labeling for the particular immunotherapy.

Embodiments of the invention include systems capable of performing one or more methods described herein. The systems can have therapeutic, diagnostic, or prognostic utilities or applications, or combinations thereof, according to various implementations. Therapeutic applications include non-thermally ablating one or more tumor or portion thereof. Diagnostic or prognostic applications include determining the likelihood of a response to one or more additional treatment, such as tissue resection, thermal ablation, non-thermal ablation, chemotherapy, radiation therapy, immunotherapy, biologic therapy, genetic therapy, and combinations thereof.

An ablation system includes one or more electrode, such as a first and second electrode, a voltage generator programmed to generate a plurality of electrical pulses between the first and second electrodes in a manner which causes ablation, such as non-thermal ablation of cells of one or more tissue, one or more sensor capable of measuring one or more treatment parameter associated with the ablation, such as current or a change in current, a memory having one or more stored values of one or more treatment parameter and a set of computer-readable instructions, a processor capable of comparing one or more measured treatment parameter to the one or more stored values of the one or more treatment parameter and determining a modality, course and/or timing of one or more treatments based on the set of instructions.

The medical diagnostic system as disclosed herein includes a generator configured to deliver one or more electrical pulses to a patient, a current output monitor to collect data relating to electrical current resulting from one or more of the electrical pulses delivered, programming configured to determine an average current or a change in current from the electrical current data collected by the current output monitor, and based on the average current and/or the change in current:

(i) determine whether and/or how the patient is expected to respond to immunotherapy as defined in the claims. The system can be also configured to
(ii) determine whether and/or when to stop delivering the one or more electrical pulses to the patient; and/or
(iii) determine a likelihood of effectiveness of a treatment; and/or
(iv) determine whether and/or when to deliver a subsequent treatment.

Another embodiment of a medical diagnostic system includes a generator configured to deliver one or more electrical pulses to a patient in an amount and for a time capable of causing non-thermal ablation of cells of tissue of the patient, a current output monitor to collect data relating to electrical current resulting from one or more of the electrical pulses delivered, programming configured to determine an average current or a change in current from the electrical current data collected by the current output monitor, and based on the average current and/or the change in current:

(v) determine whether and/or how the patient is expected to respond to immunotherapy as defined in the claims The system can be also configured to
(vi) determine whether and/or when to stop delivering the one or more electrical pulses to the patient; and/or
(vii) determine a likelihood of effectiveness of a treatment; and/or
(viii) determine whether and/or when to deliver a subsequent treatment.

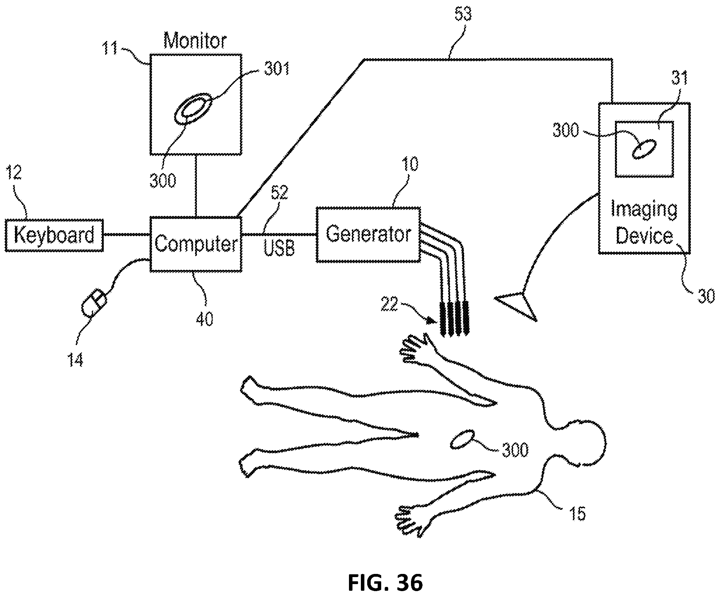

A system embodiment which is capable of executing any method described herein or portion thereof is illustrated in FIG. 36. One or more electrodes/probes 22 deliver therapeutic energy and are powered by a voltage pulse generator 10 that generates high voltage pulses as therapeutic energy such as IRE and/or HFIRE pulses capable of non-thermally ablating (e.g. irreversibly electroporating) the tissue cells. In the embodiment shown, the voltage pulse generator 10 includes six separate receptacles for receiving up to six individual probes 22 which are adapted to be plugged into the respective receptacle. The receptacles are each labeled with a number in consecutive order. In other embodiments, the voltage pulse generator 10 can have any number of receptacles for receiving more or less than six probes.

Each probe 22 includes either a monopolar electrode, bipolar electrodes having at least two electrodes (electrode conducting regions) separated by an insulating sleeve, or multipolar electrodes having greater than two electrode surfaces separated by one or more insulating sleeves which can be energized simultaneously or at different times. In one embodiment, if the probe includes a monopolar electrode, the amount of exposure of the active portion of the electrode can be adjusted by retracting or advancing an insulating sleeve relative to the electrode. See, for example, U.S. Pat. No. 7,344,533. In the embodiment shown, the probes **22** are monopolar electrodes. The generator **10** is connected to a treatment control computer **40** having input devices such as keyboard **12** and a pointing device **14,** and an output device such as a display device **11** for viewing an image of a target treatment area such as a lesion **300** surrounded by a safety margin **301.** The therapeutic energy delivery device **20** is used to treat a lesion **300** inside a patient **15.** An imaging device **30** includes a monitor **31** for viewing the lesion **300** inside the patient **15** in real time. Examples of imaging devices **30** include ultrasonic, CT, MRI and fluoroscopic devices as are known in the art.

For purposes of this application, the terms "code", "software", "program", "programming", "application", "software code", "software module", "module", "program module", and "software program" are used interchangeably to mean software instructions that are executable by a processor. The computer-executable instructions may be organized into routines, subroutines, procedures, objects, methods, functions, or any other organization of computer-executable instructions that is known or becomes known to a skilled artisan in light of this disclosure, where the computer-executable instructions are configured to direct a computer or other data processing device to perform one or more of the specified processes and operations described herein. The computer-executable instructions may be written in any suitable programming language, nonlimiting examples of which include C, C++, C#, Objective C, Swift, Ruby/Ruby on Rails, Visual Basic, Java, Python, Perl, PHP, MATLAB and JavaScript.

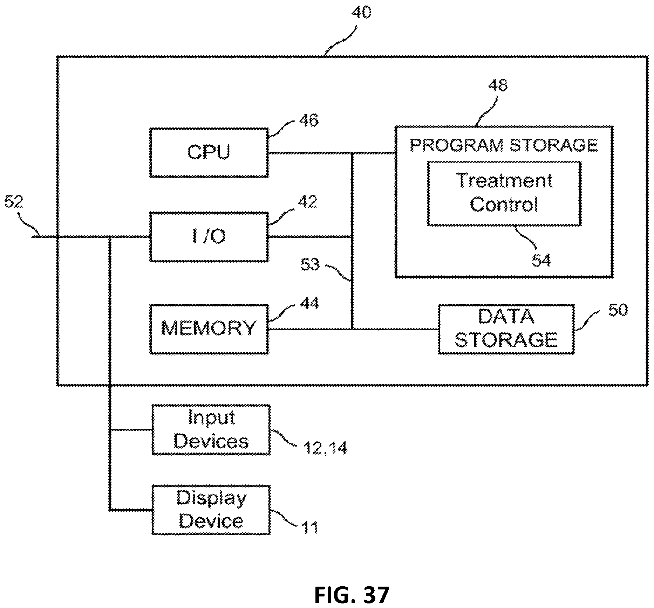

The "user" can be a physician or other medical professional. The treatment control module **54** (**FIG. 37**) executed by a processor outputs various data including text and graphical data to the monitor or display **11** associated with the generator **10.**

Referring now to **FIG. 37,** the treatment control computer **40** is connected to the communication link **52** through an I/O interface **42** such as a USB (universal serial bus) interface, which receives information from and sends information over the communication link **52** to the voltage generator **10.** The computer **40** includes memory storage **44** such as RAM, processor (CPU) **46,** program storage **48** such as ROM or EEPROM, and data storage **50,** such as hard disk, all commonly connected to each other through a bus **53.** As used herein, "memory storage", "program storage", and "data storage" are interchangeable with "nontransitory computer readable storage medium". Program storage **48** stores, among others, computer software (treatment control module **54**) which assists a user/physician to plan for, execute, and review the results of a medical treatment procedure. The treatment control module **54,** executed by the processor **46,** assists a user to plan for a medical treatment procedure by enabling a user to more accurately position each of the probes **22** of the therapeutic energy delivery device **20** in relation to the lesion **300** in a way that will generate the most effective treatment zone. The treatment control module **54** can display the anticipated treatment zone based on the position of the probes and the treatment parameters. Using any of the above described methods, the treatment control module **54** can display the progress of the treatment in real time and can display the results of the treatment procedure after it is completed. This information can be used to determine whether the treatment was successful and whether it is necessary to re-treat the patient.

The module **54** is also adapted to monitor and display the progress of the non-thermal ablation procedure and to determine a successful end point based on the electrical properties of the tissue prior to and during the treatment procedure as explained in more detail in U.S. Patent Application Publication No. 20190175248A1. Being able to in real-time monitor and see the end point of the treatment procedure is a huge advantage over the current method in which the physician is performing the treatment essentially blindly without having any idea about whether the treatment is progressing or at what point the treatment procedure is finished.

The program storage **48** stores various electrical threshold values that are used to monitor the treatment procedure. When the programmed sequence of pulses has been delivered and the end point of the procedure has not been reached, the user interface portion of the control module **54** retrieves the recommended parameter changes from the database and presents them to the user through the display **11.** The treatment control module **54** can also change the threshold values for determining the progress and the end point of the procedure based on initial treatment pulse parameters programmed by the user. For example, different body parts/organs or different health/age of patients may require different thresholds as their conductivity and susceptibility to irreversible electroporation may differ. User can manually input the various thresholds for different tissue types or the system can have these thresholds stored electronically.

Alternatively, the treatment control module **54** can also automatically derive or adjust the threshold values for determining the progress and the end point of the procedure based on test signals (e.g., AC test signals) that are applied and determining electrical properties of the cells such as impedance values or current values. The control module **54** may then store the changed threshold values in the program storage **48** for later use as the new criteria for comparison.

Further, AC intra-treatment test signals may continue to be delivered in addition to the comparative DC intra-treatment test signals. By tracking the change in impedance for the AC signal, the treatment control module **54** determines and factors out the effects on impedance occurring due to temperature rise. This enables more accurately tracking changes in the real-part of the impedance by reflecting changes encountered solely due to persistent electroporated cells. A more detailed discussion of the control module **54** is made in U.S. Patent Application Publication No. 20190175248A1.

Any of the software program modules in the program storage **48** and data from the data storage **50** can be transferred to the memory **44** as needed and is executed by the CPU **46.**

In one embodiment, the computer **40** is built into the voltage generator **10.** In another embodiment, the computer **40** is a separate unit which is connected to the voltage generator through the communications link **52.** The communication link **52** can be, for example, a USB link.

In one embodiment, the imaging device **30** is a stand-alone device which is not connected to the computer **40.** In the embodiment as shown in **FIG. 36,** the computer **40** is connected to the imaging device **30** through a communications link **53.** As shown, the communication link **53** is a USB link. In this embodiment, the computer can determine the size and orientation of the lesion **300** by analyzing the data such as the image data received from the imaging device **30,** and the computer **40** can display this information on the monitor **11.** In this embodiment, the lesion image generated by the imaging device **30** can be directly displayed on the monitor **11** of the computer running the treatment control module **54.** This embodiment would provide an accurate representation of the lesion image on the grid **200,** and may eliminate the step of manually inputting the dimensions of the lesion in order to create the lesion image on the grid **200.** This embodiment would also be useful to provide an accurate representation of the lesion image if the lesion has an irregular shape.

It should be noted that the software can be used independently of the generator **10.** For example, the user can plan the treatment in a different computer as will be explained below and then save the treatment parameters to an external memory device, such as a USB flash drive (not shown). The data from the memory device relating to the treatment parameters can then be downloaded into the computer **40** to be used with the generator **10** for treatment.

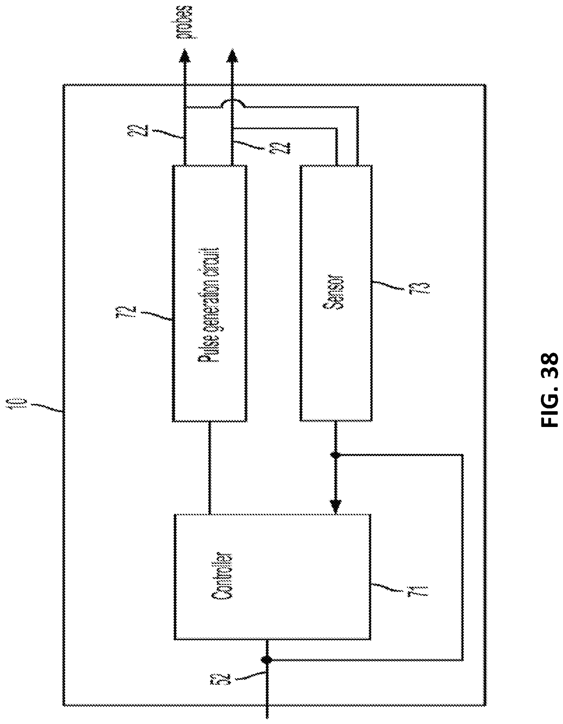

**FIG. 38** is a functional block diagram of a pulse generator **10** shown in **FIG. 36. FIG. 37** illustrates one embodiment of a circuitry to monitor the progress of and determine an end point of the treatment procedure. A USB connection **52** carries instructions from the user computer **40** to a controller **71.** The controller **71** can be a computer similar to the computer **40** as shown in **FIG. 37.** The controller **71** can include a processor, ASIC (application-specific integrated circuit), microcontroller or wired logic. The controller **71** then sends the instructions to a pulse generation circuit **72.** The pulse generation circuit **72** generates the pulses and sends electrical energy to the probes. For clarity, only one pair of probes/electrodes is shown. However, the generator **10** can accommodate any number of probes/electrodes such as 6 probes. In the embodiment shown, the pulses are applied one pair of electrodes at a time, and then switched to another pair. The pulse generation circuit **72** includes a switch, preferably an electronic switch that switches the probe pairs based on the instructions received from controller **71.**

A sensor **73** can sense the current and voltage between each pair of the probes in real time and communicate such information to the controller **71,** which in turn, communicates the information to the computer **40.** Although the treatment control module **54** houses the software code for monitoring the treatment procedure, it may be beneficial for the controller **71** to store such module as the speed of monitoring can be important in some cases.

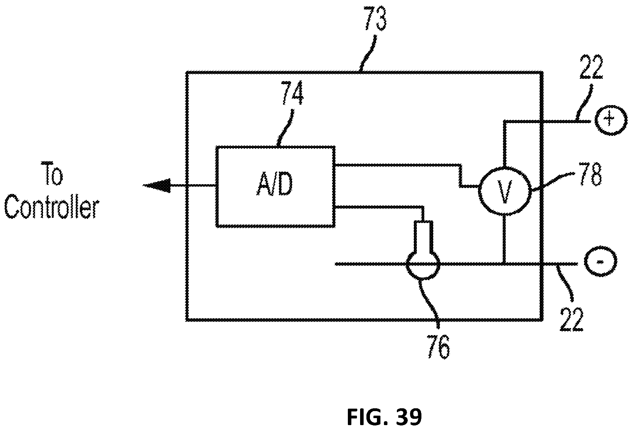

**FIG. 39** is a functional block diagram of a sensor **73** of **FIG. 38.** The sensor **73** includes a voltage sensor **78** connected across a pair of electrodes **22** and a current sensor or output monitor **76** connected to a negative electrode (return conduit) in the pair of electrodes. Although **FIGS. 38-39** show two electrodes from two wires **22,** there may be multiple electrodes between the two wires **22.** The sensed values are continuously received and digitized by an A/D converter **74** and transmitted to the controller **71.** Preferably, the A/D converter **74** can sample the sensed values at a very fast rate and preferably at a rate of at least 100 MHz (100 million samples per second) for the control module **54** to be able to accurately assess the complex impedance of test signals which may be an AC signal at a relatively high frequency.

The current sensor **76** can be any one or more sensor capable of detecting current output, including for example a transformer or current clamp meter, a fluxgate transformer, resistor, fiber optic current sensor, a Hall effect sensor/probe which in some embodiments can be positioned around an electrode so as to measure the electric current without directly interfering with the pulse signal, or can be disposed in any orientation relative to one or more electrode. Typically, the current sensor **76** is placed on the negative signal connection of the electrode pair. If the electrode pairs are switched, then only one current sensor connected at the input side of the switch is needed. Otherwise, if there are 3 pairs of electrodes, for example, and all are firing at the same time, there will be 3 current sensors so as to measure the electric current of each pair separately. In that case, the current from the three sensors will need to be added,

The voltage sensor **78** can be a conventional voltage divider, comprised of two serially connected resistors, that measures a voltage drop across a known resistance value. The voltage sensor **78** uses resistors which are of much higher resistance than the tissue ($k\Omega$-$M\Omega$, versus tissue, which is hundreds of $\Omega$), and thus induces negligible effect on the strength of the pulses delivered to the tissue. A correction factor is calculated for the divider circuit based on the resistances of the two resistors in the voltage divider circuit and the resistance of the load (tissue resistance) to determine the true delivered voltage to the tissue based on the measured voltage drop across the resistor.

The software program modules in the program storage **48** and data from the data storage **50** can be configured so that one or more treatment parameter such as current can be inputted, stored, and/or displayed to the user/physician on the display device **11.** The one or more treatment parameter can be stored in the data storage **50,** and values such as the absolute values or average values of the one or more treatment parameter or relative values, such as the amount of change in the one or more treatment parameter over time, can be inputted, stored, and/or displayed by the user/physician. In one embodiment, the treatment parameter values are stored as thresholds.

The software program modules in the program storage **48** can include programming configured to determine an average value or a change in value of a non-thermal ablation treatment parameter measured in real time during treatment. The programming can input measured values of a treatment parameter during real time monitoring such as current, and calculate an average value over time, or a change in value, such as from a baseline. The baseline can be established at various time points before or during treatment.

Embodiments of the system use real time monitoring of one or more treatment parameter such as current delivered during non-thermal ablation to determine the modality, course and/or timing of subsequent treatments. According to one embodiment, the program storage **48** and data storage **50** include one or more predictive model which can determine the modality, course and/or timing of subsequent treatments based on the monitored treatment parameter(s). For example, during non-thermal ablation, real time current output, average current and/or changes in current can be compared to data in the one or more predictive model or threshold values inputted by the user. Alternatively, or in addition, real time impedance or voltage, or their average values, or their relative values, can be utilized. The predictive model(s) can be based on data gathered from a population or populations of patients, such as one or more clinical trial patient population. In one embodiment, the program storage **48** and/or data storage **50** includes multiple predictive models. The predictive models can be based on groups of patients sharing one or more characteristic such as tumor location (tissue), tumor type, stage of cancer, patient age, and/or presence or absence of metastases. A treatment parameter such as current, impedance, or voltage can be measured during non-thermal ablation treatment of a patient tumor, and an appropriate predictive model can be chosen based on how well the individual patient's characteristics match those of patients whose data contributed to the predictive model. The selected predictive model can then provide a modality, course, and/or timing of one or more subsequent treatments, such as immunotherapy. The predictive model can be a predictive linear model as demonstrated in the Examples, or can be any other suitable predictive model or method, such as a machine learning predictive model (*e.g.* support vector machines, k-nearest neighbors, decision trees, and K-means clustering).

The software program can also be configured so that one or more treatment parameter can be monitored in relation to absolute, average, or relative values stored as a threshold. For example, if the current, impedance, or voltage measured during non-thermal treatment exceeds the threshold, the software program modules can provide a modality, course, and/or timing of one or more subsequent treatments such as immunotherapy. Thresholds can be determined by the program or by the user according to patient data and characteristics such as tumor location (tissue), tumor type, stage of cancer, patient age, and/or presence or absence of metastases. In one example, an absolute current threshold is set to 25 A. In other examples, the current threshold is set between 25 A and 100 A, such as 30 A, 35 A, 40 A, 45 A, 50 A, 55 A, 60 A, 65 A, 70 A, 75 A, 80 A, 85 A, 90 A, and 95 A, or values therebetween.

The program modules in the storage **48** can determine whether and/or how the patient is expected to respond to a modality such as immunotherapy, and/or determine whether and/or when to stop delivering the one or more electrical pulses to the patient, and/or determine a likelihood of effectiveness of a treatment, and/or determine whether and/or when to deliver a subsequent treatment.

For example, the program modules in the storage **48** can determine whether and/or how a patient is expected to respond to immunotherapy by way of reference to historical data in the storage **50** that correlates or associates immunotherapy response with measured values of one or more non-thermal ablation treatment parameter such as current. In one embodiment, high current values measured during non-thermal ablation are associated with a strong immune response against the tumor during subsequent immunotherapy, as exhibited by data such as reduced tumor volume after immunotherapy treatment. In other embodiments, other clinical data such as progression-free survival after immunotherapy treatment is correlated with high current values measured during non-thermal ablation. Similarly, the likelihood of effectiveness of other subsequent treatments, such as tissue resection, thermal ablation, non-thermal ablation, chemotherapy, radiation therapy, biologic therapy, genetic therapy, and combinations thereof, can be determined through correlations or associations of clinical data with measured values of one or more non-thermal ablation treatment parameter. The clinical data from subsequent treatments can correlate or associate with the measured non-thermal ablation treatment parameter values with respect to multiple variables, such as, for example, course, timing, dosage, therapeutic agent, and route of administration of the subsequent treatment. In one embodiment, the clinical data from subsequent treatments is associated with the measured non-thermal ablation treatment parameter values in a predictive model. The clinical data in conjunction with the non-thermal ablation parameter values such as current can also be used to determine when the patient has received an optimum dosage of non-thermal ablation treatment and the non-thermal treatment can be terminated.

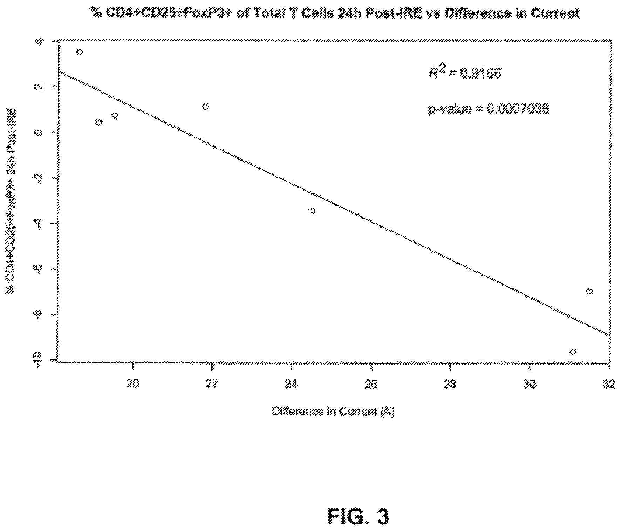

According to embodiments, the clinical data includes data on the immune response or functioning of the patient. For example, the clinical data can include data on immune cell composition, or immune molecule composition, such as the relative numbers of pro-inflammatory cells or molecules or immune inhibitory cells or molecules. An example is shown in FIG. 3, where the change in T cell levels was found to linearly correlate with changes in the delivered electrical current, and a higher change in current values during treatment resulted in a more negative change in CD4+CD25+FoxP3+ levels. In other embodiments, the clinical data can include measurements of other immune cells or substances as markers of immune response, such as interferon gamma (IFNg); TNF; IL-6; IL-12; HMGB1; ATP; IL-21; IL-22; IL-23; IL-1B (which presence indicates an increase in the immune response), or TGFB, IL-2, IL-10, Tregs, TAMs, TANs, and MDSCs (which presence indicates suppression of the immune response).

The program modules in the storage **48** can implement one or more recommendations for subsequent treatments by way of the display device **11.** The display device **11** can display one or more messages to the user/physician, such as "cease non-thermal ablation", "begin chemotherapy", "initiate immunotherapy after 5 days", and so on, as a result of utilization of the predictive models and/or thresholds by the program modules. Other implementations of the program modules can execute an alert by way of an auditory or visual alarm or message, and/or automatically stop the non-thermal treatment. Alternatively, as shown in FIG. 9, the system may display on the display device 11 a read out or other graphical display of the percentage in change in immune cell or immune molecule composition population after 24 hours (such as CD4+CD25+; CD4+CD25+FoxP3+; and/or CD4+CD25+FoxP3-) for a particular patient. This information may then be saved to the particular patient's history or records for future reference.

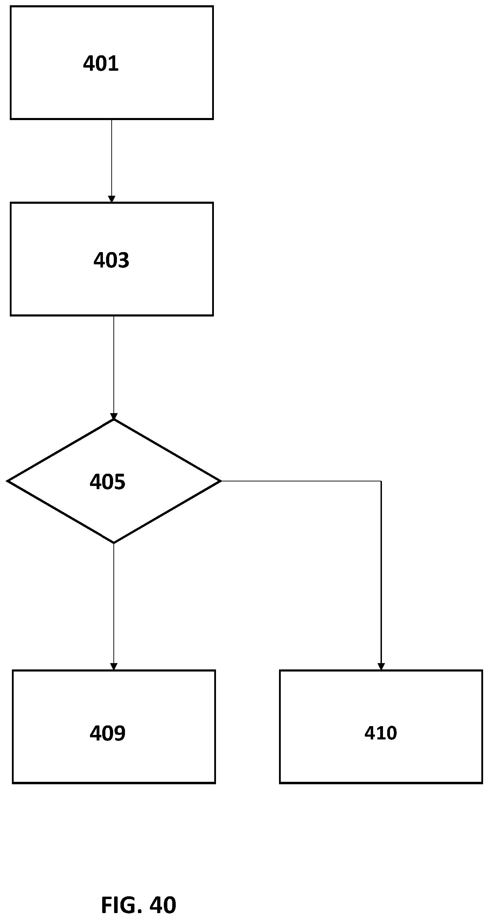

The system can be used to perform a method described as follows. As shown in **FIG. 40,** the method can include ablating the tissue using a non-thermal ablation technique **401,** measuring a treatment parameter or a change in a treatment parameter in real-time during the step of ablating **403,** and determining the modality, course and/or timing of a downstream and/or secondary treatment regimen based on the measured treatment parameter **405.** The downstream and/or secondary treatment can be a first treatment and/or administered in a first, early time period of time **409** or a second treatment and/or administered in a second, later period of time **410,** depending upon the relative or absolute value of the treatment parameter measured. The first and second treatment can be selected from treatments which include tissue resection, thermal ablation, non-thermal ablation, chemotherapy, radiation therapy, immunotherapy, biologic therapy, genetic therapy, and combinations thereof. The first and second period of time can be selected from periods of time which include 0 to 4 days after non-thermal ablation, 0 to 5 days after non-thermal ablation, 0 to 6 days after non-thermal ablation, 0 to 7 days after non-thermal ablation, 4 to 30 days after non-thermal ablation, 5 to 30 days after non-thermal ablation, 6 to 30 days after non-thermal ablation, 7 to 30 days after non-thermal ablation and so on.

According to embodiments of the systems, the step of ablating includes delivery of electrical pulses.

According to embodiments of the systems, the delivering of the electrical pulses is performed by pairs of electrodes.

According to embodiments of the systems, the delivering of the electrical pulses is performed such that pairs of electrodes deliver the electrical pulses in a manner such that an area of the target region bounded by all of the electrodes is subjected to an electrical field.

According to embodiments of the systems, the electrical energy is delivered in cycles, with or without a delay between each cycle, and optionally wherein the cycle is repeated any number of times, such as 1, 2, 3, 4, 5, 6, 7, 8, 9, 10, 11, 12, 13, 14, 15, 16, 17, 18, 19, or 20 times.

According to embodiments of the systems, the target region or tissue comprises cells.

According to embodiments of the systems, the target region or tissue comprises cancer cells and/or non-cancer cells.

According to embodiments of the systems, temperature is measured with one or more thermal sensor, such as measuring temperature of tissue, electrodes, and/or a region of tissueelectrode interface.

According to embodiments of the systems, current and/or temperature is measured, such as measuring temperature with one or more fiber optic thermal sensor.

According to embodiments of the systems, the delivering of the electrical pulses is performed in a manner to provide for pairing of two (2), four (4), six (6), eight (8), or ten (10) pairs of electrodes.

According to embodiments of the systems, the electrodes are disposed in two (2), a four (4), a six (6), an eight (8), or a ten (10) monopolar electrode configuration.

According to embodiments of the systems and methods, a bipolar pulse protocol is delivered.

According to embodiments of the systems, tissue damage is measured by an amount of white tissue coagulation, or an area of white tissue coagulation.

According to embodiments of the systems, ablation occurs by way of IRE and/or HFIRE.

According to embodiments of the systems, a treatment time of less than 5 hours, less than 4 hours, less than 3 hours, less than 2 hours, or less than 1 hour of treatment duration, such as less than any of 45 min., 30 min., 15 min., 10 min., or 5 min., such as less than about 7-17 min., wherein the treatment duration is calculated including or not including any delay.

According to embodiments of the systems, the electrical pulses are capable of electroporation-based therapy, electroporation, irreversible electroporation, reversible electroporation, electrochemotherapy, electrogenetherapy, supraporation, and/or high frequency irreversible electroporation, or combinations thereof, such as by way of a DC current.

According to embodiments of the systems, the electrical pulses are administered from two or more electrodes (e.g., two or more electrodes disposed in contact with one or the other tissue region, or two or more electrodes disposed in each or both), and from any number of electrodes, such as 1, 2, 3, 4, 5, 6, 7, 8, 9, 10, 11, 12, 13, 14, 15, 16, 17, 18, 19, or 20 electrodes and in any configuration relative to one another, such as being delivered by one or more pairs.

According to embodiments of the systems, the delivering is performed using a voltage for the plurality of electrical pulses of 0 V to 10,000 V, such as above 0 V or 1 V up to 10,000 V, and/or from 500 V up to 3,000 V, and/or from 1,000 V up to 2,000 V, such as up to 250 V, up to 300 V, up to 350 V, up to 600 V, up to 650 V, up to 800 V, up to 1,200 V, up to 1,500 V, up to 5,000 V, up to 7,500 V, or any range in between any of these ranges or endpoints, including as endpoints any number encompassed thereby. The voltage for the pulses can be the same or different.

According to embodiments of the systems, the applying is performed using at least two electrodes spaced from 0 cm to 10 cm apart, such as from above 0 cm up to 10 cm apart, or from 0.2 cm to 9 cm, such as from 0.5 cm to 5 cm, or from 1 cm to 4 cm apart, or from 2 cm to 3 cm, or 1.5 cm, or any range in between any of these ranges or endpoints, including as endpoints any number encompassed thereby.

According to embodiments of the systems, the electrodes are needle electrodes, plate electrodes, blunt tip electrodes or combinations thereof.

According to embodiments of the systems, the electrodes have a length (whether the length of the active tip of the electrode or the shaft of the probe) ranging from 1 cm to 30 cm, such as from 10 cm to 20 cm, or from 5 cm to 15 cm, and/or with a length of the active portion of the probe (e.g., energizable region) ranging from 0.5 cm to 10 cm, such as from 1 cm to 5 cm, or up to 3 cm or up to 4 cm, or any range in between any of these ranges or endpoints, including as endpoints any number encompassed thereby.

According to embodiments of the systems, one or more pulses of the electrical pulses have a pulse length (width) in the ns to second range, such as in the nanosecond to ms range, such as from 1 picosecond to 100 microseconds, or from 1 picosecond to 10 microseconds, or from 1 picosecond to 1 microsecond, or from at least 0.1 microsecond up to 1 second, or from 0.5 microseconds up to 10 microseconds, or up to 20 microseconds, or up to 50 microseconds, such as 15, 25, 30, 35, 40, 55, 60, 75, 80, 90, 110, or 200 microseconds, or any range in between any of these ranges or endpoints, including as endpoints any number encompassed thereby. The pulse length (width) of the pulses can be the same of different.

According to embodiments of the systems, the plurality of electrical pulses has a frequency in the range of 0 Hz to 100 MHz, such as from above 0 Hz or 1 Hz up to 100 MHz, such as from 2 Hz to 100 Hz, or from 3 Hz to 80 Hz, or from 4 Hz to 75 Hz, or from 15 Hz to 80 Hz, or from 20 Hz up to 60 Hz, or from 25 Hz to 33 Hz, or from 30 Hz to 55 Hz, or from 35 Hz to 40 Hz, or from 28 Hz to 52 Hz, or any range in between any of these ranges or endpoints, including as endpoints any number encompassed thereby. The plurality of electrical pulses can be delivered at high frequencies, such as 40 kHz to 100 MHz, such as 50 kHz, 60 kHz, 75 kHz, 100 kHz, 250 kHz, 500 kHz, 1 MHz, 2 MHz, 10 MHz, 20 MHz, 50 MHz, or 75 MHz, or any range encompassing these values, including as endpoints any number encompassed thereby.

According to embodiments of the systems, the plurality of electrical pulses have a waveform that is square, triangular, trapezoidal, exponential decay, sawtooth, sinusoidal, and/or alternating polarity. In embodiments, the plurality of pulses have a waveform in which the positive and negative pulse have equivalent absolute energy delivered but comprise of different shapes, voltages, or durations.

According to embodiments of the systems, the plurality of electrical pulses have a total number of pulses, and/or a total number of pulses per burst, ranging from 1 to 5,000 pulses, such as from at least 1 up to 3,000 pulses, or at least 2 up to 2,000 pulses, or at least 5 up to 1,000 pulses, or at least 10 up to 500 pulses, or from 10 to 100 pulses, such as from 20 to 75 pulses, or from 30 to 50 pulses, such as 1, 5, 10, 15, 20, 25, 30, 35, 40, 45, 50, 60, 70, or 90 pulses, or any range in between any of these ranges or endpoints, including as endpoints any number encompassed thereby.

## EXAMPLES

Now having described the embodiments of the present disclosure, in general, the following Examples describe some additional embodiments of the present disclosure. While embodiments of the present disclosure are described in connection with the following examples and the corresponding text and figures, there is no intent to limit embodiments of the present disclosure to this description. The following examples are put forth so as to provide those of ordinary skill in the art with a complete disclosure and description of how to use the probes disclosed and claimed herein. Efforts have been made to ensure accuracy with respect to numbers (e.g., amounts, temperature, etc.), but some errors and deviations should be accounted for. Unless indicated otherwise, parts are parts by weight, temperature is in °C, and pressure is at or near atmospheric. Standard temperature and pressure are defined as 20 °C and 1 atmosphere.

**Example 1.**

Patients with pancreatic cancer have one of the most dismal prognoses of all cancer types with an approximate five-year survival rate less than 5%. A primary cause of such discouraging statistics is that most cases of pancreatic cancer are diagnosed after metastasis has already occurred. Of all pancreatic cancer types, pancreatic ductal adenocarcinoma (PDAC) represents approximately 95% of cases. The only curative option for patients with PDAC is surgery, yet most patients present with unresectable tumors (due to the involvement of critical structures) and prognosis remains poor. Most patients with metastatic PDAC are given cytotoxic chemotherapy for palliative care. Recently, a combinatorial chemotherapy cocktail (FOLFIRINOX) consisting of oxaliplatin, irinotecan, fluorouracil, and leucovorin has been shown to incrementally improve survival rates for patients with metastatic pancreatic cancer, however, its associated toxicity profile limits its use to otherwise healthy patients.

To achieve an optimal clinical outcome (with destruction of both primary and metastatic cancer cells), it is important to consider how different therapeutic strategies affect the patients' immune systems. Most common chemotherapies may be considered immunosuppressive therapies and limit or hinder the natural biological immune response. What is needed in the art is a primary treatment modality, such as a non-immunosuppressive non-thermal ablation modality like IRE and/or HFIRE that targets the destruction of tumor cells while also supporting, maintaining, and enhancing the natural biological immune response. By attacking tumor and/or cancer cell using non-thermal ablative approaches, the tumor and/or cancel cell may actually act as its own multi-agent anti-cancer vaccine through the production of tumor antigens resulting from the destructed tissue and/or cells. Likewise, this could potentially afford destruction of both primary and metastatic cancers and prevent the likelihood of cancer recurrence. Such alternative modalities either as supplement to chemotherapy or as stand-alone primary treatments to enhance immune response are needed. Clinicians are also in need of treatment planning methods and systems to determine an overall treatment regime that will be most effective for a given patient. The method described in this Example 1 can aid clinicians in deciding which secondary or downstream treatment(s), after non-thermal ablation has been delivered as a primary treatment, are optimal for a given patient. Additionally, non-thermal ablation such as IRE and/or HFIRE may lend itself as a preliminary or primary treatment modality as it is a non-immunosuppressive treatment, and a clinician may decide to deliver the non-thermal ablation prior to a downstream or secondary treatment(s), such as tumor resection.

Moreover, a clinician can be able to use real-time feedback of a treatment parameter, including, but not limited to, a change in current, during the preliminary or primary non-thermal ablation treatment such as IRE and/or HFIRE to create a patient specific treatment plan that is intended to maintain, support, and/or enhance the patient's immune response. If there is a positive change in current, clinicians can leave the tumor *in situ* while the immune response is promoted, and follow up after a period of time, such as 4-30 days, with administration of downstream treatment(s) such as tumor resection, thermal ablation, a subsequent non-thermal ablation, chemotherapy, radiation therapy, immunotherapy, biologic therapy, genetic therapy (gene editing), and/or combinations thereof. If there is no positive change in current, the clinician could immediately perform the downstream or secondary treatment(s), such an immediate tissue resection and put the patient on a standard of chemotherapy therapy. Such an approach could greatly increase the number of patients that would benefit from non-thermal ablation as a preliminary or primary treatment modality.

*Background*

Irreversible electroporation (IRE), which can include H-FIRE, is a non-thermal and non-immunosuppressive ablation technique used to kill cancerous tissue by delivering short electric pulses through electrodes inserted directly into a target site. The resulting electric field increases the transmembrane potential and disrupts cell homeostasis through irreversible membrane pore formation, leading to cell death. IRE can be used to treat unresectable tumors in close proximity to vital structures, such as PDAC surrounding the superior mesenteric artery (SMA). Thus, IRE can be used to treat tumors with intricate morphologies while preserving nerves and major blood vessels in the treatment zone, making it an excellent surgical candidate when resection or thermal ablations are limited by these critical structures. This modality is also particularly attractive for tumors with perineural invasion, which is related to pain and worsened prognosis. To date, IRE has been clinically used to treat human pancreatic, prostate, liver, and kidney tumors and is currently in clinical trials for the ablation of unresectable pancreatic cancer. IRE has been shown to be promising in treating locally advanced pancreatic cancer (LAPC). In a 200-patient clinical trial, IRE combined with resection doubled the median survival rate of LAPC patients. IRE can be modulated to enrich an anti-tumor microenvironment, surpassing traditional pancreatic cancer treatment paradigms. Additionally, it is disclosed herein that using real-time a treatment parameter, such as a change in measured current, can be used to create a patient specific treatment regime that enhances this modulation and enrichment that in turn may lead to regression of primary and metastatic disease through the production of an anti-tumor immune response. Initial results suggest a positive systemic immune response resulting from local ablation, making this technology potentially beneficial for metastatic diseases. This invention utilizes the now clinically available IRE treatment option to monitor the pro-immune response of a patient in real time.

A rapidly advancing field of research in energy-based ablation technologies is based on the concept of immunomodulation that is promoted as a result of a primary tumor ablation, which could contribute to yet another mechanism of tumor cell death and destruction. Combining non-thermal ablation procedures, such as IRE and/or HFIRE, with a downstream or secondary treatment(s) of delivering immunotherapies to a subject has been shown to induce an abscopal effect in which the local non-thermal ablation promotes systematic immune responses leading to regression of tumors and/or cancer cells distant from the target or treatment site. Conversely, high temperature based thermal ablation methods destroy tumors by disruptive necrosis which results in protein denaturation and reduces the amount of intact antitumor antigens while coagulating the tissue, preventing the spill of intracellular products into systemic circulation. Whereas the non-thermal ablation, such as IRE and/or HFIRE, maintains intact intracytoplasmic organelles and proteins, while opening up the plasma membrane, releasing intact, recognizable tumor antigens. In particular, supplying the patient's immune system with adjuvants has been shown to better promote a positive systemic immune response in patients.

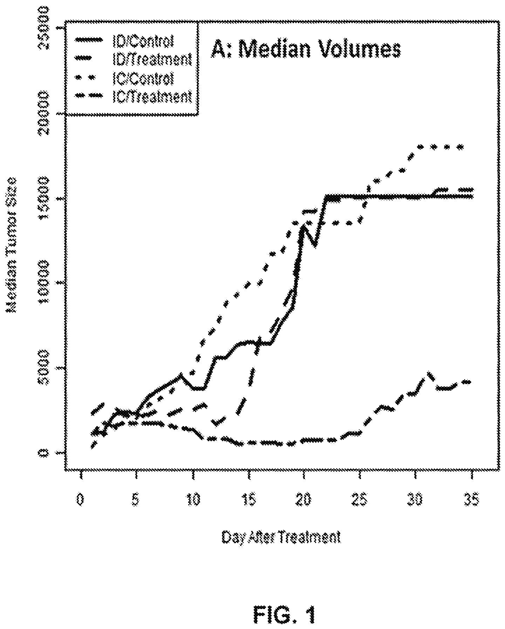

The non-thermal mechanism of IRE promotes a more pro-inflammatory and non-immunosuppressive response when compared to thermal ablation techniques. This suggests that a non-thermal ablation such as IRE and/or HFIRE can be used in conjunction with immunotherapy to optimize patient survival. This also suggests that it may be possible to create a patient specific treatment protocol or regime using real-time feedback of a treatment parameter, such as a change in current of from about 25 A-100 A, from the IRE and/or HFIRE procedure. The immune response to IRE has been examined in osteosarcoma rat and renal carcinoma mouse models. Local antitumor response (**FIG. 1**) has been shown to be greater in immunocompetent BALB/c mice when compared with immunodeficient nude mice. IC mice also exhibited higher T-cell infiltration relative to control **(FIGS. 2A-2D).** In mice treated with IRE, the therapy also promoted a systemic immune response which protected the mice from tumor re-challenge.

This Example can describe the use of real-time current feedback from IRE treatment by the clinician to determine the optimal downstream or secondary treatment(s) to maximize patient outcome and maximize the patient's immune response. Depending on monitored and measured treatment parameter feedback, such as a change in current between 25 A - 100 A, the clinician can determine if the patient is having a positive immune response and choose to continue treatment with either continuing the non-thermal ablation or select the downstream or secondary treatment and an optimal delay between these treatments if so desired. Non-thermal ablation such as IRE and/or HFIRE can create an inflammatory response and increases antigen presentation at the primary tumor, and this release of antigens and cellular content from the cells can be monitored in real time using the IRE pulse delivery device. This method can allow a clinician to make real time decisions about adjuvant therapies during the IRE treatment itself. Thus, IRE lends itself as a preliminary therapy to determine the extent of a patient's immune response and inform clinicians as to which subsequent therapy to administer.

*Method Summary*

Seven patients with unresectable Pancreatic Ductal Adenocarcinoma (PDAC) were treated with IRE. Postoperative management of patients treated for pancreatic lesions with IRE is standard and follows guidelines for any type of pancreatic resection. Forkhead box P3 (FoxP3) expressing T-regulatory cells are a sign of immune tolerance. To identify T-regulatory cells and FoxP3 subsets, isolated peripheral blood mononuclear cells (PBMC) were labeled with anti-CD4-Pe-Cy-5, CD25-Pe and anti-FoxP3- AlexaFlour488. Concentrations of CD4+ and CD25+ cells and T-regulatory levels were measured using a flow cytometer assay for PBMC in CD4+ cells. FoxP3 subset T-regulatory cells were identified as those stained with antibodies against FoxP3/CD25 and identified according to the expression of CD4+, CD25hi and FoxP3+ by fluorescence-activated cell sorting (FACS). All PBMC samples were analyzed within 3 hours of collection to ensure cell viability.

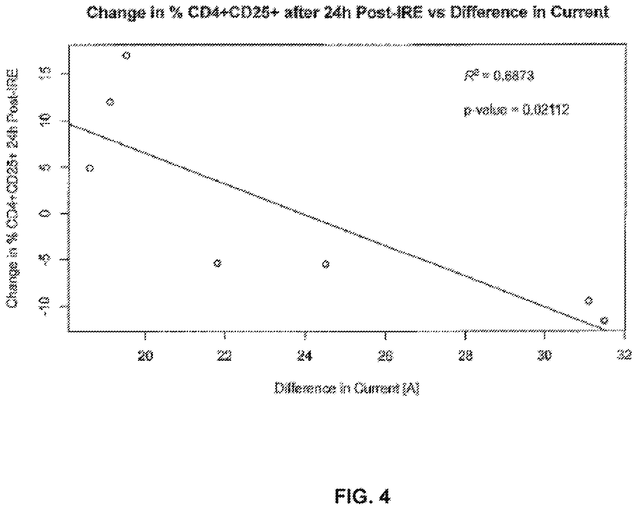

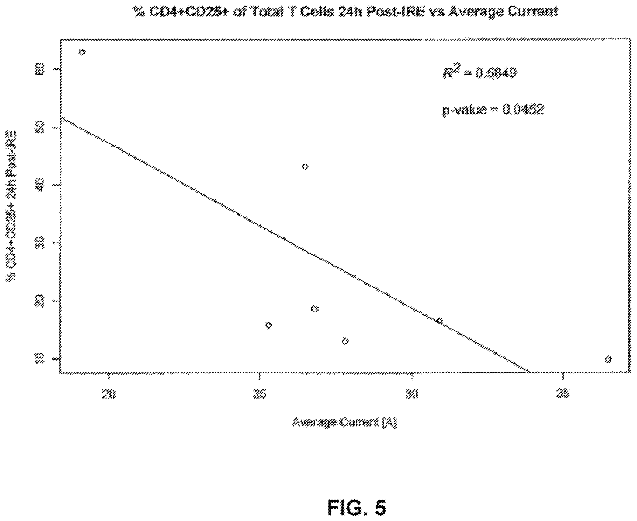

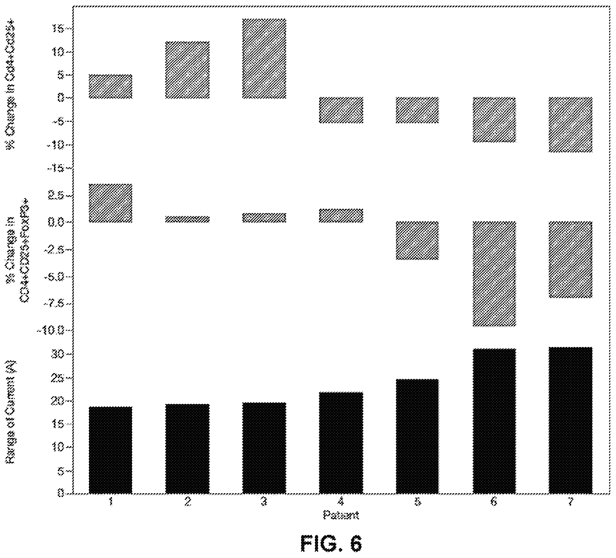

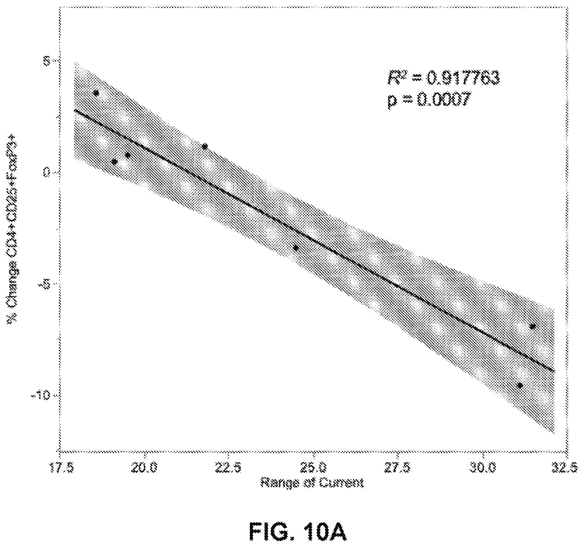

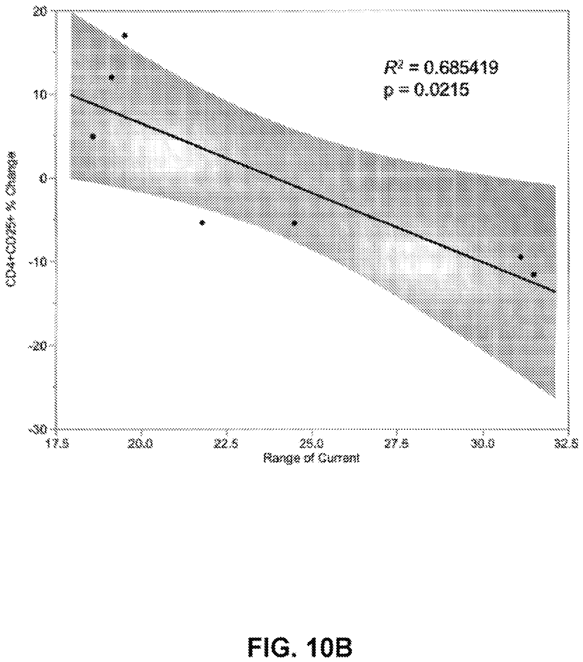

Current waveforms from the 7 performed IRE treatments were collected from the IRE pulse generator. The total change in current as well as average current delivered were calculated using a MATLAB script (vR2016a, Mathworks Inc., Natick, MA, US). A linear regression was performed to examine the relationship between current and T-cell population changes using JMP Pro software (version 13.0.0, SAS Institute, Cary, NC, USA). A statistical significance level of 0.05 was used for the analysis. T cell populations measured from blood samples before and 24 hours, but could be up to 5 days post IRE treatment, after treatment show an array of responses between the 7 different patients (**FIG.6**). This indicates a response from the patient's immune system following IRE treatment. Of the 7 patients treated with IRE, some exhibited an increase in T-cell populations while others exhibited a decrease. The change in T cell levels was found to linearly correlate with changes in the delivered electrical current. In particular, a higher change in current values during treatment responded in a more negative change in CD4+CD25+ and CD4+CD25+FoxP3+ levels. Both linear relationships were found to be statistically significant (**FIGS. 10A and 10B**). Supporting data can be found in **FIGS. 3-5**. Both average current and change in current are linearly correlated with T cell populations (*n*=7 patients) measured 24 hours after IRE treatment. **FIG. 6** shows a bar chart showing range of current and percent change in patient T-cell populations before and 24 hours after IRE.

Considering the dire need for new approaches for treating late-stage pancreatic cancers with curative intent, this method allows clinicians to make a real-time decision regarding a downstream or secondary treatment. The suggested predictive linear model can be implemented in the clinic during non-thermal ablation such as IRE and/or HFIRE treatments to correlate treatment feedback with a patient specific immune response. The relationship between current delivered and changes in the patient's immune response could position IRE as a preliminary or a primary therapy which clinicians can use to treat the underlying disease; screen patients for possible immunotherapy candidates; and help the clinician to formulate patient specific treatment regimens to increase the positive immune response and positive overall outcomes. This screening capability could prevent unnecessary rounds of ineffective treatments, and allows for a more personalized treatment plan.

Patients eligible for tumor resection surgery or another immunosuppressive therapy can first undergo a non-thermal IRE and/or HFIRE procedure. If there is a positive change in a selected treatment parameter, such as a change in current of from about 25 A-100 A, the clinician may decide to leave the tumor in situ and wait for an intentional delay in time, such as more than one day, for the optimal immune response to occur before resecting and delivering immunotherapy. If during the non-thermal procedural there is no positive change in the selected treatment parameter, such as current, the clinician could immediately resect and/or perform the immunosuppressive therapy. Such an approach could greatly increase the number of patients that would benefit from the IRE.

**Example 2.**

Patients with pancreatic cancer have one of the most dismal prognoses of all cancer types with an approximate five-year survival rate of 9%. The only curative option for patients with pancreatic ductal adenocarcinoma (PDAC) is surgical resection, yet most patients present with unresectable tumors due to the proximity of the tumor to critical structures or with metastatic disease. Immune-based therapies have recently gained attention due to their early clinical success in altering the course of disease in patients with previously untreatable cancers. Unfortunately, the relatively immunosuppressive nature of pancreatic cancer hinders the systemic delivery of immunotherapies in PDAC as compared with other tumor types. This immunosuppressive phenotype of pancreatic cancer derives from specialized immune cells, such as T-regulatory ($T_{reg}$) cells, which ultimately mask the tumor from the immune system, resulting in a reduced anti-cancer immune response.

A local anti-tumor response has been shown to be greater in immunocompetent rats when compared with immunodeficient rats following a non-thermal IRE treatment. Also, non-thermal IRE has been shown to reduce resistance to an immune checkpoint blockade in a rat model. By using tumor disruptive approaches, the tumor can act as its own anti-cancer vaccine through the production of patient-specific tumor antigens associated with the ablated tissue. The production of neoantigens along with decreases in suppressive immune cell populations like $T_{reg}$ cells in the ablation zone can lead to increased pro-inflammatory immune system involvement. This could potentially facilitate the destruction of both primary and metastatic tumors and prevent the likelihood of cancer recurrence following treatment. Non-thermal ablation such as IRE and/or HFIRE has been used to treat human prostate, liver, and kidney tumors. In locally advanced pancreatic cancer (LAPC) patients, IRE has been shown to nearly double the median survival when combined with chemotherapy. However, since some types of chemotherapy have been shown to have a cytotoxic effect on immune cells, some patients demonstrating a decline in $T_{reg}$ cells may benefit from the synergistic effects of IRE and immunotherapy.

Recently, circulating $T_{reg}$ cell populations in the blood of unresectable pancreatic cancer patients undergoing chemotherapy were shown to be associated with improved overall survival rates. This Example 3 can demonstrate report that post-IRE $T_{reg}$ cell populations are correlated with the change in a treatment parameter, here such a treatment parameter was the current delivered to the local tumor. Specifically, a change of approximately 25 A resulted in a negative change in $T_{reg}$ cell populations 24 hours after IRE treatment. Since current changes can be monitored in real-time, future changes in $T_{reg}$ cell populations may be predicted during the IRE treatment. This ability to predict changes in the patient's systemic immune system may improve treatment applications and has the potential to maximize post-treatment options.

*Methods.*

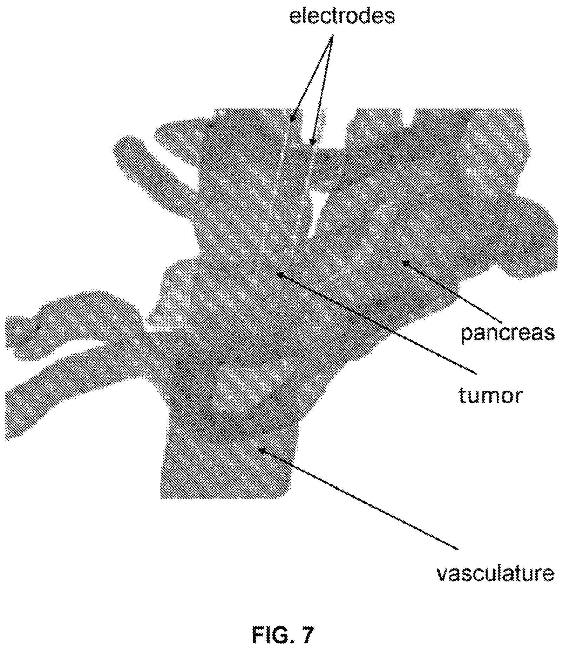

Seven patients with stage 3 pancreatic cancer were treated with IRE via laparotomy procedure. Prior to the electrode placement procedure, two-dimensional ultrasound imaging was used to check for metastatic disease and to confirm primary tumor size. Ultrasound was used during needle placement in order to bracket the primary tumor and safeguard proper needle placement (**FIG. 7**). Patients were under appropriate paralytic and narcotic protocol. The first set of pulses consisted of 20 pulses per pair of probes that were used to assess local fibrosis and tissue resistance. The remainder of the treatment consisted of 100-300 pulses per probe-pair contingent on changes in resistance measured across each probe-pair.

Postoperative management of patients treated for pancreatic lesions with IRE was standard and followed guidelines for any type of pancreatic resection.

*Flow Cytometry Assay*

Patient blood specimens were collected both before and 24 hours after the IRE procedure. Blood specimens were transported to the lab for immediate processing to isolate peripheral blood mononuclear cells (PBMCs). Briefly, red blood cells were lysed with hypotonic lysis buffer and total PBMCs were isolated and stored at -80 ºC in RPMI media with 10% human serum albumin and 10% DMSO. Forkhead box P3 (FoxP3) expressing T-regulatory cells are a sign of immune tolerance. To identify Treg and FoxP3 subsets, isolated PBMCs were stained with FoxP3 staining buffer set (130-093-142, Miltenyi Biotec, Germany) per manufacturer instructions, and evaluated using FACSCalibur (BD Biosciences, San Jose, CA). Next, the CD4+ lymphocyte population was identified using BD FACSCalibur (BD Biosciences, San Jose, CA), then sub-populations were gated and recorded. For each patient, PBMC specimens obtained before IRE and 24 hours after IRE, were processed and analyzed simultaneously to avoid experimental variability due to the thawing and staining procedure. Data were recorded and analysis was performed using FlowJo software (Ashland, OR).

*Waveform Analysis*

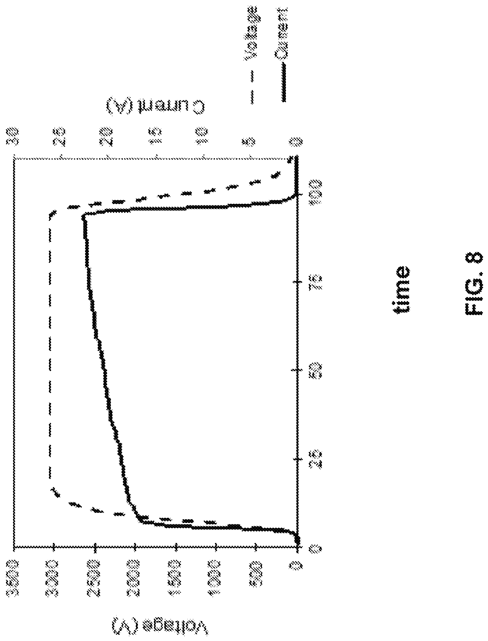

Current waveforms from the seven performed IRE treatments were collected from the IRE pulse generator **(FIG. 8)**. The total change in current as well as average current delivered were calculated using a MATLAB script (vR2016a, Mathworks Inc., Natick, MA, US). A linear regression was performed to examine the relationship between current and T-cell population changes using JMP Pro software (version 13.0.0, SAS Institute, Cary, NC, USA). A statistical significance level of 0.05 was used for the analysis.

*Results.*

$T_{reg}$ cell populations measured from blood samples before and 24 hours after treatment show an array of responses between the seven different patients **(FIG. 9)**. These $T_{reg}$ cell populations represent both natural and promoted $T_{reg}$ cells, which can attenuate anti-tumor immune responses. Of the patients treated with IRE, some exhibited an increase in T-cell populations, while others exhibited a decrease. The change in $T_{reg}$ cell levels were found to linearly correlate with changes in the delivered electrical current **(FIG. 6)**. The range in current is defined as the difference between the maximum and minimum output current values reached during treatment. In particular, a higher change in current value during treatment responded in a more negative change in CD4+CD25+ and CD4+CD25+FoxP3+ levels. Both linear relationships were found to be statistically significant **(FIGS. 10A-10B).**

*Discussion.*

The seven patients had an array of responses in output current change, ranging from approximately 17 A to 30 A. This range indicates that the content of the treatment zone varies from patient to patient, and the electrical impedance changes differently in response to treatments with similar applied voltage and electrode spacing. These results indicate that the degree to which the target tissue undergoes impedance changes affects cell populations related to the immune response in the ablation zone and the surrounding area. Electroporation induces pores in the membranes of cells in the target zone, which may induce inflammation resulting in edema and the production of antigens. In turn, these changes reduce the impedance of the tissue resulting in an increased current output. Additionally, while IRE retains the stromal elements of vasculature, the treatment may interfere with endothelial cells, which further promotes inflammation in the treatment site.

The decrease of $T_{reg}$ cells in the ablation zone also creates an inflammatory response at the primary tumor site. Since systemic $T_{reg}$ cell reduction has been previously shown to improve the prognosis of pancreatic cancer patients, removal of these inhibitory cells could shift the tumor microenvironment from an anti-inflammatory state to one more favorable for anti-tumor immune system activation/promotion. During treatment, this shift may be predicted by monitoring the change in output current delivered through the electrodes. In practice, these results imply that the decline of the $T_{reg}$ population be predicted in real time using the IRE pulse delivery device. The relationship between current delivered and changes in the patient's immune cell populations, beyond just the $T_{reg}$ cell populations, could also position IRE as a preliminary therapy that clinicians could use to screen patients for immunotherapy.

Ultimately, this correlation can provide clinicians with a metric they can use to optimize an IRE treatment and subsequent therapy.

This Example can describe and at least demonstrate the use of real-time current feedback as a treatment parameter from the IRE treatment to be used by the clinician when determining the possibility of a follow-up downstream or secondary therapy to maximize patient outcomes and maximize the immune response. The suggested predictive linear model can be implemented in the clinic during IRE treatments to correlate treatment feedback with the patient's predicted $T_{reg}$ cell population, which is normally unknown until hours following treatment. This information provides clinicians with a much-needed metric to monitor a facet of the patient's immune response. Considering the dire need for new approaches for treating late-stage pancreatic cancers, we believe that this approach could poise IRE as a potential preliminary or primary therapy which can be used to screen patients for the appropriate follow-up therapy. For example, a patient demonstrating a high predicted change in $T_{reg}$ cells would perhaps benefit more from downstream or secondary treatment of immunotherapy rather than chemotherapy, which has been shown to have a cytotoxic effect on immune cells. Additionally, the ability to predict an unchanging $T_{reg}$ cell population in some patients could prevent unnecessary rounds of ineffective treatment and provide a more personalized treatment plan. This screening capability can improve clinician decision making processes regarding follow-up treatment, and ultimately patient outcomes. As discussed elsewhere herein, the screening can confirm the increase in immune activity and/or a decrease in immunosuppressive activity and thus further optimize the downstream treatment strategy post non-thermal ablation.

**Example 3.**

Canine hepatocellular carcinoma (HCC) is an uncommon primary liver tumor that can be presented in three forms: massive, nodular, and diffuse. The massive form represents about 60% of HCCs and carries an excellent prognosis when amendable to surgery, while there is no effective treatment for the nodular and diffuse forms. The prognosis for dogs diagnosed with non-resectable HCC has remained dismal for decades, with a median survival measured in weeks to months, despite chemotherapy, targeted therapies, and radiation. Immunotherapy is an attractive therapeutic option for primary liver tumors, because they arise in the background of an immunetolerant microenvironment. Described in this Example is the effect of H-FIRE on local and systemic immunological stimulation.

High-frequency irreversible electroporation (H-FIRE) is a technique for the focal treatment of pathologic tissues that involves placing minimally invasive electrodes within the targeted tumor tissue. Based on the standard irreversible electroporation (IRE), H-FIRE induces a series of short (ranging from 1 nanosecond to 5 microseconds), bi-polar electric pulses electric pulses, which are applied to destabilize the cell membrane, by creating nanopores, and inducing cell death in a non-thermal fashion. H-FIRE preserves the extracellular matrix, major tissue vasculature, nerves, and other sensitive structures, while negating the need for paralytics during treatment. HFIRE treatments are thought to promote an antitumor immune response, are independent of local blood flow, can be delivered quickly, and can be visualized in real-time.

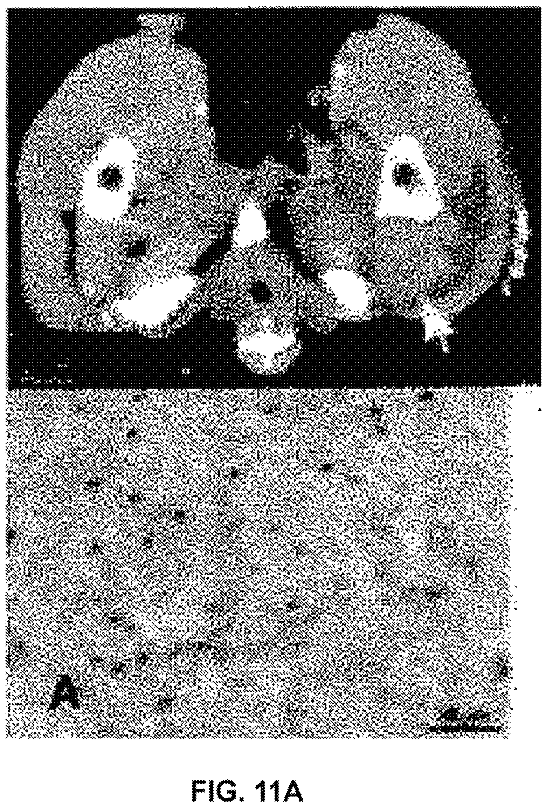

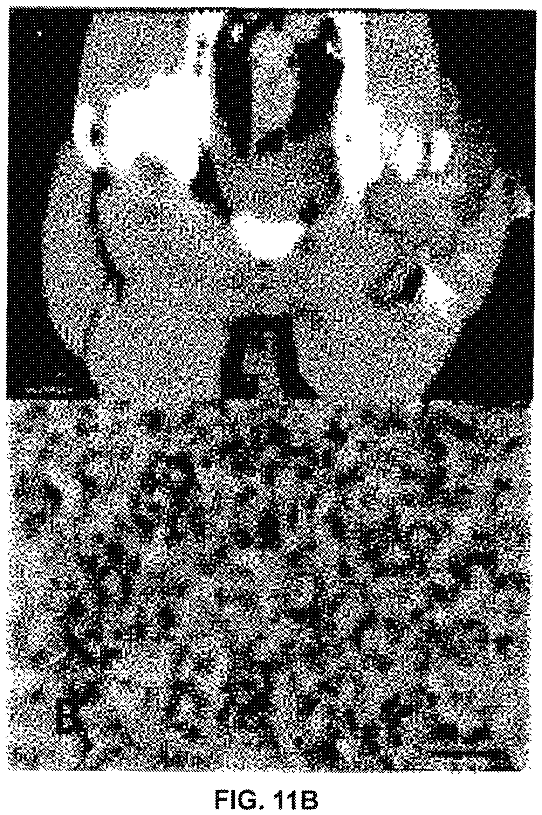

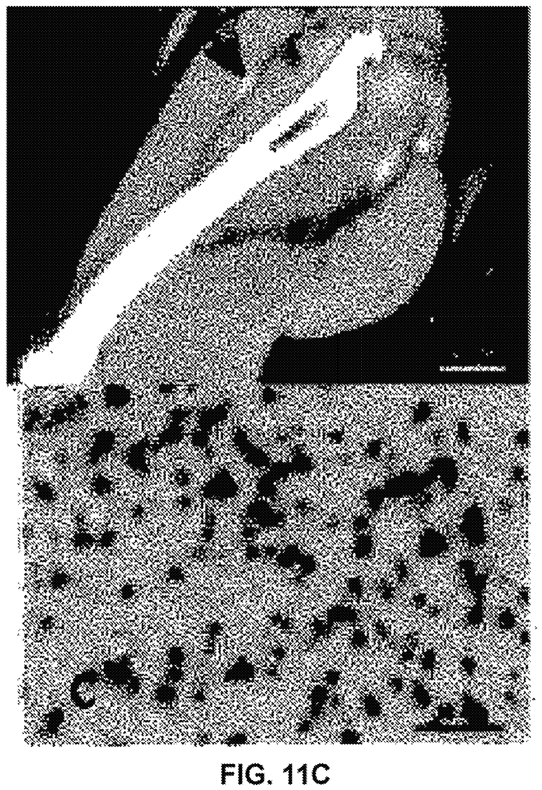

A flat-coated retriever diagnosed with histiocytic sarcoma presented with a history of degenerative coxofemoral joint disease, postural reaction deficits associated with sciatic neuropathy and a large periarticular mass of the left coxofemoral joint. A single IRE treatment to the primary tumor followed by lomustine chemotherapy was curative, as the dog achieved complete response and was euthanized for unrelated reasons about 5 years post IRE/chemotherapeutic treatment. Post-treatment biopsies indicated significant T-cell infiltrates **(FIGS. 11A-11C)**.

Following this response, the effect of IRE therapy on the immune system was examined. A murine model of renal cortical adenocarcinoma (RENCA) was used in 2 groups of mice: 1) immunodeficient mice and 2) immunocompetent mice. During these experiments, both groups of mice were implanted with RENCA cells, and upon tumor formation, treated with IRE.

Treatment resulted in significantly increased tumor response rate and overall survival in the immunocompetent mice when compared to the immunodeficient mice. A robust CD3+ lymphocytic tumor infiltration post-IRE was observed in the treated immunocompetent mice. Moreover, when re-challenged with the same cell line 18 days after the IRE treatment, tumor growth was significantly reduced or prevented entirely **(FIGS. 2A-2D).** These data can support that IRE can promote an immune response that can invoke a systemic immune reaction beyond the ablation region. As is discussed elsewhere herein, a non-immunosuppressive manner such that an initial and local innate immune response followed by promotion of the adaptive immune response, which can be effective to target and destroy circulating and/or metastatic cells.

**Example 4.**

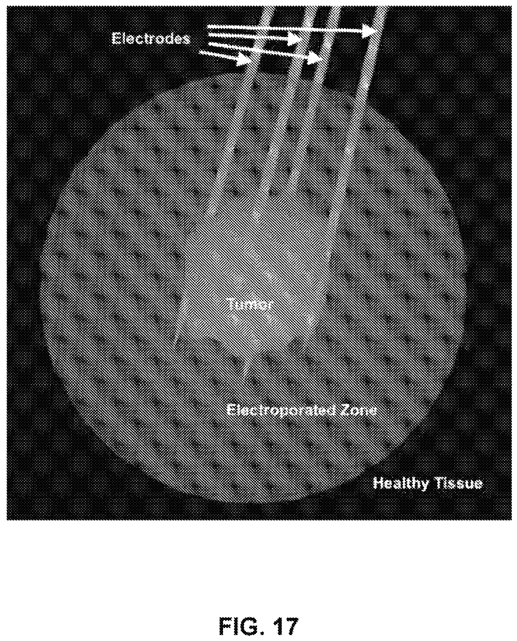

IRE is a technique designed to ablate cancerous tissue by delivering short electric pulses through electrodes inserted directly into targeted tumors (**FIG. 17**). IRE was originally developed for non-resectable tumors; however, as these techniques have evolved, the clinical applications that can benefit from the precision and non-thermal nature of the approach can now be extended, which are discussed at least in this Example.

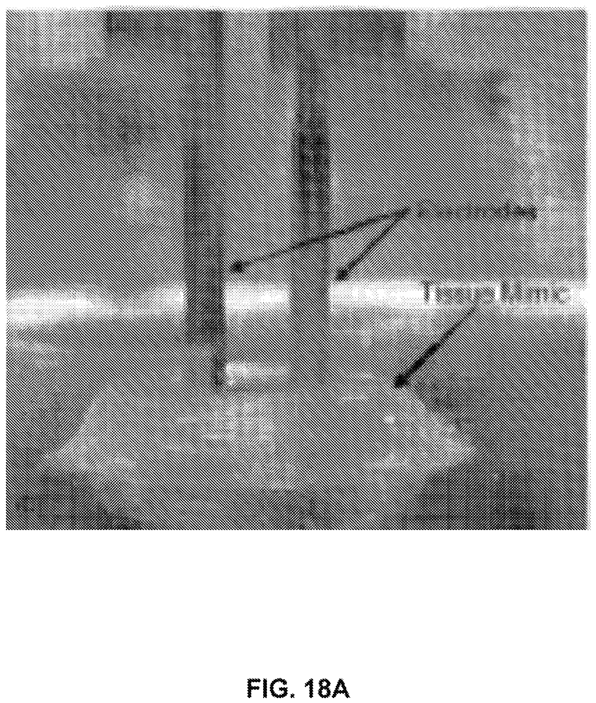

Several *in vitro* models and tissue mimics were developed to evaluate the field effects of IRE and H-FIRE. These include cell monoculture, spheroid models, 3D tissue mimics and 3D organoids **(FIGS. 18A-18E)**. Studies with these models have been validated in a variety of preclinical animal models including mouse, rate, pig, and dog, in both human and veterinary clinical trials.

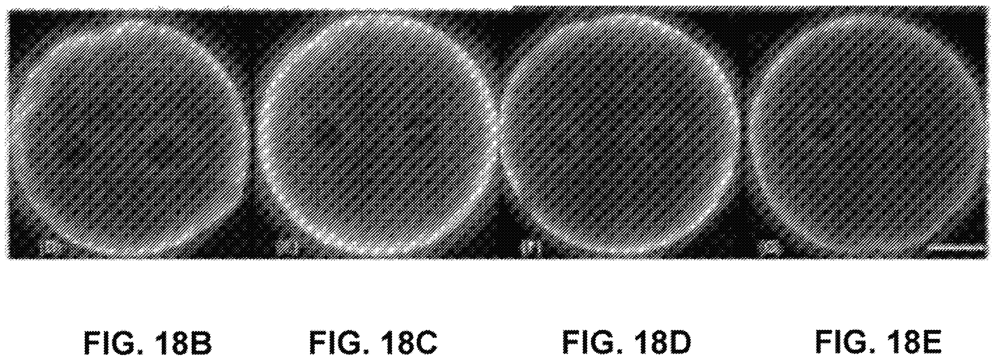

**FIGS. 18A-18E** can demonstrate H-FIRE treatment and modeling in a 3D tumor mimic. **FIG. 18A)** Experimental setup with electrodes inserted into tissue mimic. **FIGS. 18B-18E)** Live /dead staining reveals regions of the tissue impacted following 80 bursts containing **(FIG. 18B)** 2, **(FIG. 18C)** 24, and **(FIG. 18D)** 50 bipolar $2\mu$s pulses with a 2 $\mu$s delay between alternating pulses. **(FIG. 18E)** Diffuse treatment of 50 bipolar $2\mu$s pulses with 20ms between alternating pulses. Scale bar=2mm.

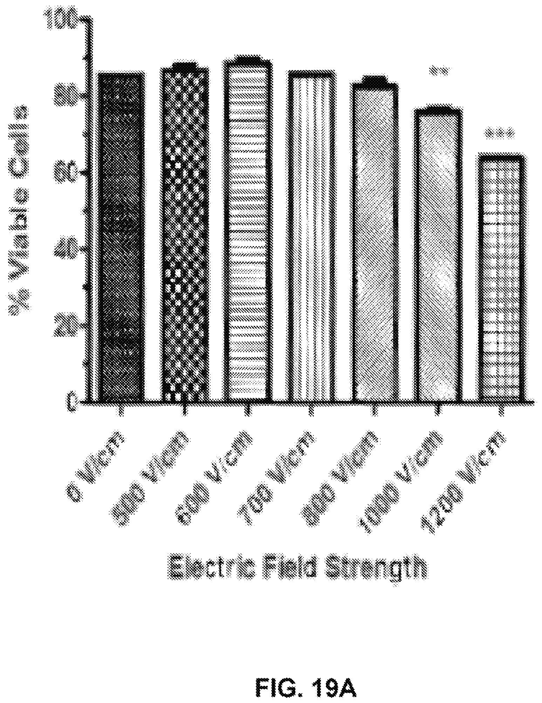

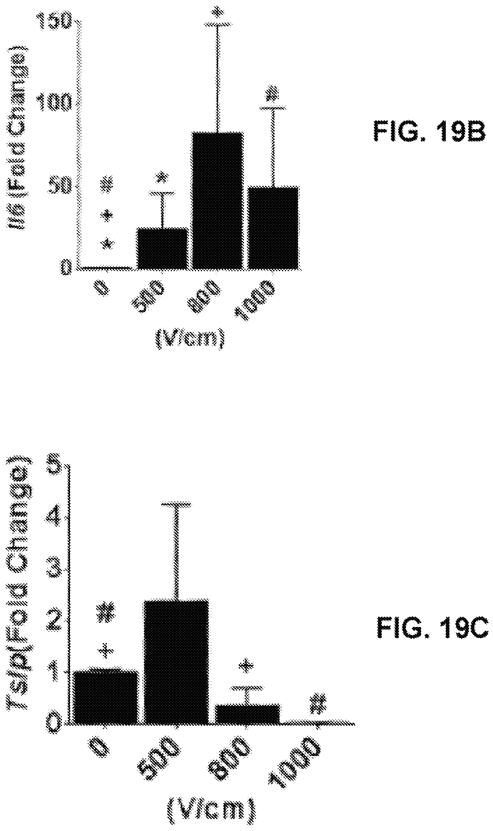

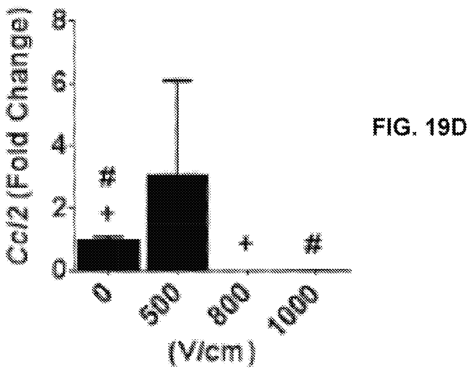

In these in vitro models, IRE treatment of mouse Pan02 pancreatic cancer cells was observed to promote a pro-inflammatory micro environment. IRE was observed to dose dependently induce cell death, while simultaneously increasing the production of pro-inflammatory mediators, such as IL-6 **(FIGS. 19A-19D)**. Furthermore, IRE treatment was shown to dose dependently reduce the concentrations of genes associated with the immunosuppressive microenvironment, including thymic stromal lymphopoietin (Tslp) and Ccl2 **(FIGS. 19A-19D)**. Epithelial derived cells are the primary producers of TSLP, which has been implicated in augmenting Th2 microenvironments and facilitating M1 macrophage polarization to M2/TAM phenotypes. Likewise, CCL2 has been shown to have multiple pro-tumorigenic roles, including promoting growth, facilitating angiogenesis, and recruiting host stromal cells to support progression.

**FIGS. 19A-19D** shows the results from gene expression pathway analysis of the tumor microenvironment following H-FIRE. To better define changes in the in situ 4T1 tumor microenvironment, we utilized gene expression profiling and commercial pathway analysis software to identify biological functions and gene networks impacted by treatment. In these studies, we observed significant down-regulation of breast cancer-associated genes in the ablated tumors compared to the untreated tumor groups. We also observed a significant shift in the balance between immunosuppressive and pro-inflammatory-associated gene transcription. Following treatment, genes associated with immunosuppression were significantly down-regulated, while genes associated with inflammation and pro-inflammatory immune responses were significantly up-regulated. Specifically, as shown here, we observed a significant shift in the tumor microenvironment to a Th1/Th17 profile and high levels of IL-6 following tumor ablation. A significant increase in IL-2 signaling was found in tumors that received a sub-ablation dose of H-FIRE, consistent with increased lymphocyte expansion and recruitment. Signaling networks associated with cytotoxicity were also significantly increased in responsive tumors, likely associated with increased cell death following treatment. Also shown here, gene networks associated with recruitment of leukocytes were significantly up-regulated in tumors following H-FIRE. IL-17 signaling and antigen presentation networks were also significantly up-regulated in some groups of animals. These data are consistent with the pro-inflammatory shift observed in the tumor microenvironment and may suggest that adaptive immune system signaling may benefit from this tumor ablation strategy. Together, these data indicate that H-FIRE treatment changes the tumor microenvironment, shifting it from one that is favorable for tumor progression to an antitumor, pro-inflammatory microenvironment driven by increased cellular immunity.

**Example 5.**

Patient specific modeling and IRE treatment have beneficial therapeutic effects on the clinical progression of pancreatic cancer. An initial study of IRE on 200 patients with radiographic stage III LAPC and perioperative 90-day outcomes, local failure, and overall survival was completed and reported (NCT02041936) (Martin et al. Treatment of 200 locally advanced (stage III) pancreatic adenocarcinoma patients with irreversible electroporation: safety and efficacy. Annals of surgery. 2015; 123(4):1008-25. All patients underwent chemotherapy and 52% of patients received chemoradiation therapy for a median of 6 months prior to IRE. The initial results suggested that IRE reduced length of hospital stays, reduced local recurrence rates, and increased survival from 13 months to 28.3 months.

Beyond these benefits, further examination into immune system response revealed a decrease in $T_{reg}$ populations in the pancreas within the first 3 days after treatment. An apparent influx of inflammatory T cells 9 days following IRE occurred. Immune cell infiltration paralleled vascular re-epithelialization, and this population was expected to contain proportionately more cytotoxic CD8+ T cells, pro-inflammatory macrophages, and anti-tumor T helper cells (Th1), as well as, fewer regulatory T cells, anti-tumor macrophages, MDSCs, and Th17 cells.

**Example 6.**

Despite promising treatments for cancer, metastatic disease and recurrence remain significant challenges. New therapeutic paradigms and treatment strategies are needed to eliminate mortality. Irreversible Electroporation (IRE) and High Frequency Irreversible Electroporation (H-FIRE) are emerging therapeutic approaches for tumor ablation. These techniques use a series of electric pulses applied through electrodes inserted directly into the targeted tumor to induce cancer cell death without the generation of significant thermal effects.

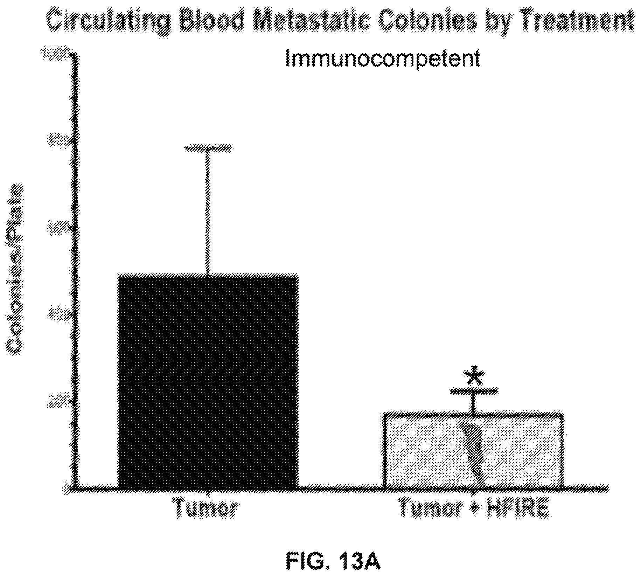

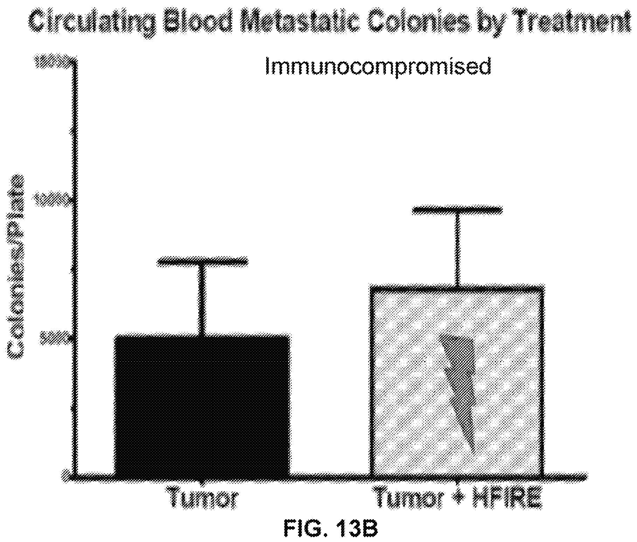

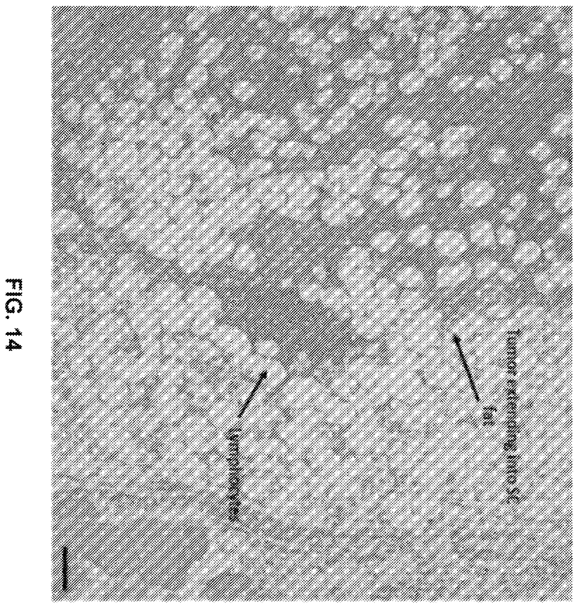

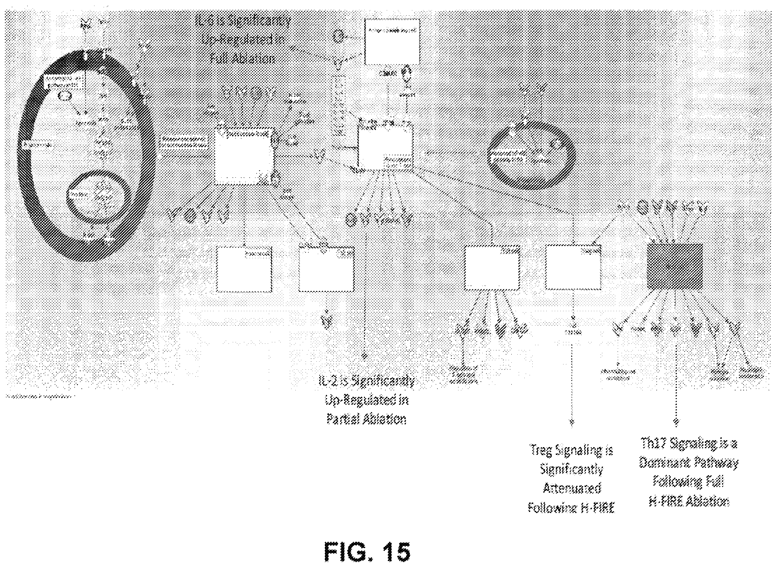

This Example can describe and demonstrate the effects of these treatment strategies on both tumor ablation and changes in the local tumor microenvironment. Both treatments were observed to be effective at improving survival outcomes and reducing tumor ablation in models of both breast and pancreatic cancer. Likewise, these treatments were both observed to improve innate and adaptive anti-tumor immunity by shifting the tumor microenvironment from an immunosuppressive environment that favors tumor growth and metastasis to a pro-inflammatory environment that restricts tumor progression. See e.g. **FIGS. 12A-16.** This can occur through the reduction of pro-tumor leukocytes in the field of treatment, the improved release of pro-inflammatory damage associated molecular patterns, and increased recruitment of antigen presenting cells and lymphocytes to the treated area. The non-thermal nature of the treatment can result in greater availability of tumor antigens in a non-denatured state, which can improve antigen presentation and engagement of the anti-tumor adaptive immune system.

Ultimately, promotion of the adaptive immune system improves detection and eradication of metastatic cells at sites distal to the primary tumor and treatment site. Together, these data can indicate that improved therapeutic outcomes can be achieved by a treat and resect approach, whereby resection occurs about 5-14 days post-IRE or post-HFIRE treatment after engagement of the adaptive immune system. The increased tumor antigen presentation and lymphocyte recruitment can improve conventional treatment strategies with chemotherapeutics and complement emerging immunomodulatory approaches targeting primary tumors, metastatic lesions, and preventing recurrence.

**Example 7.**

This Example can describe HFIRE treatment on HCC in canines. Three dogs with resectable HCC were recruited. CT was performed for HFIRE treatment planning prior to delivery on day 0. Post-treatment CT and tumor resection were performed on day 4. Dogs were hospitalized following HFIRE until discharge. Pre- and post-HFIRE biopsy samples were processed with H&E and IHC for CD3, CD4, CD8, and CD79a. Blood was collected on days 0, 2 and 4 for CBC and chemistry.

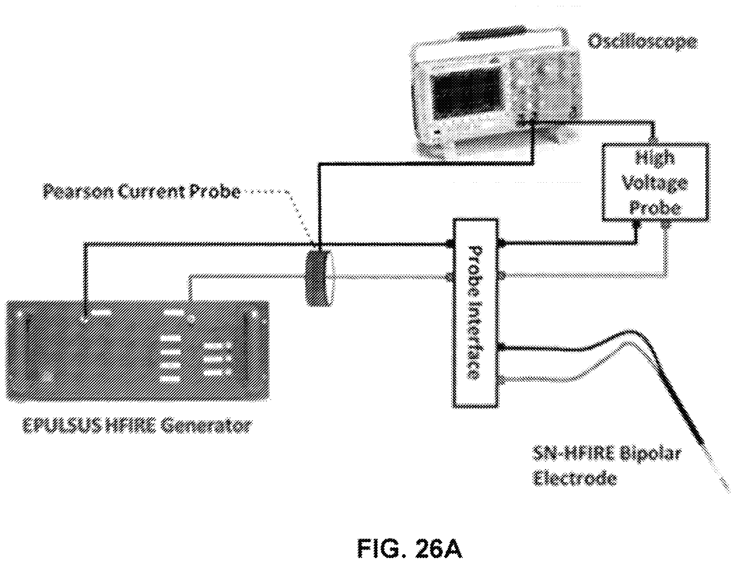

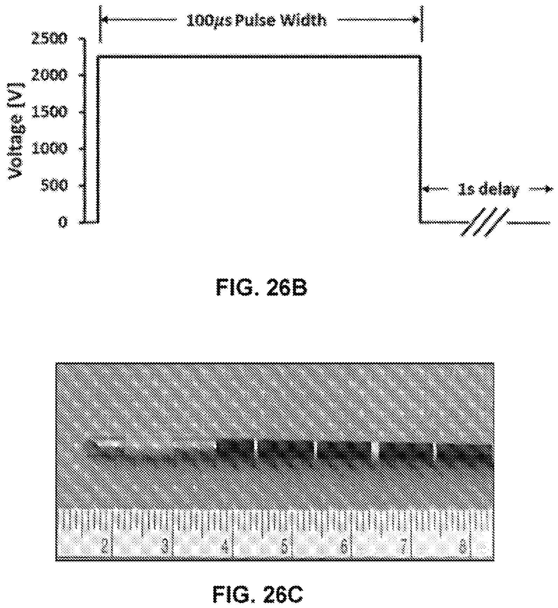

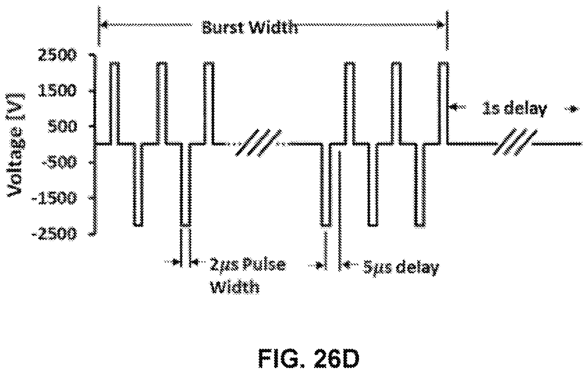

HFIRE generally resulted in predictable ablation volumes assessed by post-treatment CT and gross specimens. No detectable cardiac interference and minimal muscle contraction occurred during HFIRE. No clinically significant adverse events occurred secondary to HFIRE. Microscopically, a well-defined ablation zone surrounded by a reactive zone was evident in the majority of samples. This zone was composed primarily of maturing collagen interspersed with few fibroblasts, macrophages, and CD3+/CD4-/CD8- lymphocytes not identified in the pre-treatment samples. **FIGS. 26A-26D** can show a voltage waveform that can illustrate the bipolar electrode, a schematic of the experimental setup, and a comparison between a typical IRE voltage waveform and the HFIRE voltage waveform utilized in this assessment.

*Methods*

*Dogs.* Dogs diagnosed with HCC were screened for inclusion with a complete blood count, serum chemistry, urinalysis, prothrombin time, partial thromboplastin time, CT imaging of the thorax and abdomen with triple phase contrast enhancement and ultrasound-guided needle core biopsy of the liver mass prior to enrollment. Inclusion criteria included the presence of a surgically resectable primary liver tumor, a histologic diagnosis of HCC, and liver enzymes less than 4X the upper reference limit. Dogs were excluded if their tumor was non-resectable based on consultation with a board-certified surgeon or if survival was expected to be less than 6 weeks due to the presence of significant comorbidities.

*Imaging.* Dogs were imaged using a Toshiba Aquilion CT scanner. Dogs were anesthetized via inhalation anesthesia, breath hold during the scan. Precontrast and immediate, 1-minute delay and 3-minute delay scans of the thorax and abdomen were obtained using contrast agent and pressure injector. Images were analyzed in Horos image station. Dogs were imaged before H-FIRE treatment, and 4 days after treatment.

*H-FIRE treatment.*

A custom-built High Frequency Irreversible Electroporation (HFIRE) generator (EPULSUS®-FBM1-5, Energy Pulse Systems, Lisbon, Portugal) capable of producing sub-microsecond, bipolar pulses in rapid bursts was used to deliver HFIRE therapy via a single, 18-gauge bipolar electrode (AngioDynamics Inc., Latham, NY, USA) transcutaneously.

The generator was set to deliver 300 bursts of a voltage amplitude of 2,250 V via a voltage waveform presenting pulse widths 2 μs with a 5 μs delay between each change in polarity (2-5-2; on-off-on [μs]) for a total on-time (energized-time) of 100 μs for each burst. An oscilloscope (DPO2002B, Tektronix Inc., Beaverton, OR, USA) was used to monitor the voltage and current waveforms subsequent of the signal being attenuated using a 1000x high voltage probe (P5210A, Tektronix Inc., Beaverton, OR, USA) and passing through a current probe (2877, Pearson Electronics, Palo Alto, CA, USA). A voltage waveform Figure 1 illustrates the bipolar electrode, a schematic of the experimental set-up, and a comparison between a typical IRE voltage waveform and the HFIRE voltage waveform utilized within this assessment.

*Numerical Modeling.*

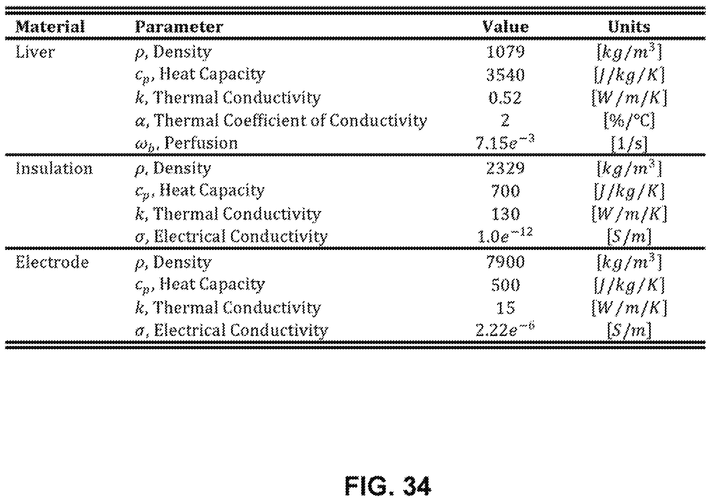

Numerical modeling can be employed predict electric field distributions, and determine electric field thresholds required prior to treatment of the patients. Abdominal computed tomography (CT) images from HCC patients were imported into 3D Slicer (an open source platform for medical image informatics), where relevant anatomical features (liver, hepatic artery, and tumor) were highlighted, labeled. Then a surface model maker tool was employed to interpolate between slices and re-construct the identified features in 3-dimensions. Each relevant geometry was transferred to 3-matic (Materialse, Leuven, Belgium), where the bipolar electrode was reconstructed and placed into the simulated tumor-tissue model. The entire assembly was meshed for import into a commercial finite element package (COMSOL Multiphysics, v.5.4; Stockholm, Sweden) for analysis using predefined boundary and initial conditions (FIG. 34). Dynamic electrical properties were then assigned to normal and malignant tissue within patient-specific hepatic geometries using tissue data normalized to hepatic tissue properties.

The methods for predicting electric field distributions for HFIRE are similar to those described for IRE therapies. The governing equation for the electric potential, $\Phi$ at the end of a pulse was determined from equation 1 using the electro-quasistatic approximation and the electric field distribution, $\bar{E}$ by taking the gradient of the electric potential as shown in equation 2.

$$0 = -\nabla \cdot (\sigma \nabla \Phi) \quad (1)$$

$$\bar{E} = -\nabla \Phi (2)$$

, where $\sigma$ is the tissue conductivity. The electrical boundary conditions at the tissue-electrode interface were set to $\Phi = V$ (source) and $\Phi = 0$ (sink). Boundaries not in contact with an electrode were treated as electrically insulating.

A modified Pennes Bioheat equation, shown in equation 3, was applied to simulate the tissue temperature throughout treatment

$$\rho c_p \frac{\partial T}{\partial t} = \nabla \cdot (k \nabla T) - \omega_b \rho_b c_b (T - T_b) + \sigma |\nabla \Phi|^2 \quad (3)$$

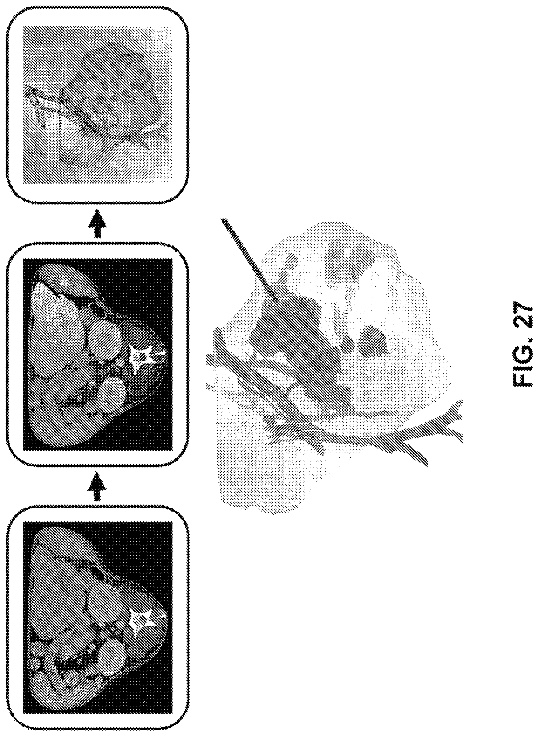

where $\rho$ is the density of the tissue, $c_b$ is the specific heat of the tissue, k is the thermal conductivity, $\omega_b$ is the blood perfusion, $\rho_b$ is the blood density, $c_b$ is the specific heat of the blood, and $T_b$ is the blood temperature. The tissue boundaries were defined as adiabatic at the edge of the domain to illustrate the maximum temperature increase within the tissue model. Further, a duty cycle approach was applied to the thermal simulation, averaging the thermal energy of a single pulse over a period of 1s to significantly reduce the computation time. **FIG. 27** provides a schematic describing each step of the 3D reconstruction process, including a patient specific finite element model.

*Dog hospitalization and tumor resection*

Dogs were hospitalized post H-FIRE treatment in the Intensive Care Unit (ICU) for monitoring, until surgical resection of their HCC. The surgery was performed 4 days post H-FIRE treatment, immediately after the second CT scan. Tumor resection was performed using standard surgical technique. After surgery, the tumor was submitted for histopathology and immunohistochemistry. Dogs were recovered in the ICU and discharged to their owners 2 days later. Recheck exam and blood collection were performed 2 weeks after surgical removal (day 14). CBC, serum chemistry and serum analyses were performed on blood collected at each time point.

*Histopathology and Immunohistochemistry.*

Pre- and post-H-FIRE biopsy samples were processed within 20 minutes of excision. Biopsy samples were evaluated by a board-certified pathologist (SLC) to confirm a definitive diagnosis and assess completeness of excision.

Post-HFIRE samples were grossly examined to identify regions of electroporation, non-treated tumor, and normal, non-neoplastic liver. Foci of electroporation were grossly identified in the majority of samples as well-demarcated foci of hemorrhage and necrosis. Sections were taken through these areas for examination of the treated/un-treated interface. Sections were routinely processed and stained with H&E. Immunohistochemistry for CD3, CD4, CD8, and CD79a was performed to evaluate the local immune response to treatment

*Gene Expression Analysis.*

Formalin-fixed paraffin embedded (FFPE) pre- and post-H-FIRE treatment tumor tissue was used in a Super-Array to compare specific gene expression between pre-treatment and post-treatment. Sections of the transition zone, at the ablated / non-ablated tumor of the post-treatment tumor were selected for RNA extraction. Total RNA was extracted from 10um thick slices of each of pre-treatment tumor biopsies, and post-treatment FFPE tissues. RNA extraction was performed using an RNA isolation kit (Zymo Research Corporation, Irvine, California, USA), according to manufacturer's directions. The extracted RNA was quantified and assessed through standard QA/QC. The resulting RNA samples went through 1st strand synthesis and qPCR amplification in an ABI 7500 FAST thermocycler, according to the Qiagen RT2 profiler protocol. A custom, canine specific, superarray developed and validated in our Lab was used, based on the Qiagen RT2 profiler platform. Our array design contains 89 genes of interest, associated with inflammation and cancer, 3 positive controls (actin, HPRT1, GAPDH), genomic DNA control, no-template control, no-RT control, and no-amplification control. The RT-qPCR data were analyzed

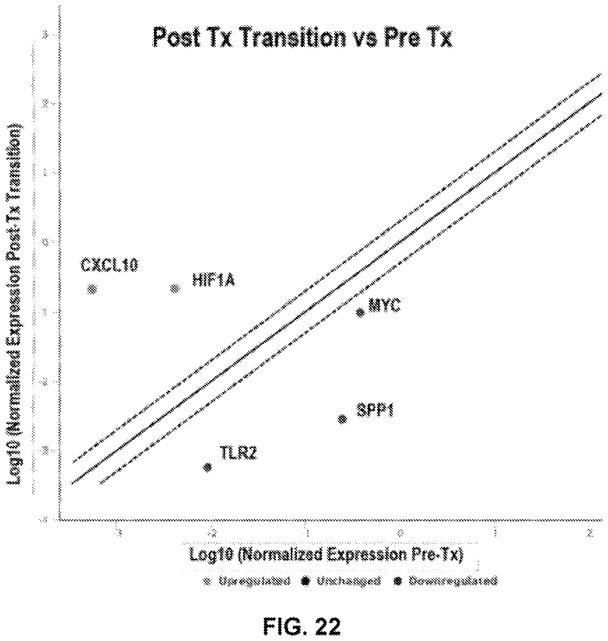

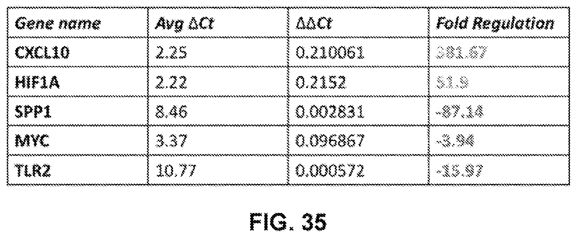

H-FIRE treatment can significantly increase cell death and innate immune system signaling. **FIG. 22** shows a scatter plot of gene expression array data illustrating the results from **FIG. 34**. CXCL10 and HIF1A expression is significantly up-regulated and TLR2, SPP1 and MYC are significantly down-regulated following H-FIRE treatment in all 3 patients compared to baseline. Change in expression of all other genes was less than 2-fold (unchanged) and represented by the solid black line using the 2(-Delta C(2)) methodology. We compared the pre-treatment gene expression profile to the post-treatment groups samples, normalized to Normal Liver tissue archived from previous dogs. The superarray results were analyzed using Qiagen's GeneGlobe Data Analysis software suite for individual gene expression differences and Ingenuity's IPA pathway analysis software for pathway analysis. **FIG. 35** shows gene expression results following HFIRE treatment from genes with significant changes in expression.

*Results.*

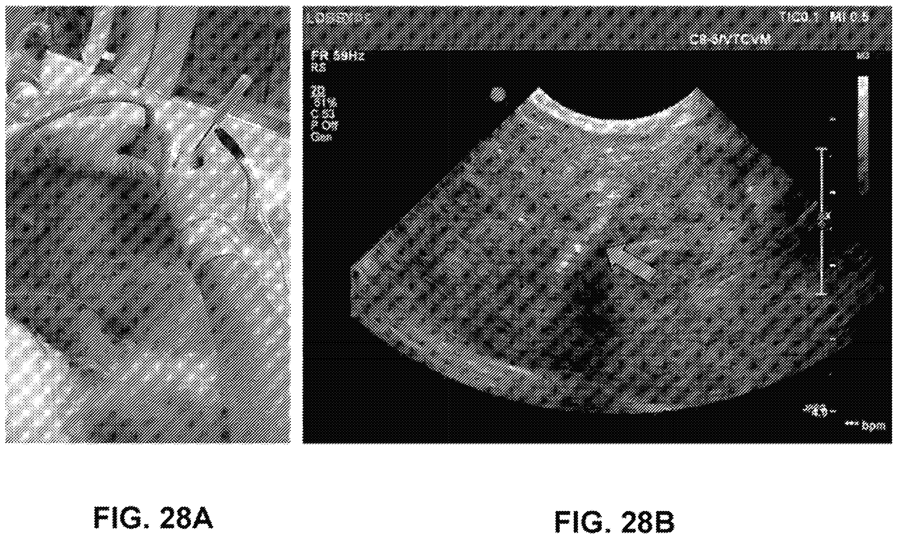

Three dogs met the inclusion criteria and were enrolled in the study. Patient 1 was a 15-year-old male castrated toy poodle with concurrent diabetes mellitus (controlled), hyperadrenocorticism (untreated) and seizures (controlled) that initially presented for general malaise and subsequent bloodwork revealed elevated liver enzymes. An MRI performed after his initial diagnosis of HCC revealed a primary brain tumor in the left frontal lobe most consistent with a meningioma and probably cerebral hemorrhages. Patient 2 was a 13-year-old female spayed mixed breed dog with concurrent hypothyroidism (medically controlled) that was asymptomatic on presentation but routine bloodwork revealed significant increases in liver enzymes, which initiated a thorough work-up. Patient 3 was a 14-year-old male castrated toy poodle with no significant concurrent medical conditions that presented with clinical signs of anorexia and weight loss. All patients had elevations in ALT and ALP at presentation. **FIGS. 28A-28B** show administration of HFIRE using ultrasound guided imaging. The arrow in **FIG. 28B** shows the bipolar treatment probe inserted into the tumor during treatment delivery.

Feasibility and Toxicity

H-FIRE Ablation

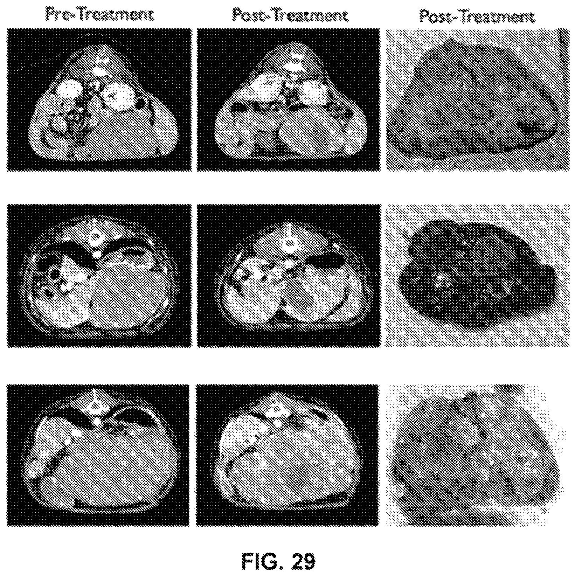

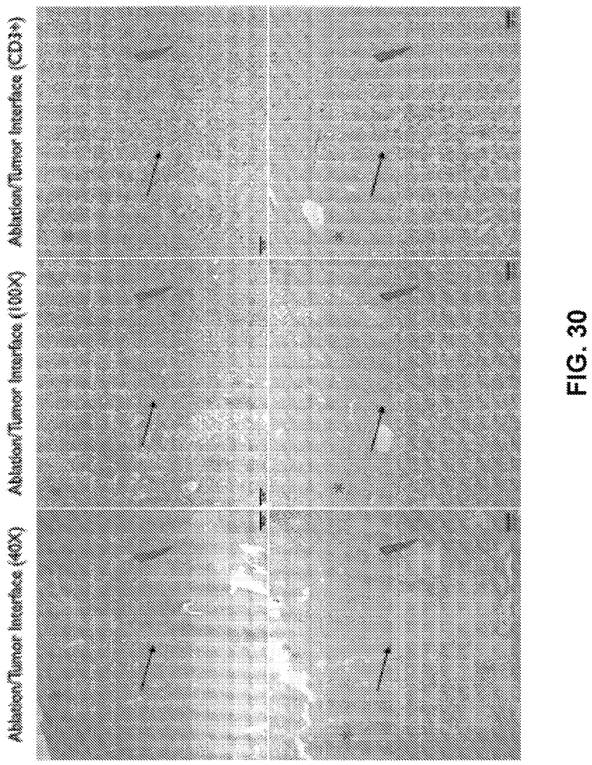

No clinically significant adverse events were associated with H-FIRE treatment. No detectable cardiac interference occurred during H-FIRE delivery and only minimal muscle contraction was noted despite the absence of paralytics. H-FIRE resulted in a predictable ablation volume visible on CT prior to surgical resection (day 4) and in gross tumor samples following tumor excision in all three dogs (**FIG. 29**). Histopathology (H&E) revealed a well-defined ablation/tumor interface following H-FIRE in 2 patients (**FIG. 30**). In 1 patient, the ablation/tumor interface was poorly defined (**FIG. 30**).

*Immunologic reaction to the ablation.*

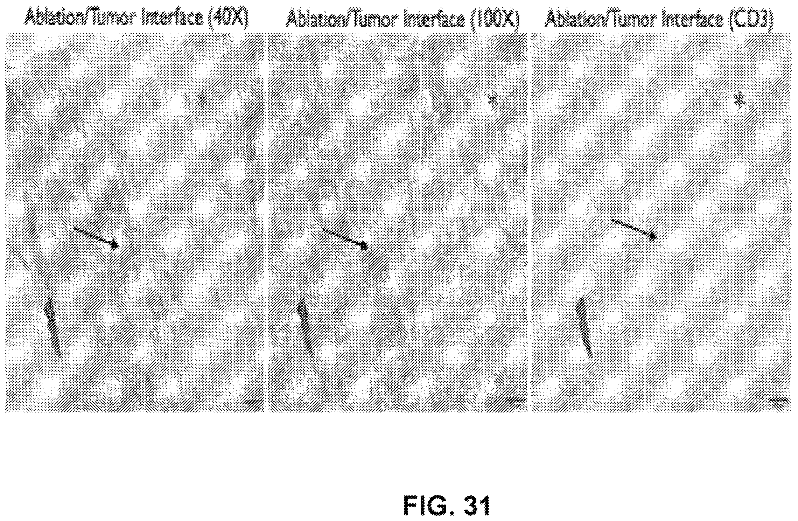

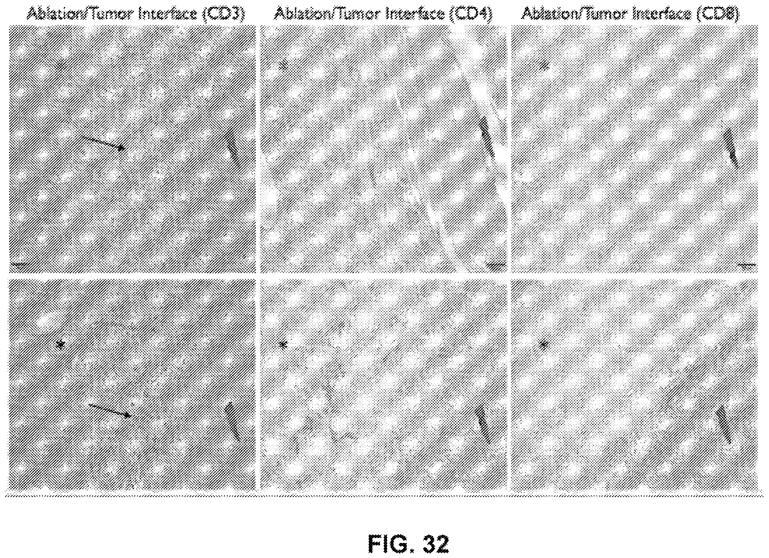

Immunohistochemistry on the tumor/ablation interface revealed infiltration with CD3+ lymphocytes following H-FIRE treatment in 2 patients (**FIG. 31**) Infiltrating T-cells were negative for both CD4 and CD8, suggesting they had a unique CD3+/CD4-/CD8- phenotype (**FIG. 32**) CD3+ lymphocyte infiltration was absent in the patient with a poorly defined ablation/tumor interface (**FIG. 30-31**) In all patients, the tumor/ablation interface was negative for CD79a+ lymphocytes.

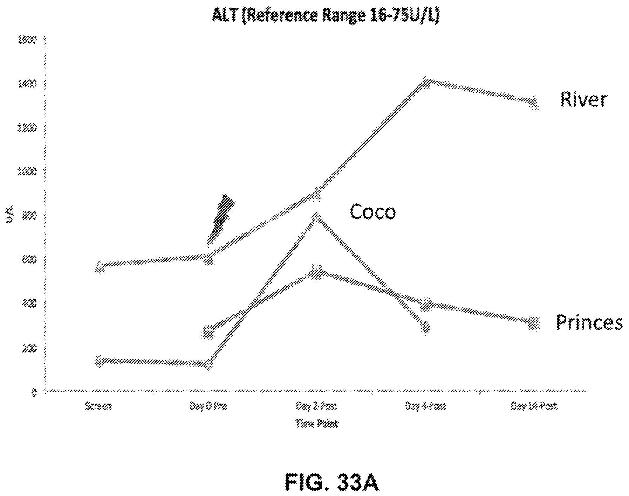

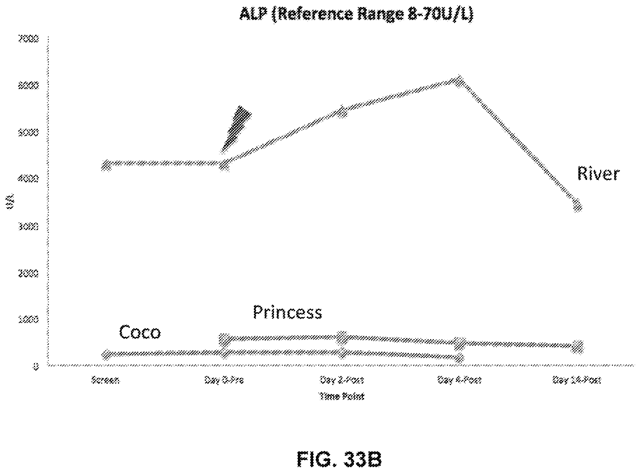

Alanine transaminase (ALT) and alkaline phosphatase (ALP) both increased compared to baseline following H-FIRE treatment (day 0) but resolved over time following tumor removal on day 4 (**FIGS. 33A-33B**). Liver enzyme elevations were most severe in patient 3 at all time points. Baseline liver values for patient 2 were obtained from the referring veterinarian so reference ranges varied significantly from serum chemistry analyzers at the VTH, thus it was excluded from the data set. Patient 1 was euthanized 8 days after surgery due to uncontrolled seizures thus recheck liver values were not available. Patients 2 and 3 are currently alive and undergo chest x-rays and an abdominal ultrasound every 3 months for re-staging. Neither recurrence nor metastasis has been identified in either patient to date.

Gene expression analysis (SuperArray) revealed up-regulation of CXCL10 and HIF1A expression following H-FIRE treatment in all 3 patients. In contrast, TLR2, SPP1 and MYC expression was down-regulated following H-FIRE treatment in all 3 patients compared to baseline (**FIGS. 22**). Change in expression of all other genes was less than 2-fold, thus considered unchanged.

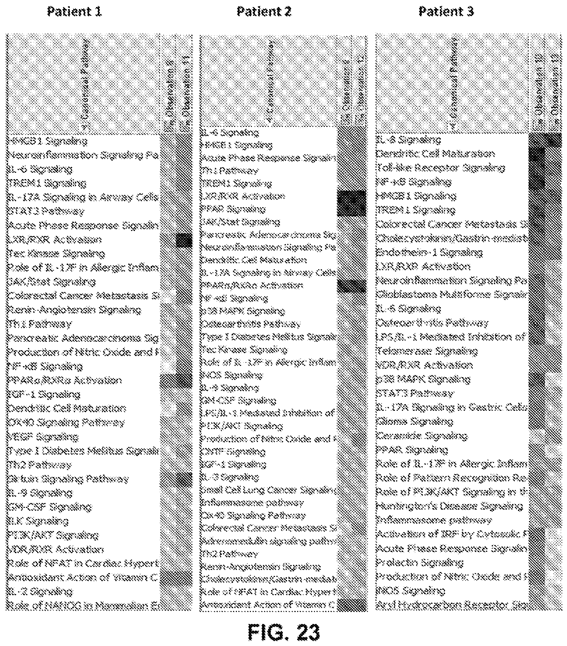

**FIG. 23** show results an Ingenuity Pathway Analysis (IPA) of global changes in gene expression patterns following H-FIRE in 3 patients. Results revealed diverse, but functionally related predications in canonical pathways significantly increased by H-FIRE for Patients 1 and 2. Conversely, the gene expression profile for Patient 3 showed either no change or down-regulation of functionally similar canonical pathways.

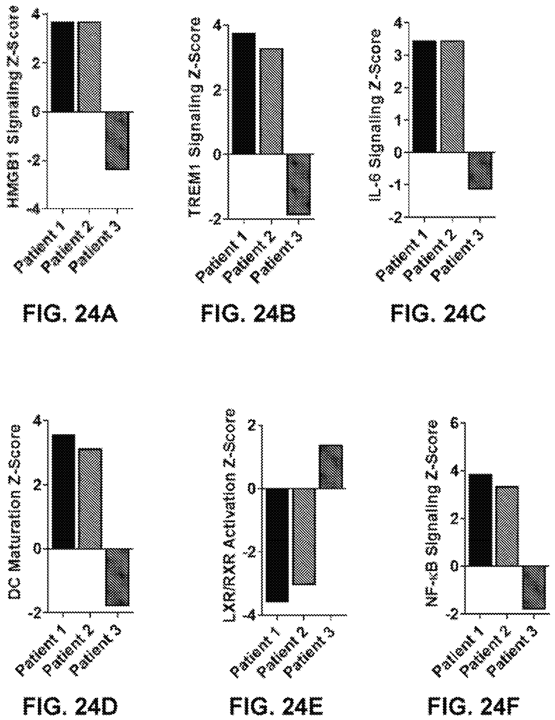

**FIGS. 24A-24F** are graphs showing results from the top 6 canonical pathways impacted by H-FIRE, comparing pre-treatment to post-treatment, ranked by z-score. Patient 1 and 2 are highly consistent, with patient 3 demonstrating opposing results.

**FIGS. 25A-25C** show pathways associated with the activation/promotion of cellular Immunity (**FIG. 25B**) and cell death (**FIG. 25A**) were significantly up-regulated following H-FIRE treatment. **FIG. 25A.** IPA network analysis identified 2 functional networks that fit the canonical pathways identified above, cell injury/death and cell mediated immunity. Both of these networks were significantly upregulated in all patients following H-FIRE treatment **FIG. 25C.** NF-$\kappa$B signaling was one of the most dominate pathways impacted by H-FIRE treatment. Gene expression patterns revealed a significant global up-regulation in NF-$\kappa$B signaling. While patient 3's data was significant up-regulated, it was a down-regulation in the network going to a less down-regulation in the network.

*Discussion*

High Frequency irreversible electroporation (H-FIRE) appears to be an effective minimally invasive method for HCC ablation in our spontaneous canine liver tumor model. In our proof of principle study, we did not need to use paralytics or cardiac synchronization, without any adverse events observed. The ablated tumor volume was in close agreement with previous work from our team in normal liver, and was characterized by a unique change in the tumor microenvironment. H-FIRE represents the next step in ablative technology that negates the need for paralytics and cardiac synchronization required during traditional IRE. H-FIRE resulted in a predictable ablation volume in all three dogs. This allows for more precise treatment planning prior to ablation of entire tumors to minimize damage to surrounding normal structures. Additionally, critical structures, such as vascular/biliary structures appear to be preserved following H-FIRE treatment, thus it could be superior to alternative ablation methods for treatment of tumors adjacent to these structures. This ablation technique was evaluated in resectable tumors to allow for thorough evaluation of the loco-regional that would otherwise be limited without removal of the entire tumor.

The unique CD3+/CD4-/CD8- phenotype of lymphocytes infiltrating the ablation/tumor interface was unexpected as terminally differentiated T-cells typically express either CD4 or CD8. Double negative T-lymphocytes appear to be involved in immune regulation and tolerance, as well as host defense and inflammation. Since the liver is an immunologically rich but tolerogenic organ, the presence of these cells may serve to prevent development of auto-immune reactions upon exposure to foreign substances entering the liver.

One explanation for the absence of T-cell infiltration within the ablation/tumor interface of patient 3 is as follows. Cell death secondary to tumor necrosis typically triggers an adaptive immune response. Despite the abundant necrosis contained within this patient's tumor, adaptive immune cell infiltration was not detected. Additionally, due to the amount of necrosis associated with this tumor, it was difficult to appreciate the ablation zone grossly, thus it is possible that sections submitted for histopathology did not represent the ablation/tumor interface.

Liver enzymes (ALT and ALP) increased above baseline in all patients following H-FIRE treatment and resolved following tumor removal. This increase was anticipated since tumor cells and normal hepatocytes likely contain similar enzymes that are released upon cell death. Patient 3 had the most severe liver enzyme elevations and took the longest for these elevations to resolve, likely secondary to the relative increase in tumor size compared to patients 1 and 2.

CXCL10 (C-X-C motif chemokine ligand 10) encodes the ligand for CXCR3. Binding of the ligand to its receptor results in stimulation of monocytes, natural killer and T-cell migration and modulation of adhesion molecule expression. HIF-1alpha (hypoxia inducible factor 1 subunit alpha) encodes for the alpha subunit of the transcription factor HIF-1, which regulates cellular and systemic homeostatic response to hypoxia, plays a role in tumor angiogenesis and may contribute to cell autophagy. Increased expression of these genes can suggest that autophagy may play a role in the mechanism of cell death.

TLR2 (toll like receptor 2) encodes a protein that functions in pathogen recognition and activation/promotion of the innate immunity and may promote apoptosis. SPP1 (secreted phosphoprotein 1) encodes a cytokine that upregulates expression of IFN-y and IL-12, which are important for activation/promotion of innate and adaptive immunity. MYC is a proto-oncogene that codes for a protein involved in cell cycle progression, apoptosis and cellular transformation. Given this information, decreased expression of TLR2, SPP1 and MYC were not expected. This may be because by post-treatment measurements represent a single point in time, so initial increases in gene expression may have been missed.

Regulatory T-cells play an important role in immunosurveillance within the tumor microenvironment. Initial attempts at performing flow cytometry on peripheral blood for FOXP3 were compromised by sample handing during shipment, and since cells need to be alive for evaluation, repeat analysis on blood/serum is not possible. We plan to develop a method to extract lymphocytes from the ablation/tumor interface of persevered samples to definitively characterize their phenotype. Additionally, immunohistochemistry for FOXP3 will be performed on existing tumor samples, specifically evaluating the ablation/tumor interface. Beyond evaluation of H-FIRE in non-resectable tumors, future studies should aim to evaluate the effect of local therapy on distant metastatic lesions (abscopal effect). Additionally, combination therapy with checkpoint inhibitors may enhance the initial immune response induced by H-FIRE delivery, and should therefore be evaluated.

The scope of the present invention is defined by the appended claims.

## Claims

1. A medical diagnostic system comprising:

   a generator configured to deliver one or more electrical pulses to a patient;
   a current output monitor to collect data relating to electrical current resulting from one or more of the electrical pulses delivered;
   programming configured to determine an average current or a change in current from the electrical current data collected by the current output monitor, and based on the average current and/or the change in current:
   determine whether and/or how the patient is expected to respond to immunotherapy, such that when there is a positive change in current it is determined that the patient is expected to respond to immunotherapy.

2. The system of claim 1, wherein the generator is configured to deliver one or more of the electrical pulses to a patient in an amount and for a time capable of causing non-thermal ablation of cells of tissue of the patient.

3. The system of claim 2, wherein the one or more electrical pulses are configured to result in a non-immunosuppressive response.

4. The system of claim 1, wherein the programming is configured to determine whether and/or when to deliver a subsequent treatment, wherein such subsequent treatments include chemotherapy, radiation, and/or other standard treatments of care, such as for treating one or more cancers, including tissue resection, thermal ablation, non-thermal ablation, immunotherapy, adjuvant therapy, biologic therapy, genetic therapy, and combinations thereof.

5. The system of claim 1, wherein the one or more electrical pulses results in electroporation; and/or results in ablation; and/or results in irreversible electroporation; and/or results in reversible electroporation; and/or results in non-thermal electroporation, irreversible electroporation, reversible electroporation, or ablation of cells of one or more tissue.

6. The system of claim 1, wherein the system is configured to allow for waiting a period of time, such as days or weeks or months, and optionally 1 day, 2 days, 3 days, 4 days, 5 days, 6 days, 7 days, 8 days, 1 week, 2 weeks, 3 weeks, and/or a month or more, to determine an immune response of the patient.

7. The system of claim 2, wherein the one or more electrical pulses are configured to alternate polarity.

8. The system of claim 7, wherein the one or more electrical pulses are configured to have a time of delivering zero voltage between alternating polarity.

9. The system of claim 1, wherein the one or more electrical pulses are administered using one or more of the following parameters:

an output voltage ranging from 2,000 V to 10,000 V;
a burst width ranging from 500 nsec to 1 msec (such as 10, 50, 100, 200, 500 μsec);
an inter-burst delay ranging from 1 msec to 5 sec (such as 100, 500, 1,000 msec);
a pulse width ranging from 100 nsec to 10 μsec (such as 0.5, 1, 2, 3, 4, 5, 6, 7, 8, 9 psec);
an inter-pulse delay ranging from 100 nsec to 1 msec (such as 500, 1,000, 5,000 nsec);
from 1-1,000 pulse bursts (such as 10, 25, 50, 75, 90, 100, 150, or 200 bursts);
and/or biphasic or monopolar pulses.

10. The system of claim 1, wherein when the change in current is at least 25 Amps it is determined that the patient is expected to respond to immunotherapy.

11. The system of claim 1, wherein when the change in current is up to 400 % it is determined that the patient is expected to respond to immunotherapy.

12. The system of any one of claims 1 to 11 for use as a diagnostic tool to:
determine a patient's amenability to immunotherapy and whether and/or when to deliver a subsequent immunotherapy treatment to a patient.

**Patentansprüche**

1. Medizinisches Diagnosesystem, umfassend:

einen Generator, der so konfiguriert ist, dass er einen oder mehrere elektrische Impulse an einen Patienten abgibt,
einen Stromausgangsmonitor zur Erfassung von Daten bezüglich des elektrischen Stroms, der sich aus einem oder mehreren der abgegebenen elektrischen Impulse ergibt;
Programmierung, die so konfiguriert ist, dass sie einen Durchschnittsstrom oder eine Stromänderung aus den vom Stromausgangsmonitor erfassten elektrischen Stromdaten bestimmt und auf der Grundlage des Durchschnittsstroms und/oder der Stromänderung:
bestimmen, ob und/oder wie der Patient voraussichtlich auf eine Immuntherapie ansprechen wird, so dass bei einer positiven Veränderung des Stroms festgestellt wird, dass der Patient voraussichtlich auf die Immuntherapie ansprechen wird.

2. System nach Anspruch 1, wobei der Generator so konfiguriert ist, dass er einen oder mehrere der elektrischen Impulse in einer Menge und für eine Dauer an einen Patienten abgibt, die geeignet sind, eine nicht-thermische Ablation von Zellen des Gewebes des Patienten zu bewirken.

3. System nach Anspruch 2, wobei der eine oder die mehreren elektrischen Impulse so konfiguriert sind, dass sie zu einer nicht-immunosuppressiven Reaktion führen.

4. System nach Anspruch 1, wobei die Programmierung so konfiguriert ist, dass sie bestimmt, ob und/oder wann eine nachfolgende Behandlung durchgeführt werden soll, wobei solche nachfolgenden Behandlungen Chemotherapie, Bestrahlung und/oder andere Standardbehandlungen umfassen, wie z.B. zur Behandlung eines oder mehrerer Krebsarten, einschließlich Geweberesektion, thermische Ablation, nicht-thermische Ablation, Immuntherapie, adjuvante Therapie, biologische Therapie, Gentherapie und Kombinationen davon.

5. System nach Anspruch 1, wobei der eine oder die mehreren elektrischen Impulse zu Elektroporation führen; und/oder zu Ablation führen und/oder zu irreversibler Elektroporation führen; und/oder zu reversibler Elektroporation führen; und/oder zu nicht-thermischer Elektroporation, irreversibler Elektroporation, reversibler Elektroporation oder Ablation von Zellen eines oder mehrerer Gewebe führen.

6. System nach Anspruch 1, wobei das System so konfiguriert ist, dass es das Abwarten einer Zeitspanne, wie Tagen oder Wochen oder Monaten, und optional 1 Tag, 2 Tagen, 3 Tagen, 4 Tagen, 5 Tagen, 6 Tagen, 7 Tagen, 8 Tagen, 1 Woche, 2 Wochen, 3 Wochen und/oder einen Monat oder mehr, ermöglicht, um eine Immunantwort des Patienten zu bestimmen.

7. System nach Anspruch 2, wobei der eine oder die mehreren elektrischen Impulse so konfiguriert sind, dass sie die Polarität wechseln.

8. Das System nach Anspruch 7, wobei der eine oder die mehreren elektrischen Impulse so konfiguriert sind, dass sie zwischen den wechselnden Polaritäten eine Zeit lang eine Nullspannung liefern.

9. System nach Anspruch 1, wobei der eine oder die mehreren elektrischen Impulse unter Verwendung eines oder mehrerer der folgenden Parameter verabreicht werden:

  einer Ausgangsspannung von 2.000 V bis 10.000 V;
  einer Burst-Breite von 500 ns bis 1 ms (z. B. 10, 50, 100, 200, 500 $\mu$s);
  einer Inter-Burst-Verzögerung von 1 ms bis 5 s (z. B. 100, 500, 1.000 ms);
  einer Impulsbreite von 100 ns bis 10 $\mu$s (z. B. 0,5, 1, 2, 3, 4, 5, 6, 7, 8, 9 $\mu$s);
  einer Verzögerung zwischen den Impulsen von 100 ns bis 1 ms (z. B. 500, 1.000, 5.000 ns);
  von 1-1.000 Pulsbursts (z. B. 10, 25, 50, 75, 90, 100, 150 oder 200 Bursts);
  und/oder biphasischen oder monopolaren Impulsen.

10. System nach Anspruch 1, wobei bei einer Stromänderung von mindestens 25 Ampere festgestellt wird, dass der Patient voraussichtlich auf eine Immuntherapie anspricht.

11. System nach Anspruch 1, wobei bei einer Stromänderung von bis zu 400 % festgestellt wird, dass der Patient voraussichtlich auf eine Immuntherapie anspricht.

12. Das System nach einem der Ansprüche 1 bis 11 zur Verwendung als Diagnosewerkzeug, um: die Eignung eines Patienten für eine Immuntherapie zu bestimmen und, ob und/oder wann dem Patienten eine nachfolgende Immuntherapie verabreicht werden soll.

**Revendications**

1. Système de diagnostic médical, comprenant :

  un générateur configuré pour délivrer une ou plusieurs impulsions électriques à un patient ;
  un moniteur de sortie de courant pour collecter des données relatives au courant électrique résultant d'une ou plusieurs des impulsions électriques délivrées ;
  une programmation configurée pour déterminer un courant moyen ou un changement de courant à partir des données de courant électrique collectées par le moniteur de sortie de courant, et sur la base du courant moyen et/ou du changement de courant :
  déterminer si et/ou comment le patient est censé répondre à une immunothérapie, de sorte que lorsqu'il y a un changement positif dans le courant, il est déterminé que le patient est censé répondre à l'immunothérapie.

2. Système selon la revendication 1, dans lequel le générateur est configuré pour délivrer une ou plusieurs des impulsions électriques à un patient en une quantité et pendant une durée capables de provoquer une ablation non thermique de cellules de tissu du patient.

3. Système selon la revendication 2, dans lequel les une ou plusieurs impulsions électriques sont configurées pour entraîner une réponse non immunosuppressive.

4. Système selon la revendication 1, dans lequel la programmation est configurée pour déterminer si et/ou quand administrer un traitement ultérieur, dans lequel de tels traitements ultérieurs comprennent une chimiothérapie, une radiothérapie et/ou d'autres traitements de soins standards, tels que pour traiter un ou plusieurs cancers, y compris une résection tissulaire, une ablation thermique, une ablation non thermique, une immunothérapie, une thérapie adjuvante, une thérapie biologique, une thérapie génétique et des combinaisons de celles-ci.

5. Système selon la revendication 1, dans lequel les une ou plusieurs impulsions électriques entraînent une électroporation ; et/ou entraînent une ablation ; et/ou entraînent une électroporation irréversible ; et/ou entraînent une électroporation réversible ; et/ou entraînent une électroporation non thermique, une électroporation irréversible, une électroporation réversible, ou une ablation de cellules d'un ou plusieurs tissus.

**6.** Système selon la revendication 1, dans lequel le système est configuré pour permettre d'attendre une période de temps, telle que des jours ou des semaines ou des mois, et facultativement 1 jour, 2 jours, 3 jours, 4 jours, 5 jours, 6 jours, 7 jours, 8 jours, 1 semaine, 2 semaines, 3 semaines, et/ou un mois ou plus, pour déterminer une réponse immunitaire du patient.

**7.** Système selon la revendication 2, dans lequel les une ou plusieurs impulsions électriques sont configurées pour alterner en termes de polarité.

**8.** Système selon la revendication 7, dans lequel les une ou plusieurs impulsions électriques sont configurées pour présenter un temps de délivrance d'une tension nulle entre une polarité changeante.

**9.** Système selon la revendication 1, dans lequel les une ou plusieurs impulsions électriques sont administrées à l'aide d'un ou plusieurs des paramètres suivants :

une tension de sortie allant de 2000 V à 10000 V ;
une largeur de salve allant de 500 ns à 1 ms (par exemple 10, 50, 100, 200, 500 $\mu$s) ;
un délai entre les salves allant de 1 ms à 5 secondes (comme 100, 500, 1 000 ms) ;
une largeur d'impulsion allant de 100 nsec à 10 psec (telle que 0,5, 1, 2, 3, 4, 5, 6, 7, 8, 9 $\mu$sec) ;
un délai entre les impulsions allant de 100 nsec à 1 msec (tel que 500, 1000, 5000 nsec) ;
de 1 à 1 000 salves d'impulsions (telles que 10, 25, 50, 75, 90, 100, 150 ou 200 salves) ;
et/ou des impulsions biphasiques ou monopolaires.

**10.** Système selon la revendication 1, dans lequel lorsque le changement de courant est d'au moins 25 ampères, il est déterminé que le patient est censé répondre à l'immunothérapie.

**11.** Système selon la revendication 1, dans lequel lorsque la variation de courant atteint 400 %, il est déterminé que le patient est censé répondre à l'immunothérapie.

**12.** Système selon l'une quelconque des revendications 1 à 11, pour utilisation en tant qu'outil de diagnostic pour : déterminer l'aptitude d'un patient à l'immunothérapie et si et/ou quand administrer un traitement d'immunothérapie ultérieur à un patient.

FIG. 1

FIGS. 2A-2D

% CD4+CD25+FoxP3+ of Total T Cells 24h Post-IRE vs Difference in Current

$R^2 = 0.9166$

p-value = 0.0190772398

FIG. 3

FIG. 4

% CD4+CD25+ of Total T Cells 24h Post-IRE v's Average Current

$R^2 = 0.6849$

p-value = 0.0452

Average Current [A]

% CD4+CD25+ 24H Post-IRE

**FIG. 5**

FIG. 6

**FIG. 7**

FIG. 8

FIG. 9

FIG. 10A

FIG. 10B

FIG. 11A

FIG. 11B

FIG. 11C

**Calculated Tumor Diameter by Day and Treatment**

FIG. 12A

FIG. 12B

Circulating Blood Metastatic Colonies by Treatment
Immunocompetent

FIG. 13A

FIG. 13B

FIG. 14

FIG. 15

FIG. 16

FIG. 17

FIG. 18A

FIG. 18B    FIG. 18C    FIG. 18D    FIG. 18E

FIG. 19A

FIG. 19B

FIG. 19C

FIG. 19D

$$K = \frac{2\pi d}{\cosh^{-1} \dfrac{2d^2 - 4r^2}{r^2}}$$

FIG. 20

| | *V* (V) | σ (S/m) | *d* (cm) | *r* (mm) | *K* | *I* (A) |
|---|---|---|---|---|---|---|
| 1 | 3000 | 0.2 | 1 | 0.5 | 0.0085 | 5.11 |
| 2 | 3000 | 0.2 | 2 | 0.5 | 0.0143 | 8.60 |
| 3 | 3000 | 0.3 | 3 | 0.5 | 0.0197 | 17.7 |
| 4 | 3000 | 1 | 1 | 0.5 | 0.0085 | 25.5 |
| 5 | 3000 | 1 | 3 | 0.5 | 0.0197 | 59.1 |

FIG. 21

FIG. 22

**FIG. 23**

EP 3 852 868 B1

FIG. 24A

FIG. 24B

FIG. 24C

FIG. 24D

FIG. 24E

FIG. 24F

FIG. 25A

FIG. 25B

**FIG. 25C**

FIG. 26A

FIG. 26B

FIG. 26C

FIG. 26D

FIG. 27

FIG. 28B

FIG. 28A

FIG. 29

Ablation/Tumor Interface (40X)　　Ablation/Tumor Interface (100X)　　Ablation/Tumor Interface (CD3+)

FIG. 30

FIG. 31

FIG. 32

FIG. 33A

FIG. 33B

| Material | Parameter | Value | Units |
|---|---|---|---|
| Liver | $\rho$, Density | 1079 | $[kg/m^3]$ |
| | $c_p$, Heat Capacity | 3540 | $[J/kg/K]$ |
| | $k$, Thermal Conductivity | 0.52 | $[W/m/K]$ |
| | $\alpha$, Thermal Coefficient of Conductivity | 2 | $[\%/°C]$ |
| | $\omega_b$, Perfusion | $7.15e^{-3}$ | $[1/s]$ |
| Insulation | $\rho$, Density | 2329 | $[kg/m^3]$ |
| | $c_p$, Heat Capacity | 700 | $[J/kg/K]$ |
| | $k$, Thermal Conductivity | 130 | $[W/m/K]$ |
| | $\sigma$, Electrical Conductivity | $1.0e^{-12}$ | $[S/m]$ |
| Electrode | $\rho$, Density | 7900 | $[kg/m^3]$ |
| | $c_p$, Heat Capacity | 500 | $[J/kg/K]$ |
| | $k$, Thermal Conductivity | 15 | $[W/m/K]$ |
| | $\sigma$, Electrical Conductivity | $2.22e^{-6}$ | $[S/m]$ |

FIG. 34

| Gene name | Avg ΔCt | ΔΔCt | Fold Regulation |
|-----------|---------|------|-----------------|
| CXCL10 | 2.25 | 0.210061 | 381.67 |
| HIF1A | 2.22 | 0.2152 | 51.9 |
| SPP1 | 8.46 | 0.002831 | -87.14 |
| MYC | 3.37 | 0.096867 | -3.94 |
| TLR2 | 10.77 | 0.000572 | -15.97 |

## FIG. 35

FIG. 36

**FIG. 37**

EP 3 852 868 B1

probes

72

22

22

Pulse generation circuit

Sensor

73

10

Controller

71

52

FIG. 38

83

FIG. 39

**FIG. 40**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017348525 A1
- US 2016066977 A1
- US 2017319851 A1
- US 2016143698 A1
- US 2018071014 A
- US 8926606 B
- US 20120109122 A
- US 20140039489 A
- US 7344533 B
- US 20190175248 A1

**Non-patent literature cited in the description**

- **MARTIN et al.** Treatment of 200 locally advanced (stage III) pancreatic adenocarcinoma patients with irreversible electroporation: safety and efficacy. *Annals of surgery.*, 2015, vol. 123 (4), 1008-25